# EUROPEAN PATENT APPLICATION

(11) **EP 2 418 207 A1**
(43) Date of publication of application: **15.02.2012**
(21) Application number: 11173579.1
(22) Date of filing: 04.05.2009
(51) Int. Cl.: C07D 271/10, C07D 403/04, C07D 413/04, C07D 417/04, C07D 271/06, C07D 413/14, C07D 417/14, C07D 405/14, A61K 31/422, A61P 37/06

(54) **Sulfone compounds which modulate the CB2 receptor**

(30) Priority: 13.05.2008 US 52658 P
(62) Divisional of application: 09747175.9
(71) Applicant: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: Hickey, Eugene Richard, Ridgefield, CT Connecticut 06877-0368 (US); Riether, Doris, Ridgefield, CT Connecticut 06877-0368 (US); Thomson, David, Smith, Ridgefield, CT Connecticut 06877-0368 (US); Zindell, Renee, M., Ridgebury, CT Connecticut 06877-0368 (US); Amouzegh, Patricia, Abingdon, Oxfordshire OX14 4SA (GB); Palmer, Christopher, Francis, Abingdon, Oxfordshire OX14 4SA (GB); Whitaker, Mark, Abingdon, Oxfordshire OX14 4SA (GB)
(74) Representative: Hammann, Heinz

(57) **Abstract**

Compounds which modulate the CB2 receptor are disclosed. Compounds according to the invention bind to and are agonists of the CB2 receptor, and are useful for treating inflammation. Those compounds which are agonists are additionally useful for treating pain.

## Description

### BACKGROUND OF THE INVENTION

### 1. TECHNICAL FIELD

The present invention relates to novel compounds which modulate the CB2 receptor and their use as medicaments.

### 2. BACKGROUND INFORMATION

Cannabinoids are a group of about 60 distinct compounds found in *Cannabis sativa* (also know as marijuana) with cannabinol, cannabidiol and Δ⁹-tetrahydrocannabinol (THC) being the most representative molecules. The therapeutic usage of *Cannabis* can be dated back to ancient dynasties of China and includes applications for various illnesses ranging from lack of appetite, emesis, cramps, menstrual pain, spasticity to rheumatism. The long history of *Cannabis* use has led to the development of several pharmaceutical drugs. For example, Marinol and Cesamet which are based on THC and its analogous nabilone, respectively, are used as anti-emetic and appetite stimulant. Despite of the clinical benefits, the therapeutic usage of cannabis is limited by its psychoactive effects including hallucination, addiction and dependence. Mechoulam R, ed. Cannabinoids as Therapeutic Agents, Boca Raton, FL; CRC Press, 1986 provides a review of the medicinal use of cannabis.

The physiological effects of cannabinoids are mediated by at least two G-protein coupled receptors, CB 1 and CB2. Autoradiographic studies have demonstrated that CB 1 receptors are expressed primarily in the central nervous system, specifically in the cerebral cortex, hippocampus, basal ganglia and cerebellum. They are also found to a lesser degree in the reproductive system and other peripheral tissues including that of the immune system. CB1 receptors regulate the release of neurotransmitters from the pre-synaptic neurons and are believed to mediate most of the euphoric and other central nervous system effects of cannabis, such as THC-induced ring-catalepsy, hypomobility, and hypothermia, which were found to be completely absent in mice with a deletion of the CB1 gene (Zimmer et al., Increased mortality, hypoactivity, and hypoalgesia in cannabinoid CB 1 receptor knockout mice. Proc Natl Acad Sci USA. (1999) 96:5780-5785.)

CB2 receptors are almost exclusively found in the immune system, with the greatest density in the spleen. It is estimated that the expression level of CB2 in the immune cells is about 10 to 100 times higher than CB1. Within the immune system, CB2 is found in various cell types, includung B cells, NK cells, monocytes, microglial cells, neutrophils, T cells, dentritic cells and mast cells, suggesting that a wide range of immune functions can be regulated through CB2 modulators (Klein et al., The cannabinoid system and immune system. J Leukoc Biol (2003) 74:.486-496). This is supported by the finding that the immunomodulatory effect of THC is absent in CB2 deficient mice mice (Bicklet et al., Immunomodulation by cannabinoid is absent in mice deficient for the cannabinoid CB2 receptor. Eur J Pharmacol (2000) 396:141-149). CB2 selective ligands have been developed and tested for their effects in various imflammatory settings. For example, in animal models of inflammation, CB2 selective agonists, inverse agonists and antagonists have been shown to be effective in suppressing inflammation (Hanus et al., HU-308: a specific agonist for CB(2), a peripheral cannabinoid receptor. Proc Natl Acad Sci USA. (1999) 96:14228-14233, Ueda et al., Involvement of cannabinoid CB(2) receptor-mediated response and efficacy of cannabinoid CB(2) receptor inverse agonist, JTE-907, in cutaneous inflammation in mice. Eur J Pharmacol. (2005) 520:164-171 and Smith et al., The anti-inflammatory activities of cannabinoid receptor ligands in mouse peritonitis models Eur J Pharmacol. (2001) 432:107-119.). Furthermore, CB2 selective agonists inhibit disease severity and spasticity in animal models for multiple sclerosis (Baker et al., Cannabinoids control spasticity and tremor in a multiple sclerosis model. Nature (2000) 404:84-87.Arevalo-Martin et al., Therapeutic action of cannabinoids in a murine model of multiple sclerosis J Neurosci. (2003) 23:2511-2516.). Taken together, these results support the notion that CB2 receptor modulators can be employed for the treatment of medical conditions having an inflammatory component.

In addition to inflammation, CB2 agonists have been shown to inhibit pain and emesis. For instance, CB2 selective agonists blunt the pain response induced by thermal or other stimuli (Malan et al., CB2 cannabinoid receptor-mediated peripheral antinociception. Pain. (2001) 93:239-45 and Nackley et al., Selective activation of cannabinoid CB(2) receptors suppresses spinal fos protein expression and pain behavior in a rat model of inflammation. Neuroscience (2003) 119:747-57.) CB2 activation has also been demonstrated to inhibit neuropathic pain response (Ibrahim et al., Activation of CB2 cannabinoid receptors by AM1241 inhibits experimental neuropathic pain: pain inhibition by receptors not present in the CNS. Proc Natl Acad Sci USA. (2003) 100:10529-33.) Finally, in contrast to the earlier data which did not find CB2 in the brain, a recent article demonstrated the expression of CB2 in the brain, at about 1.5 % of the level in the spleen. CB2 activation is shown by this article to be responsible for the anti-emetic effect of endocannabinoid (Van Sickle et al., Identification and functional characterization of brainstem cannabinoid CB2 receptors. Science. 2005 310:329-332.) The foregoing results confirm that CB2 agonists can be used for the treatment of inflammatory and neuropathic pain as well as emesis.

### BRIEF SUMMARY OF THE INVENTION

The present invention provides novel compounds which bind to and modulate the CB2 receptor. The invention also provides a method and pharmaceutical compositions for treating inflammation by way of the administration of therapeutic amounts of these compounds. Lastly, the invention provides a method and pharmaceutical compositions for treating pain by way of the administration of therapeutic amounts of the new compounds which are CB2 agonists.

### DETAILED DESCRIPTION OF THE INVENTION

In its broadest generic aspect the invention provides compounds of formula I, wherein

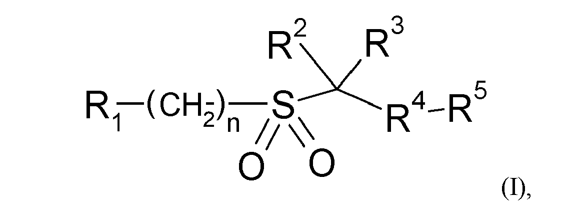

**R¹** is aryl optionally independently substituted with 1 to 3 substituents chosen from C₁₋₆ alkyl, C₃₋₆cycloalkyl, C₁₋₆ alkoxy, C₁₋₆ alkylthio, C₁₋₆alkylsulfonyl, C₁₋₆ alkoxycarbonyl, C₁₋₆ alkylaminocarbonyl, C₁₋₆acylamino, C₁-C₆ dialkylaminocarbonyl, halogen, cyano, nitro, aryl and heteroaryl;
C₁₋₁₀ alkyl, C₃₋₁₀ cycloalkyl, 3-10 membered saturated heterocyclic ring, each optionally independently substituted with 1-3 substituents chosen from C₁₋₁₀ alkyl, C₁₋₁₀ alkoxy, C₃₋₁₀ cycloalkyl, C₁-₆ acyl, cyano, phenyl, oxo, hydroxyl and halogen; each **R¹** and **R¹** substituent where possible is optionally substituted with 1 to 3 halogen atoms;
**R² and R³** are independently hydrogen or C₁-₆ alkyl; or R² and R³ together with the carbon which they are attached to form a 3- to 6-membered cycloalkyl or heterocyclic ring;
**R⁴** is heteroaryl optionally independently substituted with 1 to 3 substituents chosen from C₁₋₆ alkyl (which is optionally substituted with 1 to 3 halogen atoms), hydroxyl, halogen and cyano,
**R⁵** is aryl, heteroaryl or C₃₋₁₀ cycloalkyl each optionally independently substituted with 1 to 3 substituents chosen from C₁₋₆ alkyl (which is optionally substituted with 1 to 3 halogen atoms or with hydroxy), C₁₋₆ alkoxy (which is optionally substituted with 1 to 3 halogen atoms), C₁₋₆ cycloalkyl, phenoxy, halogen, cyano, phenyl (which is optionally substituted with 1 to 2 halogen atoms or C₁₋₄ alkyl optionally substituted with halogen), thienyl (which is optionally substituted with 1 to 2 halogen atoms or C₁₋₄ alkyl optionally substituted with halogen) and pyridinyl (which is optionally substituted with 1 to 2 C₁₋₄ alkyl optionally substituted with halogen);
n is 0, 1, 2 for 3
or a pharmaceutically acceptable salt thereof.

In a first subgeneric aspect, the invention provides compounds of the formula I wherein,
**R¹** is phenyl, naphthyl each optionally independently substituted with 1 to 3 substituents chosen from C₁-₆ alkyl, C₃-₆ cycloalkyl, C₁-₆ alkoxy, C₁-₆ alkylthio, C₁-₆ alkylsulfonyl,
C₁-₆ alkoxycarbonyl, C₁-₆ alkylaminocarbonyl, C₁-₆acylamino, C₁-₆ dialkylaminocarbonyl, halogen, cyano, nitro and phenyl;
C₁₋₁₀ alkyl, C₃₋₁₀ cycloalkyl, heterocyclic ring chosen from tetrahydropyranyl, tetrahydrofuranyl, morpholinyl, piperidinyl, piperazinyl and pyrrolidinyl, each optionally independently substituted with 1-3 substituents chosen from C₁₋₁₀ alkyl, C₃₋₁₀ cycloalkyl, C₁-₆ acyl, cyano, phenyl, oxo, hydroxyl and halogen; each **R¹** and **R¹** substituent where possible is optionally substituted with 1 to 3 halogen atoms;
**R² and R³** are independently hydrogen or C₁-₆ alkyl or R² and R³ together with the carbon which they are attached to form a 3- to 6-membered cycloalkyl;
**R⁴** is furanyl, oxazolyl, isoxazolyl, oxadiazolyl, triazolyl, thiazolyl, pyrazolyl, pyrrolyl, imidazolyl, thienyl or thiadiazolyl optionally substituted with 1 to 3 substituents chosen from C₁-₆ alkyl (which is optionally substituted with 1 to 3 halogen atoms), hydroxyl, halogen and cyano;
**R⁵** is aryl, C₃₋₁₀ cycloalkyl, furanyl, pyranyl, oxazolyl, isoxazolyl, oxadiazolyl, triazolyl, isothiazoyl, thiazolyl, pyrazolyl, pyrrolyl, imidazolyl, thienyl, thiadiazolyl, pyridinyl, pyrimidinyl, pyridazinyl, pyrazinyl or triazinyl, each optionally independently substituted with 1 to 3 substituents chosen from C₁-₆ alkyl (which is optionally substituted with 1 to 3 halogen atoms or with a heterocyclyl group), C₁-₆ alkoxy (which is optionally substituted with 1 to 3 halogen atoms), C₁-₆ cycloalkyl, phenoxy, halogen, cyano, dimethylaminoalkyl, phenyl (which is optionally substituted with 1 to 2 halogen atoms or C₁-₄ alkyl optionally substituted with halogen), thienyl (which is optionally substituted with 1 to 2 halogen atoms or C₁₋₄ alkyl optionally substituted with halogen), and pyridinyl (which is optionally substituted with 1 to 2 C₁₋₄ alkyl optionally substituted with halogen);
n is 0, 1 or 2

In a further subgeneric aspect, the invention provides compounds of the formula I wherein
**R¹** is phenyl optionally independently substituted with 1 to 3 substituents chosen from
C₁₋₆ alkyl, C₃₋₆ cycloalkyl, C₁₋₆ alkoxy, C₁₋₆ alkylthio, C₁₋₆ alkylsulfonyl, C₁₋₆ alkoxycarbonyl, C₁₋₆ alkylaminocarbonyl, C₁₋₆ acylamino, C₁₋₆ dialkylaminocarbonyl, halogen, cyano and nitro; C₁₋₁₀ alkyl, C₃₋₇ cycloalkyl, heterocyclic ring chosen from tetrahydropyranyl, and tetrahydrofuranyl, each optionally independently substituted with 1-3 substituents chosen from C₁₋₆ alkyl, C₃₋₇ cycloalkyl, C₁₋₆ acyl, cyano, phenyl, oxo, hydroxyl and halogen; each **R¹** and **R¹** substituent where possible is optionally substituted with 1 to 3 halogen atoms;
**R² and R³** are independently hydrogen or C₁-₅ alkyl or R² and R³ together with the carbon which they are attached to form a 3- to 5-membered cycloalkyl;
**R⁴** is oxazolyl, isoxazolyl, oxadiazolyl, triazolyl, thiazolyl, pyrazolyl, pyrrolyl, imidazolyl or thiadiazolyl optionally substituted with 1 to 3 substituents chosen from C₁-₆ alkyl (which is optionally substituted with 1 to 3 halogen atoms), hydroxyl, halogen and cyano,
**R⁵** is C₃₋₁₀ cycloalkyl, oxazolyl, isoxazolyl, oxadiazolyl, triazolyl, thiazolyl, pyrazolyl, pyrrolyl, imidazolyl or thiadiazolyl, each independently substituted with 1 to 3 substituents chosen from C₁-₆ alkyl (which is optionally substituted with 1 to 3 halogen atoms or with a heterocyclyl group), C₁-₆ alkoxy (which is optionally substituted with 1 to 3 halogen atoms), C₁-₆ cycloalkyl, phenoxy, halogen, cyano, dimethylaminoalkyl, phenyl (which is optionally substituted with 1 to 2 halogen atoms or C₁-₄ alkyl optionally substituted with halogen), thienyl (which is optionally substituted with 1 to 2 halogen atoms or C₁-₄ alkyl optionally substituted with halogen), and pyridinyl (which is optionally substituted with 1 to 2 C₁-₄ alkyl optionally substituted with halogen);
n is 0, 1 or 2

In another subgeneric aspect, the invention provides compounds of the formula I wherein,
**R¹** is phenyl optionally independently substituted with 1-3 substituents chosen from C₁₋₃ alkyl, C₃₋₆ cycloalkyl, cyano, phenyl, and halogen,
or
**R¹** is C₁₋₆ alkyl, C₃₋₆ cycloalkyl or tetrahydropyranyl optionally substituted with 1-3 substituents chosen from C₁₋₆ alkyl, C₃₋₇ cycloalkyl, C₁₋₆ cyano, phenyl, oxo, hydroxyl and halogen; each **R¹** and **R¹** substituent where possible is optionally substituted with 1 to 3 halogen atoms;
**R² and R³** are independently hydrogen or C₁-₄ alkyl or R² and R³ together with the carbon which they are attached to form a 3- to 4-membered cycloalkyl;
**R⁴** is oxazolyl, oxadiazolyl, triazolyl, imidazolyl or thiadiazolyl optionally substituted with 1 to 3 substituents chosen from C₁-₆ alkyl (which is optionally substituted with 1 to 3 halogen atoms), hydroxyl, halogen and cyano,
**R⁵** is cyclohexyl, isoxazolyl or pyrazolyl, each independently substituted with 1 to 3 substituents chosen from C₁-₆ alkyl (which is optionally substituted with 1 to 3 halogen atoms), C₁-₆ alkoxy (which is optionally substituted with 1 to 3 halogen atoms), C₁-₆ cycloalkyl, phenoxy, halogen, cyano, dimethylaminoalkyl, phenyl (which is optionally substituted with 1 to 2 halogen atoms or C₁-₄ alkyl optionally substituted with halogen), thienyl (which is optionally substituted with 1 to 2 halogen atoms or C₁-₄ alkyl optionally substituted with halogen), and pyridinyl (which is optionally substituted with 1 to 2 C₁₋₄ alkyl optionally substituted with halogen).
n is 0, 1 or 2

In a still further subgeneric aspect, the invention provides compounds of the formula I wherein, **R¹** is C₁₋₄ alkyl, C₃₋₆ cycloalkyl and phenyl; each optionally independently substituted with 1-3 substituents chosen from C₁₋₃ alkyl, C₃₋₆ cycloalkyl, cyano, phenyl, and halogen, and n is 0 or **R¹** is tetrahydropyranyl
and n is 0 or 1;
**R² and R³** are independently hydrogen or C₁-₃alkyl or R² and R³ together with the carbon which they are attached to form cyclopropyl;
**R⁴** is imidazolyl, oxazolyl, oxadiazolyl, triazoyl or thiadiazolyl, each optionally independently substituted with one substituent chosen from C₁₋₆ alkyl, hydroxyl and halogen;
**R⁵** is cyclohexyl,, isoxazolyl or pyrazolyl, each independently substituted with 1 to 3 substituents chosen from C₁₋₆ alkyl (which is optionally substituted with 1 to 3 halogen atoms).

In another subgeneric aspect, the invention provides compounds of the formula I wherein,
**R¹** is phenyl optionally independently substituted with 1 to 3 substituents chosen from C₁-₃ alkyl (which is optionally substituted with 1 to 3 halogen atoms), halogen and cyano. or **R¹** is C₁₋₅ alkyl or cyclohexyl, each optionally independently substituted with 1 to 3 substituents chosen from C₁-₂ alkyl (which is optionally substituted with 1 to 3 atoms), hydroxyl, fluoro and chloro.

In a still further subgeneric aspect, the invention provides compounds of the formula I wherein, **R¹** is phenyl optionally independently substituted with 1 to 3 substituents chosen from C₁-₃ alkyl (which is optionally substituted with 1 to 3 halogen atoms), halogen and cyano
or **R¹** is C₁₋₅ alkyl or cyclohexyl, each optionally independently substituted with 1 to 3 substituents chosen from C₁-₂ alkyl (which is optionally substituted with 1 to 3 atoms), hydroxyl, fluoro and chloro;
**R² and R³** are methyl or R² and R³ together with the carbon which they are attached to form cyclopropyl.

In another subgeneric aspect, the invention provides compounds of the formula IA: 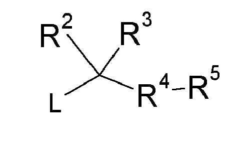 wherein for the formula (IA) 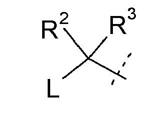 is chosen independently from members of column A in Table I, and 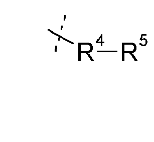 is chosen independently from members of column B in Table I:

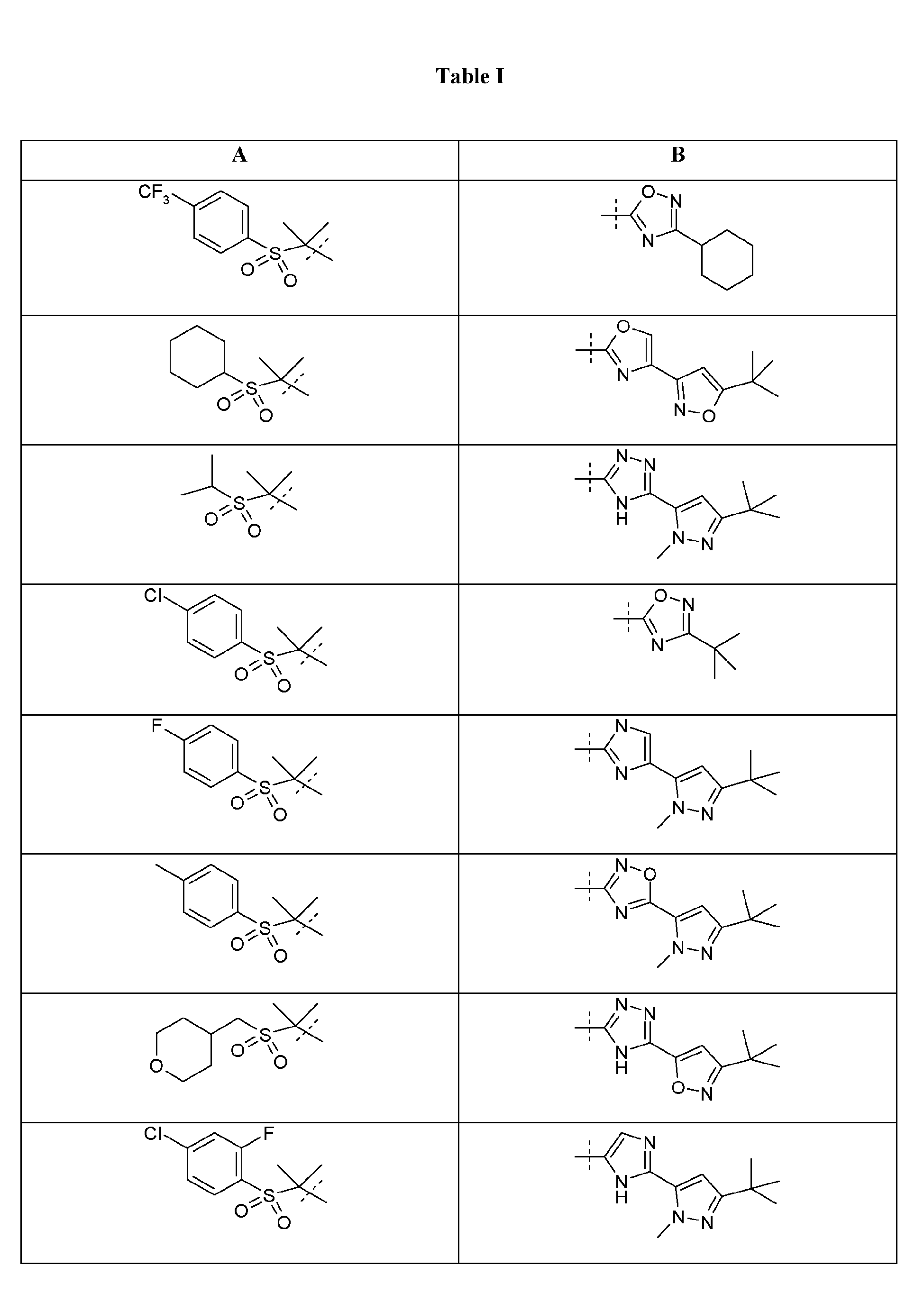

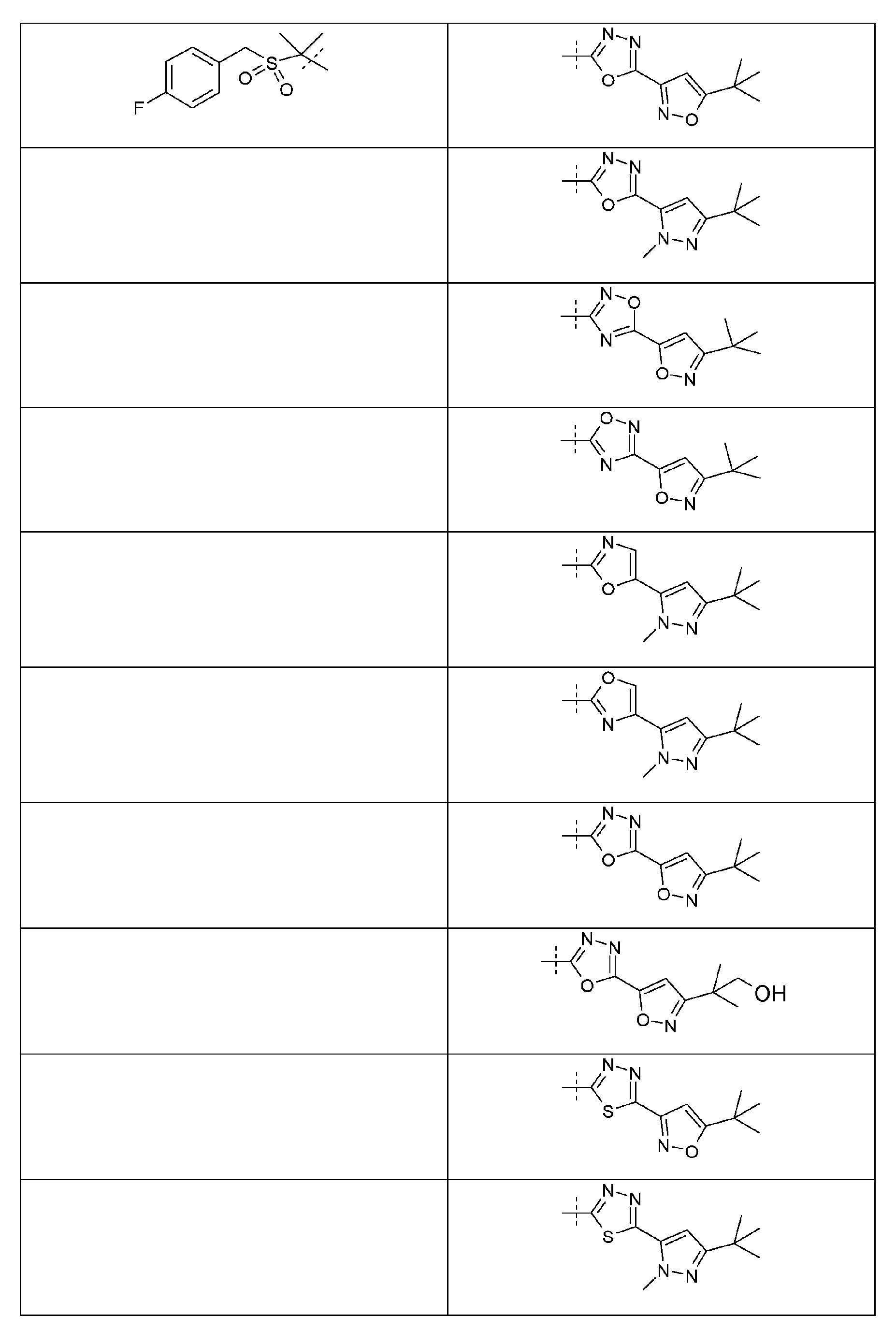

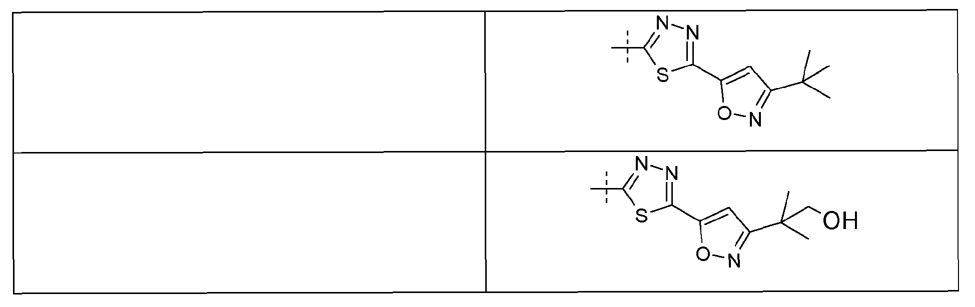

or a pharmaceutically acceptable salt thereof.

In another embodiment, the invention provides compounds in Table II which can be made in view of the general schemes, examples and methods known in the art.

**Table II**

| Structure | Name |
|---|---|
| 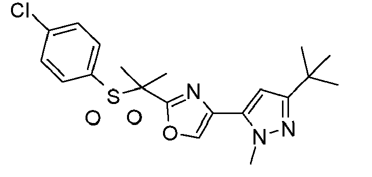 | 4-(5-tert-butyl-2-methyl-2H-pyrazol-3-yl)-2-[1-(4-chloro-benzenesulfonyl)-1-methyl-ethyl]-oxazole |
| 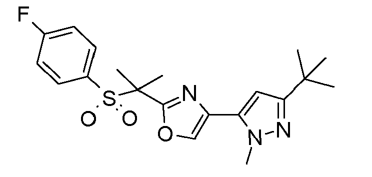 | 4-(5-tert-butyl-2-methyl-2H-pyrazol-3-yl)-2-[1-(4-fluoro-benzenesulfonyl)-1-methyl-ethyl]-oxazole |
| 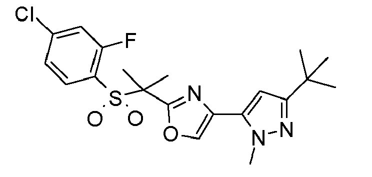 | 4-(5-tert-butyl-2-methyl-2H-pyrazol-3-yl)-2-[1-(4-chloro-2-fluoro-benzenesulfonyl)-1-methyl-ethyl]-oxazole |
| 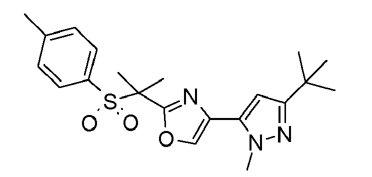 | 4-(5-tert-butyl-2-methyl-2H-pyrazol-3-yl)-2-[1-methyl-1-(toluene-4-sulfonyl)-ethyl]-oxazole |
| 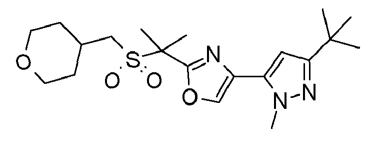 | 4-(5-tert-butyl-2-methyl-2H-pyrazol-3-yl)-2-[1-methyl-1-(tetrahydro-pyran-4-ylmethanesulfonyl)-ethyl]-oxazole |
| 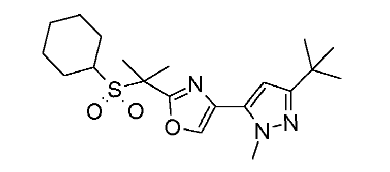 | 4-(5-tert-butyl-2-methyl-2H-pyrazol-3-yl)-2-(1-cyclohexanesulfonyl-1-methyl-ethyl)-oxazole |
| 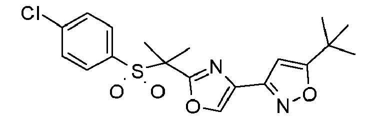 | 5-tert-butyl-3-{2-[1-(4-chloro-benzenesulfonyl)-1-methyl-ethyl]-oxazol-4-yl}-isoxazole |
| 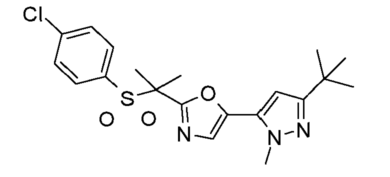 | 5-(5-tert-butyl-2-methyl-2H-pyrazol-3-yl)-2-[1-(4-chloro-benzenesulfonyl)-1-methyl-ethyl]-oxazole |
| 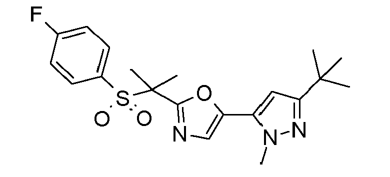 | 5-(5-tert-butyl-2-methyl-2H-pyrazol-3-yl)-2-[1-(4-fluoro-benzenesulfonyl)-1-methyl-ethyl]-oxazole |
| 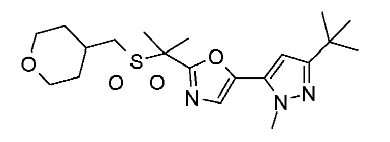 | 5-(5-tert-butyl-2-methyl-2H-pyrazol-3-yl)-2-[1-methyl-1-(tetrahydro-pyran-4-ylmethanesulfonyl)-ethyl]-oxazole |
| 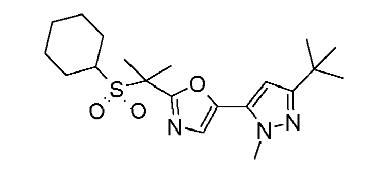 | 4-(5-tert-butyl-2-methyl-2H-pyrazol-3-yl)-2-(1cyclohexanesulfonyl-1-methyl-ethyl)-oxazole |
| 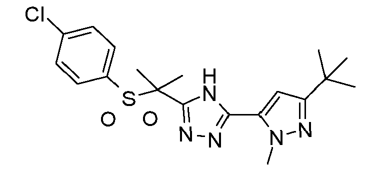 | 3-(5-tert-butyl-2-methyl-2H-pyrazol-3-yl)-5-[1-(4-chloro-benzenesulfonyl)-1-methyl-ethyl]-4H-[1,2,4]triazole |
| 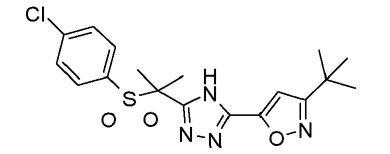 | 3-(3-tert-butyl-isoxazol-5-yl)-5-[1-(4-chloro-benzenesulfonyl)-1-methyl-ethyl]-4H-[1,2,4]triazole |
| 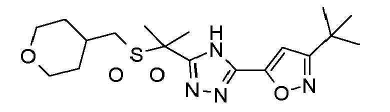 | 3-(3-tert-butyl-isoxazol-5-yl)-5-[1-methyl-1-(tetrahydro-pyran-4-ylmethanesulfonyl)-ethyl]-4H-[1,2,4]triazole |
| 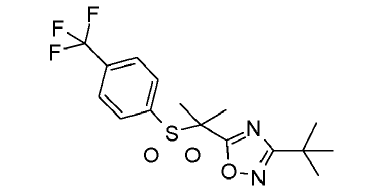 | 3-tert-butyl-5-[1-methyl-1-(4-trifluoromethyl-benzenesulfonyl)-ethyl]-[1,2,4]oxadiazole |
| 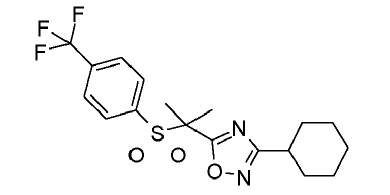 | 3-cyclohexyl-5-[1-methyl-1-(4-trifl uoromethyl-benzenesulfonyl)-ethyl]-[1,2,4]oxadiazole |
| 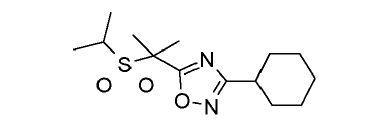 | 3-cyclohexyl-5-[1-methyl-1-(propane-2-sulfonyl)-ethyl]-[1,2,4]oxadiazole |
| 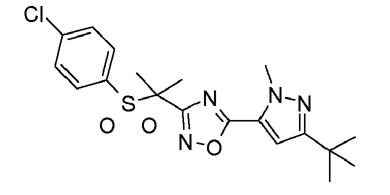 | 5-(5-tert-butyl-2-methyl-2H-pyrazol-3-yl)-3-[1-(4-chloro-benzenesulfonyl)-1-methyl-ethyl]-[1,2,4]oxadiazole |
| 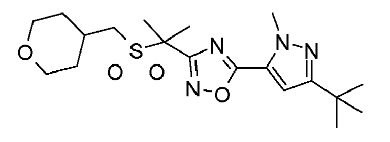 | 5-(5-tert-butyl-2-methyl-2H-pyrazol-3-yl)-3-[1-methyl-1-(tetrahydro-pyran-4-ylmethanesulfonyl)-ethyl]-[1,2,4]oxadiazole |
| 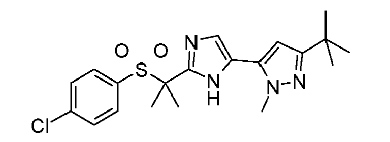 | 3-tert-butyl-5-{2-[1-(4-chloro-benzenesulfonyl)-1-methyl-ethyl]-3H-imidazol-4-yl}-1-methyl-1H-pyrazole |
| 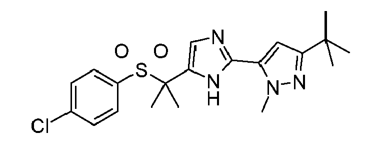 | 3-tert-butyl-5-{5-[1-(4-chloro-benzenesulfonyl)-1-methyl-ethyl]-1H-imidazol-2-yl}-1-methyl-1H-pyrazole |
| 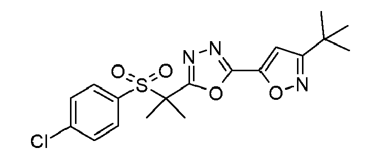 | 2-(3-tert-butyl-isoxazol-5-yl)-5-[1-(4-chloro-benzenesulfonyl)-1-methyl-ethyl]-[1,3,4]oxadiazole |
| 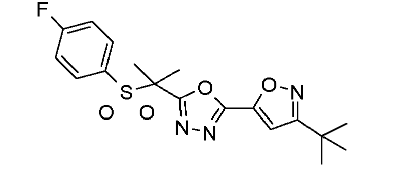 | 2-(3-tert-butyl-isoxazol-5-yl)-5-[1-(4-fl uoro-benzenesulfonyl)-1-methyl-ethyl]-[1,3,4]oxadiazole |
| 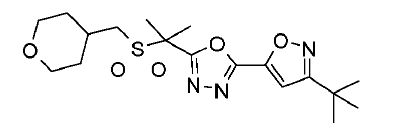 | 2-(3-tert-butyl-isoxazol-5-yl)-5-[1-methyl-1-(tetrahydro-pyran-4-ylmethanesulfonyl)-ethyl]-[1,3,4]oxadiazole |
| 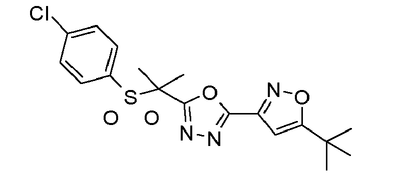 | 2-(5-tert-butyl-isoxazol-3-yl)-5-[1-(4-chloro-benzenesulfonyl)-1-methyl-ethyl]-[1,3,4]oxadiazole |
| 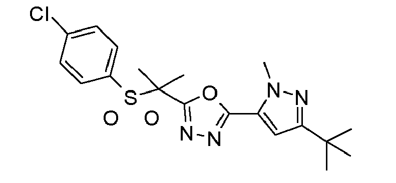 | 2-(5-tert-butyl-2-methyl-2H-pyrazol-3-yl)-5-[1-(4-chloro-benzenesulfonyl)-1-methyl-ethyl]-[1,3,4]oxadiazole |
| 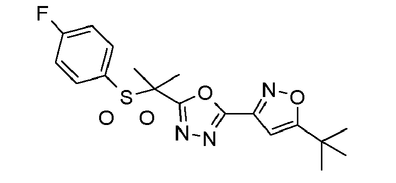 | 2-(5-tert-butyl-isoxazol-3-yl)-5-[1-(4-fl uoro-benzenesulfonyl)-1-methyl-ethyl]-[1,3,4]oxadiazole |
| 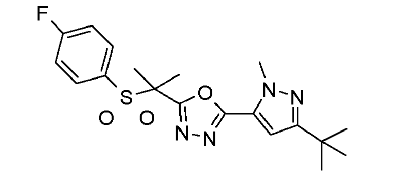 | 2-(5-tert-butyl-2-methyl-2H-pyrazol-3-yl)-5-[1-(4-fluoro-benzenesulfonyl)-1-methyl-ethyl]-[1,3,4]oxadiazole |
| 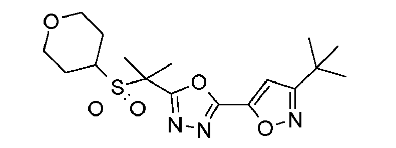 | 2-(3-tert-butyl-isoxazol-5-yl)-5-[1-methyl-1-(tetrahydro-pyran-4-sulfonyl)-ethyl]-[1,3,4]oxadiazole |
| 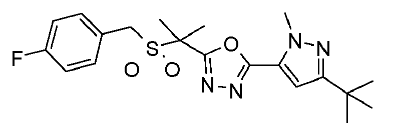 | 2-(5-tert-butyl-2-methyl-2H-pyrazol-3-yl)-5-[1-(4-fluoro-phenylmethanesulfonyl)-1-methyl-ethyl]-[1,3,4]oxadiazole |
| 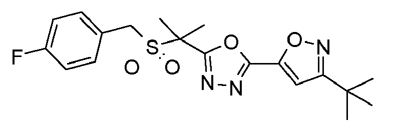 | 2-(3-tert-butyl-isoxazol-5-yl)-5-[1-(4-fl uoro-phenylmethanesulfonyl)-1-methyl-ethyl]-[1,3,4]oxadiazole |
| 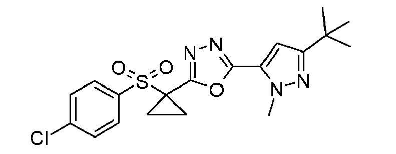 | 2-(5-tert-butyl-2-methyl-2H-pyrazol-3-yl)-5-[1-(4-chloro-benzenesulfonyl)-cyclopropyl]-[1,3,4]oxadiazole |
| 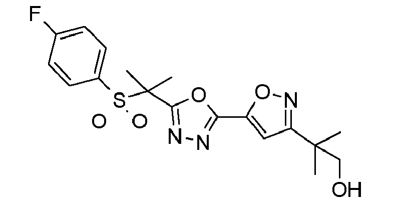 | 2-(5-{5-[1-(4-fluoro-benzenesulfonyl)-1-methyl-ethyl]-[1,3,4]oxadiazol-2-yl}-isoxazol-3-yl)-2-methyl-propan-1-ol |
| 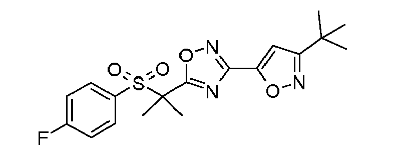 | 3-(3-tert-butyl-isoxazol-5-yl)-5-[1-(4-fl uoro-benzenesulfonyl)-1-methyl-ethyl]-[1,2,4]oxadiazole |
| 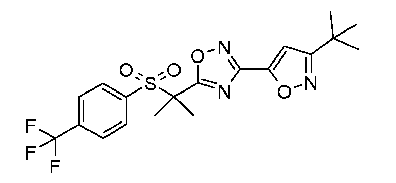 | 3-(3-tert-butyl-isoxazol-5-yl)-5-[1-methyl-1-(4-trifluoromethyl-benzenesulfonyl)-ethyl]-[1,2,4]oxadiazole |
| 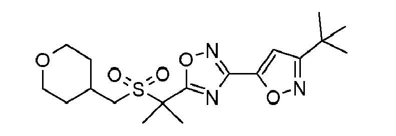 | 3-(3-tert-butyl-isoxazol-5-yl)-5-[1-methyl-1-(tetrahydro-pyran-4-ylmethanesulfonyl)-ethyl]-[1,2,4]oxadiazole |
| 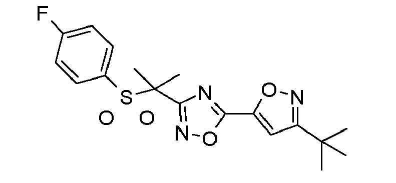 | 5-(3-tert-butyl-isoxazol-5-yl)-3-[1-(4-fl uoro-benzenesulfonyl)-1-methyl-ethyl]-[1,2,4]oxadiazole |
| 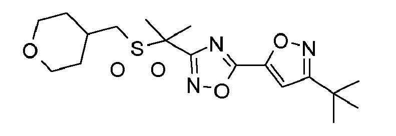 | 5-(3-tert-butyl-isoxazol-5-yl)-3-[1-methyl-1-(tetrahydro-pyran-4-ylmethanesulfonyl)-ethyl]-[1,2,4]oxadiazole |
| 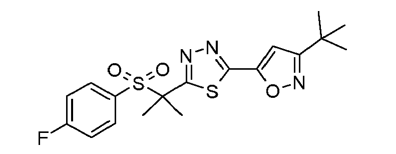 | 2-(3-tert-butyl-isoxazol-5-yl)-5-[1-(4-fl uoro-benzenesulfonyl)-1-methyl-ethyl]-[1,3,4]thiadiazole |
| 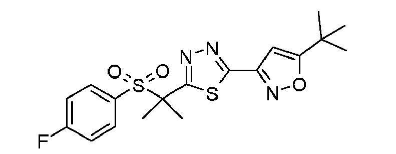 | 2-(5-tert-butyl-isoxazol-3-yl)-5-[1-(4-fl uoro-benzenesulfonyl)-1-methyl-ethyl]-[1,3,4]thiadiazole |
| 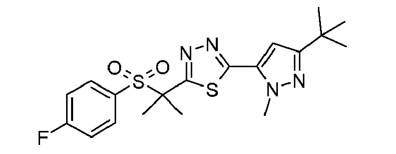 | 2-(5-tert-butyl-2-methyl-2H-pyrazol-3-yl)-5-[1-(4-fluoro-benzenesulfonyl)-1-methyl-ethyl]-[1,3,4]thiadiazole |
| 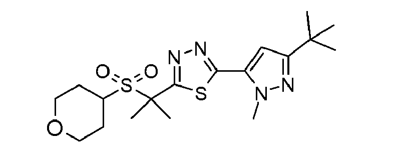 | 2-(5-tert-butyl-2-methyl-2H-pyrazol-3-yl)-5-[1-methyl-1-(tetrahydro-pyran-4-sulfonyl)-ethyl]-[1,3,4]thiadiazole |
| 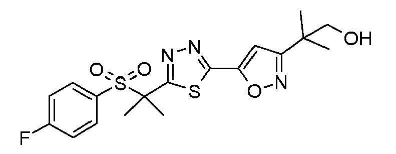 | 2-(5-{5-[1-(4-fluoro-benzenesulfonyl)-1-methyl-ethyl]-[1,3,4]thiadiazol-2-yl}-isoxazol-3-yl)-2-methyl-propan-1-ol |
| 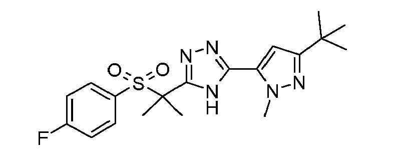 | 3-(5-tert-butyl-2-methyl-2H-pyrazol-3-yl)-5-[1-(4-fluoro-benzenesulfonyl)-1-methyl-ethyl]-4H-[1,2,4]triazole |
| 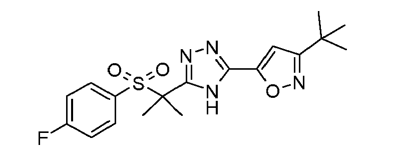 | 3-(3-tert-butyl-isoxazol-5-yl)-5-[1-(4-fl uoro-benzenesulfonyl)-1-methyl-ethyl]-4H-[1,2,4]triazole |
| 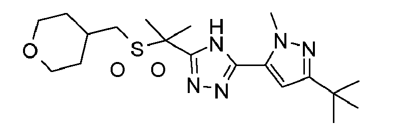 | 3-(5-tert-butyl-2-methyl-2H-pyrazol-3-yl)-5-[1-methyl-1-(tetrahydro-pyran-4-ylmethanesulfonyl)-ethyl]-4H-[1,2,4]triazole |
| 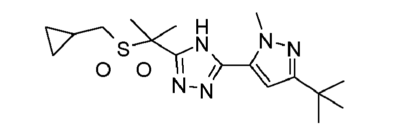 | 3-(5-tert-butyl-2-methyl-2H-pyrazol-3-yl)-5-(1-cyclopropylmethanesulfonyl-1-methyl-ethyl)-4H-[1,2,4]triazole |
| 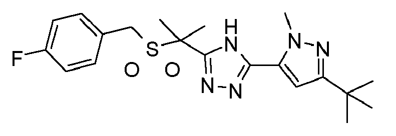 | 3-(5-tert-butyl-2-methyl-2H-pyrazol-3-yl)-5-[1-(4-fluoro-phenylmethanesulfonyl)-1-methyl-ethyl]-4H-[1,2,4]triazole |
| 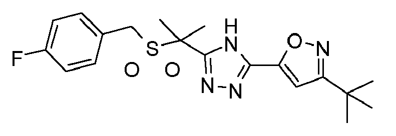 | 3-(3-tert-Butyl-isoxazol-5-yl)-5-[1-(4-fl uoro-phenylmethanesulfonyl)-1-methyl-ethyl]-4H-[1,2,4]triazole |
| 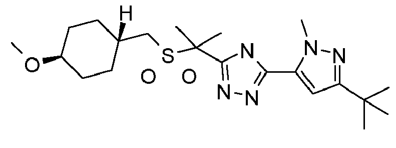 | 3-(5-tert-butyl-2-methyl-2H-pyrazol-3-yl)-5-[1-(4-methoxy-cyclohexylmethanesulfonyl)-1-methyl-ethyl]-4H-[1,2,4]triazole |
| 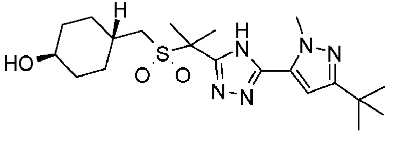 | 4-{2-[5-(5-tert-butyl-2-methyl-2H-pyrazol-3-yl)-4H-[1,2,4]triazol-3-yl]-propane-2-sulfonylmethyl}-cyclohexanol |

or a pharmaceutically acceptable salt thereof.

Of the above compounds, the following are preferred CB2 agonists:

**Table III**

| Compound | CB2 EC50 (nM) |
|---|---|
| 4-(5-tert-Butyl-2-methyl-2H-pyrazol-3-yl)-2-[1-(4-chloro-benzenesulfonyl)-1-methyl-ethyl]-oxazole | 0.92 |
| 4-(5-tert-Butyl-2-methyl-2H-pyrazol-3-yl)-2-[1-(4-fluoro-benzenesulfonyl)-1-methyl-ethyl]-oxazole | 0.35 |
| 4-(5-tert-Butyl-2-methyl-2H-pyrazol-3-yl)-2-[1-(4-chloro-2-fluoro-benzenesulfonyl)-1-methyl-ethyl]-oxazole | 3.9 |
| 4-(5-tert-Butyl-2-methyl-2H-pyrazol-3-yl)-2-[1-methyl-1-(toluene-4-sulfonyl)-ethyl]-oxazole | 12 |
| 4-(5-tert-Butyl-2-methyl-2H-pyrazol-3-yl)-2-[1-methyl-1-(tetrahydro-pyran-4-ylmethanesulfonyl)-ethyl]-oxazole | 145 |
| 4-(5-tert-Butyl-2-methyl-2H-pyrazol-3-yl)-2-(1-cyclohexanesulfonyl-1-methyl-ethyl)-oxazole | 371 |
| 5-tert-Butyl-3-{2-[1-(4-chloro-benzenesulfonyl)-1-methyl-ethyl]-oxazol-4-yl}-isoxazole | 0.90 |
| 5-(5-tert-Butyl-2-methyl-2H-pyrazol-3-yl)-2-[1-(4-chloro-benzenesulfonyl)-1-methyl-ethyl]-oxazole | 22 |
| 5-(5-tert-Butyl-2-methyl-2H-pyrazol-3-yl)-2-[1-(4-fluoro-benzenesulfonyl)-1-methyl-ethyl]-oxazole | 5.8 |
| 5-(5-tert-Butyl-2-methyl-2H-pyrazol-3-yl)-2-(1-cyclohexanesulfonyl-1-methyl-ethyl)-oxazole | 417 |
| 3-(5-tert-Butyl-2-methyl-2H-pyrazol-3-yl)-5-[1-(4-chloro-benzenesulfonyl)-1-methyl-ethyl]-4H-[1,2,4]triazole | 12 |
| 3-(3-tert-Butyl-isoxazol-5-yl)-5-[1-methyl-1-(tetrahydro- | 159 |
| pyran-4-ylmethanesulfonyl)-ethyl]-4H-[1,2,4]triazole | |
| 3-Cyclohexyl-5-[1-methyl-1-(4-trifluoromethyl-benzenesulfonyl)-ethyl]-[1,2,4]oxadiazole | 12 |
| 5-(5-tert-Butyl-2-methyl-2H-pyrazol-3-yl)-3-[1-(4-chloro-benzenesulfonyl)-1-methyl-ethyl]-[1,2,4]oxadiazole | 2.7 |
| 5-(5-tert-Butyl-2-methyl-2H-pyrazol-3-yl)-3-[1-methyl-1-(tetrahydro-pyran-4-ylmethanesulfonyl)-ethyl]-[1,2,4]oxadiazole | 9.2 |
| 2-(3-tert-Butyl-isoxazol-5-yl)-5-[1-(4-chloro-benzenesulfonyl)-1-methyl-ethyl]-[1,3,4]oxadiazole | 5.3 |
| 2-(3-tert-Butyl-isoxazol-5-yl)-5-[1-methyl-1-(tetrahydro-pyran-4-ylmethanesulfonyl)-ethyl]-[1,3,4]oxadiazole | 48 |
| 2-(5-tert-Butyl-isoxazol-3-yl)-5-[1-(4-chloro-benzenesulfonyl)-1-methyl-ethyl]-[1,3,4]oxadiazole | 6.9 |
| 2-(5-tert-Butyl-2-methyl-2H-pyrazol-3-yl)-5-[1-(4-chloro-benzenesulfonyl)-1-methyl-ethyl]-[1,3,4]oxadiazole | 9.0 |
| 2-(5-tert-tutyl-isoxazol-3-yl)-5-[1-(4-fluoro-benzenesulfonyl)-1-methyl-ethyl]-[1,3,4]oxadiazole | 35 |
| 2-(5-tert-butyl-2-methyl-2H-pyrazol-3-yl)-5-[1-(4-fluoro-benzenesulfonyl)-1-methyl-ethyl]-[1,3,4]oxadiazole | 16 |
| 2-(3-tert-butyl-isoxazol-5-yl)-5-[1-methyl-1-(tetrahydro-pyran-4-sulfonyl)-ethyl]-[1,3,4]oxadiazole | 27 |
| 2-(3-tert-butyl-isoxazol-5-yl)-5-[1-(4-fluoro-phenylmethanesulfonyl)-1-methyl-ethyl]-[1,3,4]oxadiazole | 162 |
| 2-(5-tert-butyl-2-methyl-2H-pyrazol-3-yl)-5-[1-(4-chloro-benzenesulfonyl)-cyclopropyl]-[1,3,4]oxadiazole | 3.7 |
| 3-(3-tert-butyl-isoxazol-5-yl)-5-[1-(4-fluoro-benzenesulfonyl)-1-methyl-ethyl]-[1,2,4]oxadiazole | 1.8 |
| 3-(3-tert-butyl-isoxazol-5-yl)-5-[1-methyl-1-(4-trifluoromethyl-benzenesulfonyl)-ethyl]-[1,2,4]oxadiazole | 5.9 |
| 3-(3-tert-butyl-isoxazol-5-yl)-5-[1-methyl-1-(tetrahydro-pyran-4-ylmethanesulfonyl)-ethyl]-[1,2,4]oxadiazole | 15 |
| 5-(3-tert-butyl-isoxazol-5-yl)-3-[1-(4-fluoro-benzenesulfonyl)-1-methyl-ethyl]-[1,2,4]oxadiazole | 1.0 |
| 5-(3-tert-butyl-isoxazol-5-yl)-3-[1-methyl-1-(tetrahydro-pyran-4-ylmethanesulfonyl)-ethyl]-[1,2,4]oxadiazole | 210 |
| 2-(3-tert-butyl-isoxazol-5-yl)-5-[1-(4-fluoro-benzenesulfonyl)-1-methyl-ethyl]-[1,3,4]thiadiazole | 6.7 |
| 2-(5-tert-butyl-isoxazol-3-yl)-5-[1-(4-fluoro-benzenesulfonyl)-1-methyl-ethyl]-[1,3,4]thiadiazole | 17 |
| 2-(5-tert-butyl-2-methyl-2H-pyrazol-3-yl)-5-[1-(4-fluoro-benzenesulfonyl)-1-methyl-ethyl]-[1,3,4]thiadiazole | 2.5 |
| 2-(5-tert-butyl-2-methyl-2H-pyrazol-3-yl)-5-[1-methyl-1-(tetrahydro-pyran-4-sulfonyl)-ethyl]-[1,3,4]thiadiazole | 117 |
| 2-(5-{5-[1-(4-fluoro-benzenesulfonyl)-1-methyl-ethyl]-[1,3,4]thiadiazol-2-yl}-isoxazol-3-yl)-2-methyl-propan-1-ol | 175 |
| 3-(5-tert-butyl-2-methyl-2H-pyrazol-3 -yl)-5 - [1-(4-fluoro-benzenesulfonyl)-1-methyl-ethyl]-4H-[1,2,4]triazole | 2.0 |
| 3-(3-tert-butyl-isoxazol-5-yl)-5-[1-(4-fluoro-benzenesulfonyl)-1-methyl-ethyl]-4H-[1,2,4]triazole | 255 |
| 3-(5-tert-butyl-2-methyl-2H-pyrazol-3-yl)-5-[1-methyl-1-(tetrahydro-pyran-4-ylmethanesulfonyl)-ethyl]-4H-[1,2,4]triazole | 414 |
| 3-(5-tert-butyl-2-methyl-2H-pyrazol-3 -yl)-5 - [1-(4-fluoro-phenylmethanesulfonyl)-1-methyl-ethyl]-4H-[1,2,4]triazole | 129 |
| 3-(3-tert-Butyl-isoxazol-5-yl)-5-[1-(4-fluoro-phenylmethanesulfonyl)-1-methyl-ethyl]-4H-[1,2,4]triazole | 293 |
| 4- {2-[5-(5-tert-butyl-2-methyl-2H-pyrazol-3-yl)-4H-[1,2,4]triazol-3-yl]-propane-2-sulfonylmethyl}-cyclohexanol | 459 |

In all the compounds disclosed hereinabove in this application, in the event the nomenclature is in conflict with the structure, it shall be understood that the compound is defined by the structure. The invention also relates to pharmaceutical preparations, containing as active substance one or more compounds of the invention, or the pharmaceutically acceptable derivatives thereof, optionally combined with conventional excipients and/or carriers.

Compounds of the invention also include their isotopically-labelled forms. An isotopically-labelled form of an active agent of a combination of the present invention is identical to said active agent but for the fact that one or more atoms of said active agent have been replaced by an atom or atoms having an atomic mass or mass number different from the atomic mass or mass number of said atom which is usually found in nature. Examples of isotopes which are readily available commercially and which can be incorporated into an active agent of a combination of the present invention in accordance with well established procedures, include isotopes of hydrogen, carbon, nitrogen, oxygen, phosphorous, fluorine and chlorine, e.g., ²H, ³H, ¹³C, ¹⁴C, ¹⁵N, ¹⁸O, ¹⁷O, ³¹P, ³²P, ³⁵S, ¹⁸F, and ³⁶Cl, respectively. An active agent of a combination of the present invention, a prodrug thereof, or a pharmaceutically acceptable salt of either which contains one or more of the above-mentioned isotopes and/or other isotopes of other atoms is contemplated to be within the scope of the present invention.

The invention includes the use of any compounds of described above containing one or more asymmetric carbon atoms may occur as racemates and racemic mixtures, single enantiomers, diastereomeric mixtures and individual diastereomers. Isomers shall be defined as being enantiomers and diastereomers. All such isomeric forms of these compounds are expressly included in the present invention. Each stereogenic carbon may be in the R or S configuration, or a combination of configurations.

Some of the compounds of the invention can exist in more than one tautomeric form. The invention includes methods using all such tautomers.

All terms as used herein in this specification, unless otherwise stated, shall be understood in their ordinary meaning as known in the art. For example, "C₁₋₄alkoxy" is a C₁₋₄alkyl with a terminal oxygen, such as methoxy, ethoxy, propoxy, butoxy. All alkyl, alkenyl and alkynyl groups shall be understood as being branched or unbranched where structurally possible and unless otherwise specified. Other more specific definitions are as follows:
Carbocyclic or cycloalkyl groups include hydrocarbon rings containing from three to twelve carbon atoms. These carbocyclic or cycloalkyl groups may be either aromatic or non-aromatic ring systems. The non-aromatic ring systems may be mono- or polyunsaturated. Preferred carbocycles include but are not limited to cyclopropyl, cyclobutyl, cyclopentyl, cyclopentenyl, cyclohexyl, cyclohexenyl, cycloheptanyl, cycloheptenyl, phenyl, indanyl, indenyl, benzocyclobutanyl, dihydronaphthyl, tetrahydronaphthyl, naphthyl, decahydronaphthyl, benzocycloheptanyl and benzocycloheptenyl. Certain terms for cycloalkyl such as cyclobutanyl and cyclobutyl shall be used interchangeably.

The term "heterocycle" refers to a stable nonaromatic 4-8 membered (but preferably, 5 or 6 membered) monocyclic or nonaromatic 8-11 membered bicyclic or spirocyclic heterocycle radical which may be either saturated or unsaturated. Each heterocycle consists of carbon atoms and one or more, preferably from 1 to 4 heteroatoms chosen from nitrogen, oxygen and sulfur. The heterocycle may be attached by any atom of the cycle, which results in the creation of a stable structure.

The term "heteroaryl" shall be understood to mean an aromatic 5-8 membered monocyclic or 8-11 membered bicyclic ring containing 1-4 heteroatoms such as N,O and S.

Unless otherwise stated, heterocycles and heteroaryl include but are not limited to, for example furanyl, pyranyl, benzoxazolyl, benzothiazolyl, benzimidazolyl, tetrahydropyranyl, dioxanyl, tetrahydrofuranyl, oxazolyl, isoxazolyl, oxadiazolyl, triazolyl, thiazolyl, pyrazolyl, pyrrolyl, imidazolyl, thienyl, thiadiazolyl, thiomorpholinyl, 1,1-dioxo-1λ⁶-thiomorpholinyl, morpholinyl, pyridinyl, pyrimidinyl, pyridazinyl, pyrazinyl, triazinyl, pyrrolidinyl, piperidinyl, piperazinyl, purinyl, quinolinyl, Dihydro-2H-quinolinyl, isoquinolinyl, quinazolinyl, indazolyl, thieno[2,3-d]pyrimidinyl, indolyl, isoindolyl, benzofuranyl, benzopyranyl and benzodioxolyl, or 2-azaspiro[4.5]dec-2-yl, 1-aza-spiro[4.5]dec-1-yl, 1-aza-spiro[4.4]non-1-yl, 2-aza-spiro[4.4]non-2-yl, 2-aza-spiro[5.5]undec-2-yl, 1-aza-spiro[5.5]undec-1-yl.

The term "heteroatom" as used herein shall be understood to mean atoms other than carbon such as O, N, S and P.

In all alkyl groups or carbon chains one or more carbon atoms can be optionally replaced by heteroatoms: O, S or N, it shall be understood that ifN is not substituted then it is NH, it shall also be understood that the heteroatoms may replace either terminal carbon atoms or internal carbon atoms within a branched or unbranched carbon chain. Such groups can be substituted as herein above described by groups such as oxo to result in definitions such as but not limited to: alkoxycarbonyl, acyl, amido and thioxo.

The term "aryl" as used herein shall be understood to mean aromatic carbocycle or heteroaryl as defined herein. Each aryl or heteroaryl unless otherwise specified includes it's partially or fully hydrogenated derivative. For example, quinolinyl may include decahydroquinolinyl and tetrahydroquinolinyl, naphthyl may include its hydrogenated derivatives such as tetrahydranaphthyl. Other partially or fully hydrogenated derivatives of the aryl and heteroaryl compounds described herein will be apparent to one of ordinary skill in the art.

As used herein, "nitrogen" and "sulfur" include any oxidized form of nitrogen and sulfur and the quaternized form of any basic nitrogen. For example, for an -S-C₁₋₆alkyl radical, unless otherwise specified, this shall be understood to include -S(O)-C₁₋₆ alkyl and -S(O)₂-C₁₋₆ alkyl.

The term "alkyl" refers to a saturated aliphatic radical containing from one to ten carbon atoms or a mono- or polyunsaturated aliphatic hydrocarbon radical containing from two to twelve carbon atoms. The mono- or polyunsaturated aliphatic hydrocarbon radical containing at least one double or triple bond, respectively. "Alkyl" refers to both branched and unbranched alkyl groups. It should be understood that any combination term using an "alk" or "alkyl" prefix refers to analogs according to the above definition of "alkyl". For example, terms such as "alkoxy", "alkythio" refer to alkyl groups linked to a second group via an oxygen or sulfur atom. "Alkanoyl" refers to an alkyl group linked to a carbonyl group (C=O).

The term "halogen" as used in the present specification shall be understood to mean bromine, chlorine, fluorine or iodine, preferably fluorine. The definitions "halogenated", "partially or fully halogenated"; partially or fully fluorinated; "substituted by one or more halogen atoms", includes for example, mono, di or tri halo derivatives on one or more carbon atoms. For alkyl, a nonlimiting example would be -CH₂CHF₂, -CF₃ etc.

Each alkyl, carbocycle, heterocycle or heteroaryl, or the analogs thereof, described herein shall be understood to be optionally partially or fully halogenated.

The compounds of the invention are only those which are contemplated to be 'chemically stable' as will be appreciated by those skilled in the art. For example, a compound which would have a `dangling valency', or a `carbanion' are not compounds contemplated by the inventive methods disclosed herein.

The invention includes pharmaceutically acceptable derivatives of compounds of formula (I). A "pharmaceutically acceptable derivative" refers to any pharmaceutically acceptable salt or ester, or any other compound which, upon administration to a patient, is capable of providing (directly or indirectly) a compound useful for the invention, or a pharmacologically active metabolite or pharmacologically active residue thereof. A pharmacologically active metabolite shall be understood to mean any compound of the invention capable of being metabolized enzymatically or chemically. This includes, for example, hydroxylated or oxidized derivative compounds of the invention.

Pharmaceutically acceptable salts include those derived from pharmaceutically acceptable inorganic and organic acids and bases. Examples of suitable acids include hydrochloric, hydrobromic, sulfuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids. Other acids, such as oxalic acid, while not themselves pharmaceutically acceptable, may be employed in the preparation of salts useful as intermediates in obtaining the compounds and their pharmaceutically acceptable acid addition salts. Salts derived from appropriate bases include alkali metal (*e.g*., sodium), alkaline earth metal (*e.g*., magnesium), ammonium and N-(C₁-C₄ alkyl)₄⁺ salts.

In addition, within the scope of the invention is use of prodrugs of compounds of the invention. Prodrugs include those compounds that, upon simple chemical transformation, are modified to produce compounds of the invention. Simple chemical transformations include hydrolysis, oxidation and reduction. Specifically, when a prodrug is administered to a patient, the prodrug may be transformed into a compound disclosed hereinabove, thereby imparting the desired pharmacological effect.

The compounds of formula I may be made using the general synthetic methods described below, which also constitute part of the invention.

### GENERAL SYNTHETIC METHODS

The invention also provides processes for making compounds of Formula (I). Compounds of Formula (IA) may be made using the same Schemes. In all Schemes, unless specified otherwise, R¹, R², R³, R⁴, R⁵ and n in the Formulas below shall have the meaning of R¹, R², R³, R⁴, R⁵ and n in Formula (I) of the invention described herein above.

Optimum reaction conditions and reaction times may vary depending on the particular reactants used. Unless otherwise specified, solvents, temperatures, pressures, and other reaction conditions may be readily selected by one of ordinary skill in the art. Specific procedures are provided in the Synthetic Examples section. Typically, reaction progress may be monitored by thin layer chromatography (TLC), if desired, and intermediates and products may be purified by chromatography on silica gel and/or by recrystallization.

The examples which follow are illustrative and, as recognized by one skilled in the art, particular reagents or conditions could be modified as needed for individual compounds without undue experimentation. Starting materials and intermediates used, in the Schemes below, are either commercially available or prepared from commercially available materials by those skilled in the art.

Compounds of Formula (I) may be prepared by the Schemes 1 - 8.

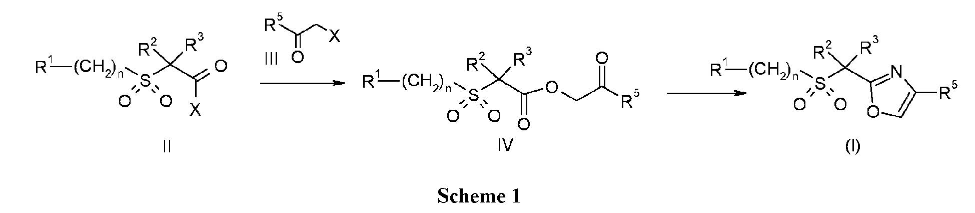

As illustrated in Scheme 1, reaction of an acid or its corresponding acid chloride of Formula (II) with a carbonyl compound of Formula (III), wherein X= hydroxyl or halogen, in a suitable solvent, in the presence of a suitable base, provides an intermediate of Formula (IV). Reaction of the intermediate of Formula (IV) with reagents such as acetamide and borontrifluoride diethyl etherate, in a suitable solvent provides a compound of Formula (I).

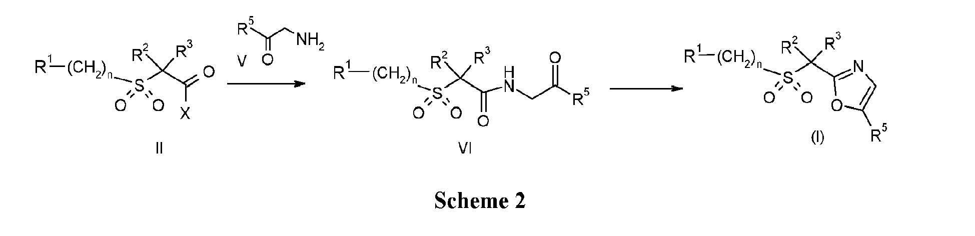

As illustrated in Scheme 2, reaction of an acid chloride of Formula (II) with an amino compound of Formula (V), in a suitable solvent, in the presence of a suitable base, provides an intermediate of Formula (VI). Heating the intermediate of Formula (VI), in a suitable solvent, in the presence of a reagent such as Burgess reagent, provides a compound of Formula (I).

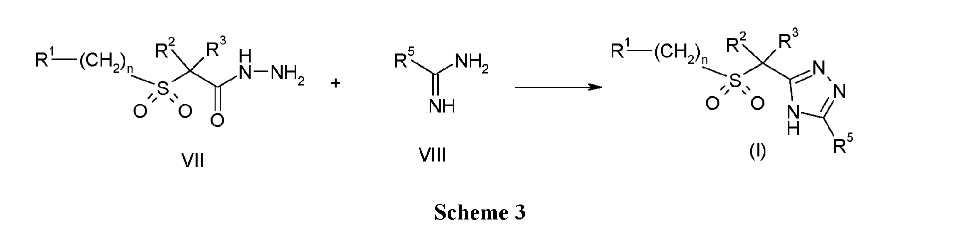

As shown in Scheme 3, reaction of a hydrazide of Formula (VII) with an amidine of Formula (VIII), in a suitable solvent, in the presence of a suitable base, provides a compound of Formula (I).

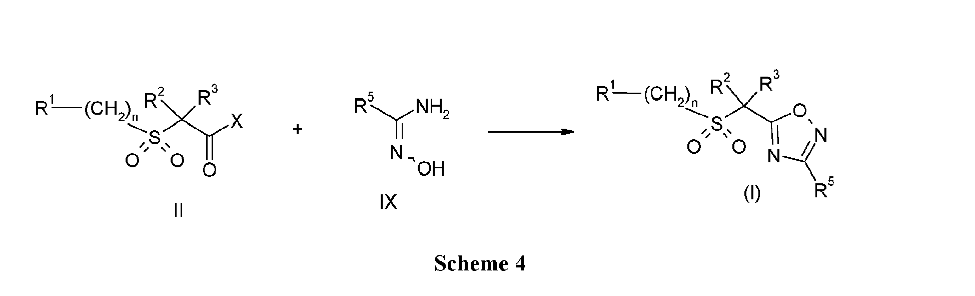

As illustrated in Scheme 4, reaction of an acid chloride of Formula (II) with an N-hydroxy amidine of Formula (IX), in a suitable solvent, in the presence of a suitable base, provides a compound of Formula (I)

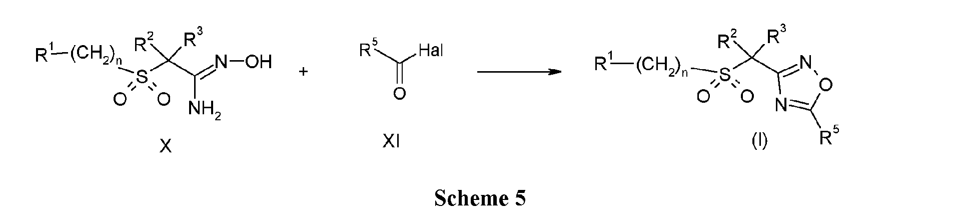

As outlined in Scheme 5, reaction of an acid chloride of Formula (XI) with an N-hydroxy amidine of Formula (X), in a suitable solvent, in the presence of a suitable base, provides a compound of Formula (I)

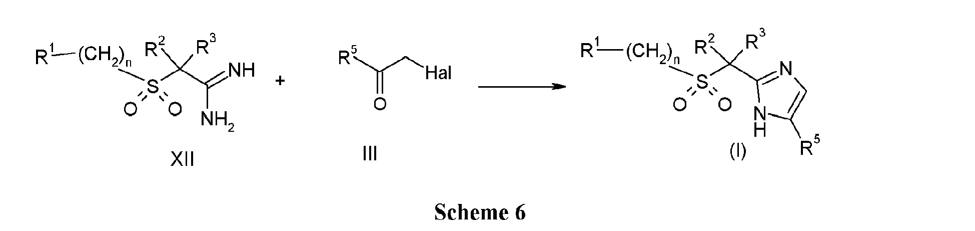

As shown in Scheme 6, reaction of an amidine of Formula (XII) with a carbonyl compound of Formula (III), in a suitable solvent, in the presence of a suitable base, provides a compound of Formula (I)

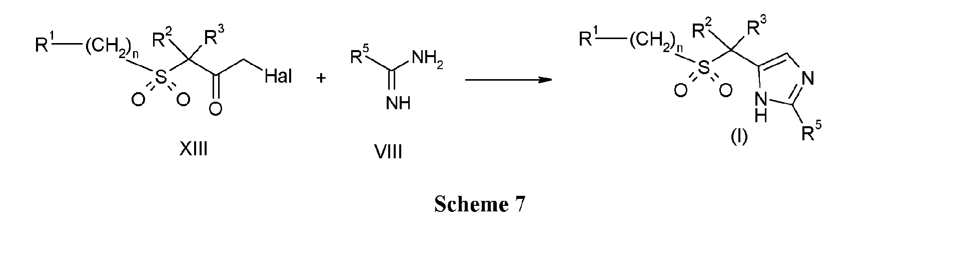

As outlined in Scheme 7, reaction of a sulfonyl carbonyl compound of Formula (XIII) with an amidine of Formula (VIII), in a suitable solvent, provides a compound of Formula (I)

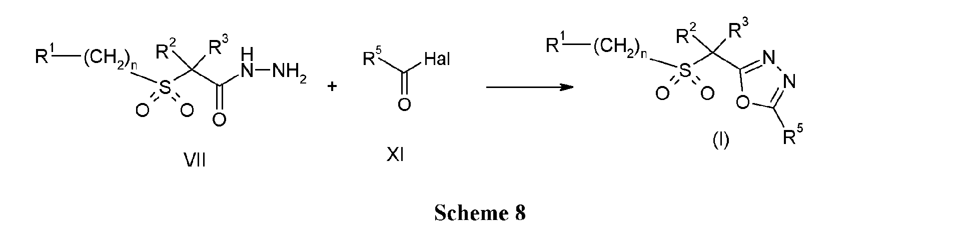

As shown in Scheme 8, reaction of the hydrazide of Formula (VII) with an acid chloride of Formula (XI), in a suitable solvent, in the presence of a suitable base, provides a compound of Formula (I).

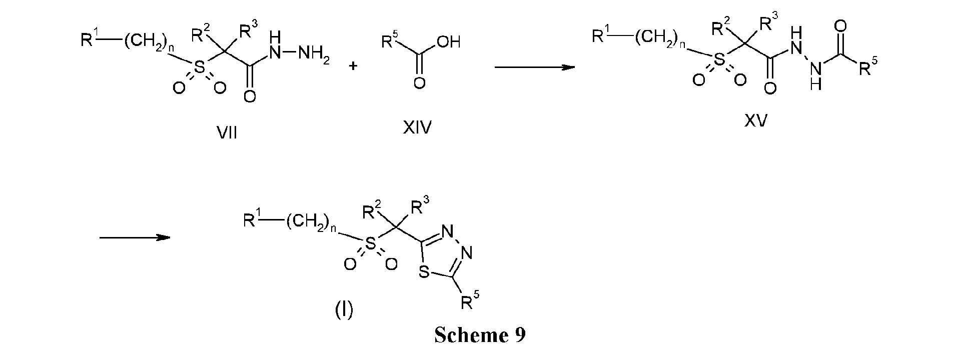

As outlined in Scheme 9, reaction of a hydrazide of Formula (VII) with an acid of Formula (XIV), in a suitable solvent, in the presence of a reagent such as phosphorus oxychloride, provides an intermediate hydrazide of Formula (XV). Reaction of this intermediate hydrazide (XV) with Lawesson's reagent, in a suitable solvent, at a suitable temperature, provides a compound of Formula (I)

Further modification of the initial product of Formula (I) by methods known to one skilled in the art and illustrated in the examples below, provides additional compounds of this invention.

### Method A

### Synthesis of 4-(5-tert-butyl-2-methyl-2H-pyrazol-3-yl)-2-[1-(4-chloro-benzenesulfonyl)-1-methyl-ethyl]-oxazole (Example 1 in Table 7)

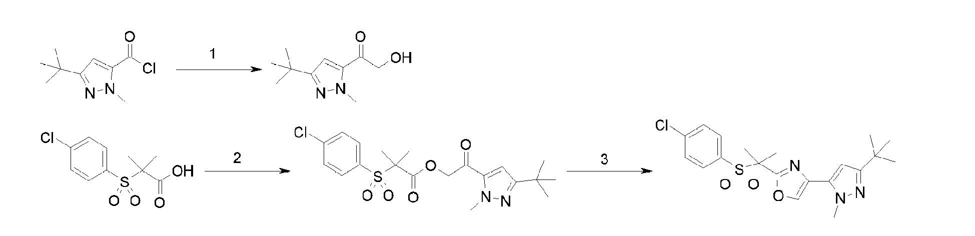

1 i TMSCHN₂, toluene, 0 °C- r.t.; ii water/EtOH, reflux
2 i (COCl)₂, cat. DMF, DCM, r.t.; ii TEA, DMAP, DCM, r.t.
3 MeCONH₂, BF₃.OEt, o-xylene, microwave, 200 °C

### Step 1: Synthesis of 1-(5-tert-butyl-2-methyl-2H-pyrazol-3-yl)-2-hydroxy-ethanone

The title compound is prepared from commercially available materials by those skilled in the art by adaptation of a literature procedure (Fisher et al, J. Am. Chem. Soc., 1944, 66, 4, 598-601).

To a solution of trimethylsilyl diazomethane 2M in hexanes (14.95 mL, 3.42 g, 29.90 mmol) in toluene (7 mL) is added dropwise at 0 °C a solution of 3-(tert-butyl)-1-methyl-1H-pyrazole-5-carbonyl chloride (1.00 g, 4.98 mmol) in toluene (3 mL). The reaction is stirred at 0 °C for 1 h and then at room temperature for 16 h. After this time, the reaction is concentrated under reduced pressure and the residue is dissolved in ethanol (10 mL). The solution is added slowly to water (50 mL) and heated at reflux for 3 h. The mixture is then cooled to room temperature and concentrated under reduced pressure to remove ethanol. The pH is adjusted to ~10 with a saturated aqueous solution of sodium carbonate and the aqueous layer is extracted with chloroform (3 x 100 mL). The organic layers are combined, washed with brine (20 mL), dried (Na₂SO₄), filtered and concentrated under reduced pressure. The residue is purified by chromatography on silica eluting with 7/3 cyclohexane/ethyl acetate to provide the title compound as a yellow oil (477.5 mg, 49%), m/z 197 [M+H⁺]. ¹H NMR (400 MHz, *CHLOROFORM-d* δ ppm 1.25 (9 H, s), 4.08 (3 H, s), 4.63 (2 H, s), 6.59 (1 H, s).

### Step 2: Synthesis of 2-(4-chloro-benzenesulfonyl)-2-methyl-propionic acid 2-(5-tert-butyl-2-methyl-2H-pyrazol-3-yl)-2-oxo-ethyl ester (Intermediate 1, Table 1)

To a solution of 2-(4-chloro-benzenesulfonyl)-2-methyl-propionic acid prepared according to W02008014199 (0.57 g, 2.17 mmol) in dichloromethane (20 mL) is added under nitrogen oxalyl chloride (1.12 mL, 1.65 g, 13.0 mmol) and *N,N*-dimethylformamide (3 drops). The reaction is stirred at room temperature for 16 h. After this time, the mixture is concentrated under reduced pressure and the crude acid chloride is used without further purification.

To a solution of the acid chloride (~2.17 mmol) in dichloromethane (10 mL) under nitrogen is added at room temperature triethylamine (0.46 mL, 0.33 g, 3.25 mmol), 4-dimethylaminopyridine (cat.) and a solution of 1-(5-tert-butyl-2-methyl-2H-pyrazol-3-yl)-2-hydroxy-ethanone (0.43 g, 2.17 mmol) in dichloromethane (10 mL). The reaction is stirred at room temperature for 16 h. After this time, the mixture is washed with a saturated aqueous solution of sodium bicarbonate (5 mL), brine (5 mL), dried (Na₂SO₄), filtered and concentrated under reduced pressure. The residue is purified by chromatography on silica eluting with 99.8/0.2 dichloromethane/methanol to provide the title compound as a white solid (721.7 mg, 75%), m/z 441 [M+H⁺]. ¹H NMR (400 MHz, *CHLOROFORM-d* δ ppm 1.20 (9 H, s), 1.62 (6 H, s), 3.98 (3 H, s), 5.07 (2 H, s), 6.60 (1 H, s), 7.43 (2 H, d, *J*=8.68 Hz), 7.77 (2 H, d, *J*=8.68 Hz).

Intermediates listed in Table 1 are prepared according to a similar procedure with the following modifications noted:
- For intermediate 5, the acid chloride intermediate is prepared in thionyl chloride at 60 °C for 2-3 h and the ester formation is performed without a catalytic amount of 4-dimethylaminopyridine.
- For intermediates 2 to 6, purification by column chromatography is achieved eluting with a heptane/ethyl acetate gradient (8/2 to 7/3).

**Table 1: Ester intermediates**

| # | Structure | Name | Yield [%] | m/z [M+H⁺] |
|---|---|---|---|---|
| Int 1 | 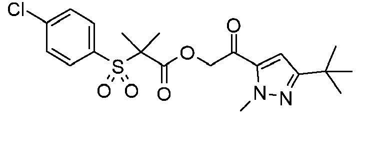 | 2-(4-chloro-benzenesulfonyl)-2-methyl-propionic acid 2-(5-tert-butyl-2-methyl-2H-pyrazol-3-yl)-2-oxo-ethyl ester | 75 | 441 |
| Int 2 | 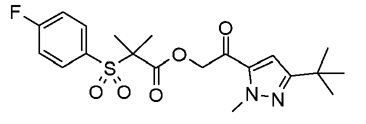 | 2-(4-fluoro-benzenesulfonyl)-2-methyl-propionic acid 2-(5-tert-butyl-2-methyl-2H-pyrazol-3-yl)-2-oxo-ethyl ester | 70 | 425 |
| Int 3 | 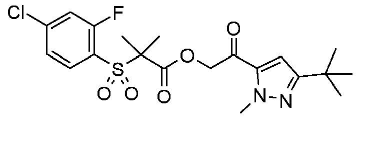 | 2-(4-chloro-2-fluoro-benzenesulfonyl)-2-methyl-propionic acid 2-(5-tert-butyl-2-methyl-2H-pyrazol-3-yl)-2-oxo-ethyl ester | 65 | 459 |
| Int 4 | 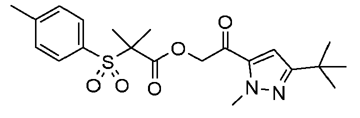 | 2-methyl-2-(toluene-4-sulfonyl)-propionic acid 2-(5-tert-butyl-2-methyl-2H-pyrazol-3-yl)-2-oxo-ethyl ester | 57 | 421 |
| Int 5 | 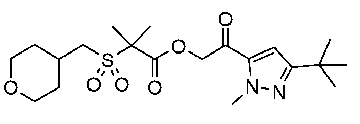 | 2-methyl-2-(tetrahydro-pyran-4-ylmethanesulfonyl)-propionic acid 2-(5-tert-butyl-2-methyl-2H-pyrazol-3-yl)-2-oxo-ethyl ester | 45 | 429 |
| Int 6 | 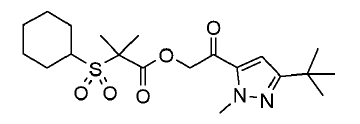 | 2-cyclohexanesulfonyl-2-methyl-propionic acid 2-(5-tert-butyl-2-methyl-2H-pyrazol-3-yl)-2-oxo-ethyl ester | 48 | 413 |

### Step 3: Synthesis of 4-(5-tert-butyl-2-methyl-2H-pyrazol-3-yl)-2-[1-(4-chlorobenzenesulfonyl)-1-methyl-ethyl]-oxazole (Example 1 in Table 7)

The title compound is prepared from intermediate 1 by those skilled in the art by adaptation of a literature procedure (Huang et al, Tetrahedron, 1996, 52, 30, 10131-6).

To a suspension of 2-(4-chloro-benzenesulfonyl)-2-methyl-propionic acid 2-(5-tert-butyl-2-methyl-2H-pyrazol-3-yl)-2-oxo-ethyl ester (Intermediate 1) (100 mg, 0.23 mmol) in o-xylene (2 mL) are added acetamide (67 mg, 1.13 mmol) and boron trifluoride diethyl etherate (14.2 µL, 16.1 mg, 0.11 mmol). The mixture is heated at 200 °C in a microwave for 6 h. After this time, the mixture is concentrated under reduced pressure and the residue is dissolved in dichloromethane (3 mL). The solution is washed with a saturated aqueous solution of sodium bicarbonate (1mL), dried (Na₂SO₄), filtered and concentrated under reduced pressure. The residue is purified twice by chromatography on silica eluting with 8/2 cyclohexane/ethyl acetate to provide the title compound as an orange oil (25.2 mg, 26%),, m/z 422 [M+H⁺]. ¹H NMR (400 MHz, *CHLOROFORM-d*) δ ppm 1.32 (9 H, s), 1.88 (6 H, s), 3.84 (3 H, s), 6.23 (1 H, s), 7.42 - 7.48 (2 H, m), 7.48 - 7.54 (2 H, m), 7.82 (1 H, s).

Using a similar procedure, examples in Table 7 Method A were prepared with the following modifications noted:
- For example 2, the final compound is purified by chromatography on silica eluting with 8/2 heptane/ethyl acetate.
- For examples 4 and 5, the cyclisation step is carried out at 220 °C in a microwave.

### Method B:

### Synthesis of 5-tert-butyl-3-{2-[1-(4-chloro-benzenesulfonyl)-1-methyl-ethyl]-oxazol-4-yl} isoxazole (Example 7 in Table 7)

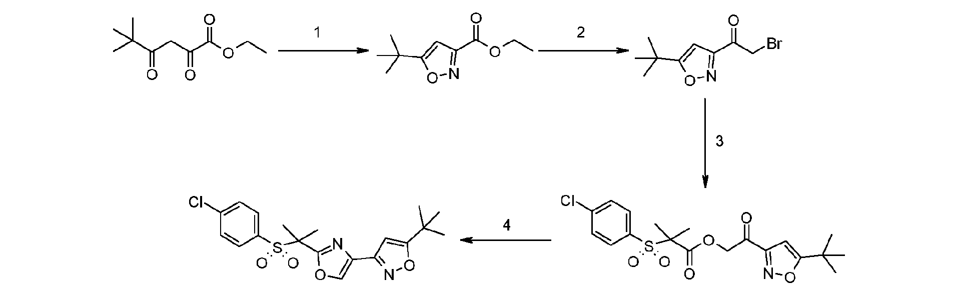

1 NH₂OH.HCl, NaHCO₃, EtOH, reflux
2 CH₂Br₂, MeLi, THF, -78 °C
3 Na₂CO₃, DMF, r.t
4 MeCONH₂, BF₃.OEt, o-xylene, microwave, 220 °C

### Step 1: Synthesis of 5-tert-butyl-isoxazole-3-carboxylic acid ethyl ester

The title compound is prepared by those skilled in the art according to a literature procedure (Lepage et al, Eur. J. Med. Chem., 1992, 27, 6, 581-93).

To a solution of sodium hydrogen carbonate (2.10 g, 25 mmol) and hydroxylamine hydrochloride (1.73 g, 25 mmol) in ethanol (25 mL) is added ethyl trimethyl acetopyruvate (5.00 g, 25 mmol). The mixture is heated at reflux for 16 h. After this time the sodium chloride is removed by filtration and the filtrate is concentrated under reduced pressure. The residue is purified by chromatography on silica eluting with 8/2 cyclohexane/ethyl acetate to provide the title compound as a yellow oil (3.2 g, 65%), m/z 198 [M+H⁺]. ¹H NMR (400 MHz, *CHLOROFORM-d*) δ ppm 1.3 8 (9 H, s), 1.42 (3 H, t, *J*=7.15 Hz), 4.44 (2 H, q, *J*=7.15 Hz), 6.38 (1 H, s).

### Step 2: Synthesis of 2-bromo-1-(5-tert-butyl-isoxazol-3-yl)-ethanone

The title compound is prepared from 5-tert-butyl-isoxazole-3-carboxylic acid ethyl ester by those skilled in the art by adaptation of a literature procedure *(*Kaluza et al, Tetrahedron, 2003, 59, 31,5893-5903).

To a solution of 5-tert-butyl-isoxazole-3-carboxylic acid ethyl ester (0.5 g, 2.54 mmol) in anhydrous tetrahydrofuran (10 mL) is added under nitrogen dibromomethane (356 µL, 0.88 g, 5.07 mmol). The mixture is cooled to -78 °C and 1.6M methyl lithium in diethyl ether (3.2 mL, 5.07 mmol) is added dropwise. The solution is stirred at -78 °C for 40 min and then quenched with acetic acid (582 µL, 610 mg, 10.16 mmol). The mixture is warmed to 0 °C and poured onto ice/water (40 mL) and extracted with tert-butyl methyl ether (3 x 40 mL). The organic layers are combined, dried (Na₂SO₄), filtered and concentrated under reduced pressure. The residue is purified by chromatography on silica eluting with a heptane/dichloromethane gradient (1/0 to 1/1) to provide the title compound as a yellow oil (351 mg, 62%), m/z 246 [M+H⁺]. ¹H NMR (400 MHz, *CHLOROFORM-d*) δ ppm 1.39 (9 H, s), 4.58 (2 H, s), 6.41 (1 H, s).

### Step 3: Synthesis of 2-(4-chloro-benzenesulfonyl)-2-methyl-propionic acid 2-(5-tert-butylisoxazol-3-yl)-2-oxo-ethyl ester

To a solution of 2-(4-chloro-benzenesulfonyl)-2-methyl-propionic acid (320 mg, 1.22 mmol) in *N,N*-dimethylformamide (30 mL) is added sodium carbonate (129 mg, 1.22 mmol). The suspension is stirred at room temperature for 15 min then 2-bromo-1-(5-tert-butyl-isoxazol-3-yl)-ethanone (300 mg, 1.22 mmol) is added and the mixture is stirred at room temperature for 16 h. After this time, the mixture is concentrated under reduced pressure and the residue is dissolved in dichloromethane (20 mL). The solution is washed with a saturated aqueous solution of sodium bicarbonate (5 mL), dried (Na₂SO₄), filtered and concentrated under reduced pressure. The residue is purified by chromatography on silica eluting with 8/2 cyclohexane/ethyl acetate to provide the title compound as a yellow oil (160 mg, 31%), m/z 428 [M+H⁺]. ¹H NMR (400 MHz, *CHLOROFORM-d*) δ ppm 1.37 (9 H, s), 1.72 (6 H, s), 5.35 (2 H, s), 6.34 (1 H, s), 7.53 (2 H, d, *J*=8.68 Hz), 7.87 (2 H, d, *J*=8.68 Hz).

### Step 4: Synthesis of 5-tert-butyl-3-{2-[1-(4-chloro-benzenesulfonyl)-1-methyl-ethyl]-oxazol-4-yl}-isoxazote (Example 7 in Table 7)

The title compound is prepared from 2-(4-chloro-benzenesulfonyl)-2-methyl-propionic acid 2-(5-tert-butyl-isoxazol-3-yl)-2-oxo-ethyl ester by those skilled in the art by adaptation of a literature procedure (Huang et al, Tetrahedron, 1996, 52, 30, 10131-6).

To a suspension of 2-(4-chloro-benzenesulfonyl)-2-methyl-propionic acid 2-(5-tert-butylisoxazol-3-yl)-2-oxo-ethyl ester (155 mg, 0.36 mmol) in o-xylene (5 mL) are added acetamide (107 mg, 1.81 mmol) and boron trifluoride diethyl etherate (32 µL, 36 mg, 0.25 mmol). The mixture is heated at 220 °C in a microwave for 7 h. After this time, the mixture is concentrated under reduced pressure and the residue is dissolved in dichloromethane (3 mL). The solution is washed with a saturated aqueous solution of sodium bicarbonate (1 mL), dried (Na₂SO₄), filtered and concentrated under reduced pressure. The residue is first purified by chromatography on silica eluting with 8/2 cyclohexane/ethyl acetate and then by mass-triggered preparative HPLC before it is freebased with Ambersep 900-OH resin to provide the title compound as an orange oil_(9.2 mg, 6%), m/z 409 [M+H⁺]. ¹H NMR (400 MHz, *CHLOROFORM-d*) δ ppm 1.39 (9 H, s), 1.89 (6 H, s), 6.19 (1 H, s), 7.41 - 7.53 (4 H, dm), 8.11 (1 H, s).

Using a similar procedure, examples in Table 7 Method **B** were prepared:

### Method C

### Synthesis of 5-(5-tert-butyl-2-methyl-2H-pyrazol-3-yl)-2-[1-(4-chloro-benzenesulfonyl)-1-methyl-ethyl]-oxazole (Example 8 in Table 7)

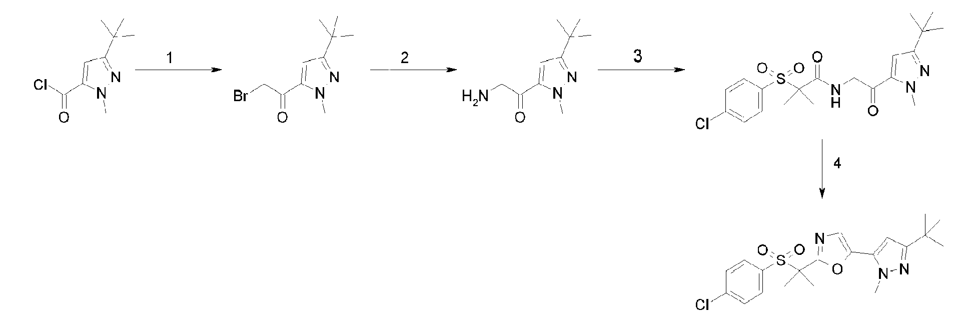

1 i TMSCHN₂, toluene, 0 °C- r.t.; ii 48% HBr, 1,4-dioxane, r.t.
2 i Hexamethylenetetramine, CHCl₃, 50 °C; ii HCl, EtOH, r.t.
3 i SOCl₂, 50 °C; ii DIPEA, DCM, r.t.
4 Burgess reagent, THF, 100 °C

### Step 1: Synthesis of 2-bromo-1-(5-tert-butyl-2-methyl-2H-pyrazol-3-yl)-ethanone

The title compound is prepared from commercially available materials by those skilled in the art by adaptation of a literature procedure (Fisher et al, J. Am. Chem. Soc., 1944, 66,4, 598-601). To a solution of 3-(tert-butyl)-1-methyl-1H-pyrazole-5-carbonyl chloride (1.20 g, 6 mmol) in toluene (10 mL) is added dropwise at 0 °C a solution of 2M trimethylsilyl diazomethane in hexanes (15 mL, 3.43 g, 30 mmol). The reaction is stirred at room temperature for 16 h. After this time, 1,4-dioxane (15 mL) is added and the reaction is cooled to 0 °C before a solution of 48% aqueous hydrobromic acid in 1,4-dioxane (5 mL) is introduced slowly. The reaction is stirred at room temperature for 1 h and then the pH is adjusted to ∼8-9 with a saturated aqueous solution of sodium carbonate. The aqueous layer is extracted with ethyl acetate (3 x 40 mL) and the organic layers are combined, washed with brine (20 mL), dried (Na₂SO₄), filtered and concentrated under reduced pressure. The residue is purified by chromatography on silica eluting with dichloromethane to provide the title compound as a colourless oil (1.25 g, 90%), m/z 259 [M+H⁺]. ¹H NMR (400 MHz, *CHLOROFORM-d*) δ ppm 1.11 (9 H, s), 3.91 (3 H, s), 4.12 (2 H, s), 6.53 (1H, s).

### Step 2: Synthesis of 2-amino-1-(5-tert-butyl-2H-pyrazol-3-yl)-ethanone

The title compound is prepared from 2-bromo-1-(5-tert-butyl-2-methyl-2H-pyrazol-3-yl)-ethanone by those skilled in the art following literature precedent.

To a solution of 2-bromo-1-(5-tert-butyl-2-methyl-2H-pyrazol-3-yl)-ethanone (1 g, 3.86 mmol) in chloroform (5 mL) is added hexamethylenetetramine (0.57 g, 4.05 mmol). The mixture is heated at 50 °C for 2 h. After this time, the reaction mixture is cooled to room temperature and the precipitate is collected by filtration and washed with additional chloroform (3 x 10 mL). The white solid that is obtained after filtration (1.25 g) is suspended in ethanol (12.5 mL) and 12N aqueous hydrochloric acid (6 mL) is added. The mixture is stirred at room temperature for 2 h and then the mixture is concentrated under reduced pressure to afford the hydrochloride salt of the title compound as a white solid (1.14 g). This solid is used without further purification.

### Step 3: Synthesis of N-[2-(5-tert-butyl-2-methyl-2H-pyrazol-3-yl)-2-oxo-ethyl]-2-(4-chlorobenzenesulfonyl)-2-methyl-propionamide (Intermediate 7, Table 2)

2-(4-Chloro-benzenesulfonyl)-2-methyl-propionic acid (289 mg, 1.1 mmol) is added to thionyl chloride (3 mL) and the solution is heated at 50-60 °C for 2 h. After this time, the mixture is concentrated under reduced pressure and the crude acid chloride product is used without further purification.

To a solution of the crude acid chloride (∼1.1 mmol) in dichloromethane (5 mL) are added under nitrogen a solution of 2-amino-1-(5-tert-butyl-2H-pyrazol-3-yl)-ethanone hydrochloric salt in dichloromethane (5 mL) and *N,N*-diisopropyldiethylamine (870 µL, 646 mg, 5 mmol). The mixture is stirred at room temperature for 2 h. After this time, the solution is washed with a saturated aqueous solution of sodium bicarbonate (1 mL), brine (1 mL), dried (Na₂SO₄), filtered and concentrated under reduced pressure. The residue is purified by chromatography on silica eluting with 9/1 dichloromethane/ethyl acetate to provide the title compound as a yellow oil (221 mg, 50%), m/z 440 [M+H⁺]. ¹H NMR (400 MHz, *CHLOROFORM-d*) δ ppm 1.32 (9 H, s), 1.64 (6 H, s), 4.17 (3 H, s), 4.60 (2 H, d, *J*=4.77 Hz), 6.75 (1 H, s), 7.50 - 7.58 (2 H, m), 7.72 - 7.81 (1 H, m), 7.83 - 7.93 (2 H, m).

Intermediates listed in Table 2 are prepared according to a similar procedure with the following modifications noted. For intermediates 8 to 10, purification by column chromatography is performed using a dichloromethane/ethyl acetate gradient: 10/0 to 9/1 for intermediates 8 and 10 and 10/0 to 8/2 for intermediate 7.

**Table 2: Amide intermediates**

| # | Structure | Name | Yield [%] | m/z [M+H⁺] |
|---|---|---|---|---|
| Int 7 | 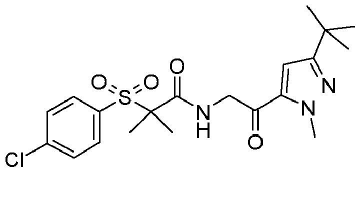 | *N*-[2-(5-tert-butyl-2-methyl-2H-pyrazol-3-yl)-2-oxo-ethyl]-2-(4-chloro-benzenesulfonyl)-2-methyl-propionamide | 50 | 440 |
| Int 8 | 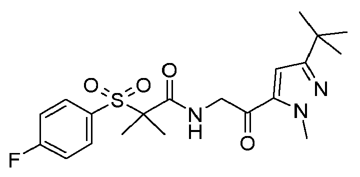 | *N*-[2-(5-tert-butyl-2-methyl-2H-pyra zol-3-yl)-2-oxo-ethyl]-2-(4-fluoro-benzenesulfonyl)-2-methyl-propionamide | 17 | 424 |
| Int 9 | 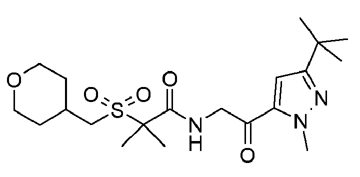 | *N*-[2-(5-tert-butyl-2-methyl-2H-pyra zol-3-yl)-2-oxo-ethyl]-2-methyl-2-(tetrahydro-pyran-4-ylmethanesulfonyl)-propionamide | 48 | 428 |
| Int 10 | 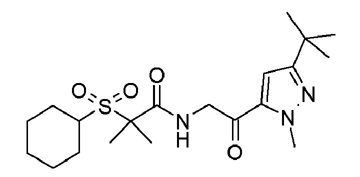 | *N*-[2-(5-tert-butyl-2-methyl-2H-pyra zol-3-yl)-2-oxo-ethyl]-2-cyclohexanesulfonyl-2-methyl-propionamide | 29 | 412 |

### Step 4: Synthesis of 5-(5-tert-butyl-2-methyl-2H-pyrazol-3-yl)-2-[1-(4-chlorobenzenesulfonyl)-1-methyl-ethyl]-oxazole (Example 8 in Table 7)

The title compound is prepared from intermediate 7 by those skilled in the art by adaptation of a literature procedure (Davies et al, J. Org. Chem., 2005, 70, 14, 5840-5851).

To a solution of *N*-[2-(5-tert-butyl-2-methyl-2H-pyrazol-3-yl)-2-oxo-ethyl]-2-(4-chlorobenzenesulfonyl)-2-methyl-propionamide (Intermediate 7) (175 mg, 0.40 mmol) in tetrahydrofuran (2 mL) is added (methoxycarbonylsulfamoyl)triethylammonium hydroxide (Burgess reagent, 236 mg, 0.99 mmol). The mixture is heated at 100 °C in a sealed tube for 16 h. After this time, the mixture is diluted in ethyl acetate (20 mL), washed with brine (5 mL), dried (Na₂SO₄), filtered and concentrated under reduced pressure. The residue is purified first by chromatography on silica eluting with a dichloromethane/ethyl acetate gradient (10/0 to 9/1) and then by preparative HPLC to provide the title compound as a yellow oil_(23 mg, 14%)" m/z 422 [M+H⁺]. ¹H NMR (400 MHz, *CHLOROFORM-d*) δ ppm 1.33 (9 H, s), 1.88 (6 H, s), 3.97 (3 H, s), 6.28 (1 H, s), 7.13 (1 H, s), 7.40 - 7.56 (4 H, m).

Examples listed in Table 7 Method C are prepared according to a similar procedure with the following modifications noted. For examples 8 to 11, purification was achieved by column chromatography only.

### Method D Synthesis of 3-(5-tert-butyl-2-methyl-2H-pyrazol-3-yl)-5-[1-(4-chlorobenzenesulfonyl)-1-methyl-ethyl]-4H-[1,2,4]triazole (Example 12 in Table 7)

### Synthesis of 5-tert-butyl-2-methyl-2H-pyrazole-3-carboxamidine (Intermediate 11)

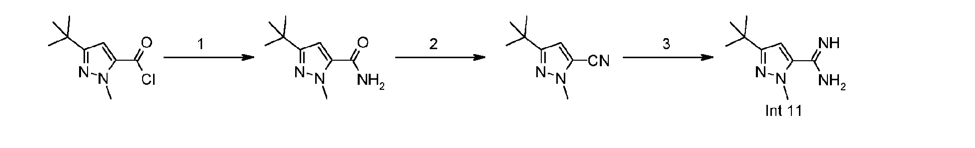

1 NH₄OH, DCM, 0 °C - r.t
2 POCl₃, 60 °C
3 i NaH, MeOH, 0 - 40 °C; ii NH₄Cl, 70 °C

### Step 1: Synthesis of 5-tert-butyl-2-methyl-2H-pyrazole-3-carboxylic acid amide

To a vigorously stirred aqueous solution of ammonium hydroxide (20 mL) at 0 °C is added dropwise 3-tert-butyl-1-methyl-1H-pyrazole-5-carboxylic chloride (1.94 g, 9.66 mmol) in dichloromethane (20 mL). The mixture is warmed to room temperature where it is maintained for 2 h. After this time, the reaction mixture is extracted with dichloromethane (3 x 50 mL) and the organic layers are combined, washed with brine (20 mL), dried (Na₂SO₄), filtered and concentrated under reduced pressure to afford the title compound as a white solid (1.71 mg, 97%), m/z 182 [M+H⁺]. ¹H NMR (400 MHz, *CHLOROFORM-d*) δ ppm 1.31 (9 H, s), 4.13 (3 H, s), 5.30-6.11 (2 H, m), 6.38 (1 H, s).

### Step 2: Synthesis of 5-tert-butyl-2-methyl-2H-pyrazole-3-carbonitrile

To 5-tert-butyl-2-methyl-2H-pyrazole-3-carboxylic acid amide (1.39 g, 7.67 mmol) is added phosphorus oxychloride (30 mL) and the solution is heated at 60 °C for 24 h. After this time, the mixture is concentrated under reduced pressure and the residue dissolved in dichloromethane (25 mL). The solution is washed with a saturated aqueous solution of sodium bicarbonate (10 mL), brine (10 mL), dried (Na₂SO₄), filtered and concentrated under reduced pressure to give the title compound as a yellow oil (1.21 g, 97%), m/z 164 [M+H⁺]. ¹H NMR (400 MHz, *CHLOROFORM-d*) δ ppm 1.23 (9 H, s), 3.94 (3 H, s), 6.56 (1 H, s).

### Step 3: Synthesis of 5-tert-butyl-2-methyl-2H-pyrazole-3-carboxamidine (Intermediate 11)

Intermediate 11 is prepared from 5-tert-butyl-2-methyl-2H-pyrazole-3-carbonitrile by those skilled in the art following literature precedent.

To methanol (20 mL) at 0 °C under nitrogen is added sodium hydride (60% dispersion in mineral oil, 1.77 g, 44.1 mmol) in portions over 15 min. The suspension is stirred for 10 min at 0 °C then a solution of 5-tert-butyl-2-methyl-2H-pyrazole-3-carbonitrile (0.71 g, 4.3 mmol) in methanol (10 mL) is added and the mixture is heated at 40 °C for 2 h. After this time, solid ammonium chloride (2.33 g, 43.6 mmol) is introduced and the mixture is heated at 70 °C for 16 h. The solvent is removed under reduced pressure and the residue dissolved in a 1N aqueous solution of hydrochloric acid (20 mL). The aqueous layer is extracted with diethyl ether (2 x 100 mL) and then the pH is adjusted to ∼9 with a 5N aqueous solution of sodium hydroxide. The mixture is extracted with ethyl acetate (5 x 50 mL) and the combined organic layers were dried (Na₂SO₄), filtered and concentrated under reduced pressure to give the title compound as a cream solid (386 mg, 49%), m/z 181 [M+H⁺]. ¹H NMR (250 MHz, *CHLOROFORM-d*) δ ppm 1.29 (9 H, s), 4.00 (3 H, s), 5.55 (3 H, br. s.), 6.30 (1 H, s).

### Synthesis of 2-(4-chloro-benzenesulfonyl)-2-methyl-propionic acid hydrazide (Intermediate 12 , Table 3)

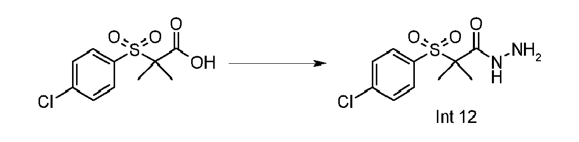

i Thionyl chloride, 60 °C; ii BocHNNH₂, DCM, r.t.; iii 6N HCl, MeOH, r.t.; iv Ambersep 900-OH resin, MeOH, r.t.

Intermediate 12 is prepared according to the following procedure developed in house. 2-(4-Chloro-benzenesulfonyl)-2-methyl-propionic acid (0.5 g, 1.9 mmol) is added to thionyl chloride (2.5 mL) and the solution is heated at 60 °C for 2 h. After this time, the mixture is concentrated under reduced pressure and the crude acid chloride product is used without further purification.

To a solution of tert-butyl carbazate (264 mg, 2 mmol) in dichloromethane (2.5 mL) is added under nitrogen a solution of the crude acid chloride (∼1.9 mmol) in dichloromethane (2.5 mL). The reaction is stirred at room temperature for 1 h. After this time, the mixture is washed with a saturated aqueous solution of sodium bicarbonate (1 mL), dried (Na₂SO₄), filtered and concentrated under reduced pressure. The residue is dissolved in methanol (3 mL) and a 6N aqueous solution of hydrochloric acid (1.6 mL, 9.5 mmol) is added. The mixture is stirred at room temperature for 16 h. After this time, the mixture is concentrated under reduced pressure and the residue dissolved in methanol (3 mL). Ambersep 900-OH resin (3 mmol/g, 2 g, 6 mmol) is added and the suspension is shaken at room temperature for 2 h. The mixture is filtered and the resin washed with methanol (3 x 10 mL). The filtrates are combined and concentrated under reduced pressure to give the title compound as a colourless oil (420 mg, 80%), m/z 277 [M+H⁺]. ¹H NMR (400 MHz, *CHLOROFORM-d*) δ ppm 1.60 (6 H, s), 7.47 - 7.63 (2 H, m), 7.70 - 7.86 (2 H, m), 8.13 (1 H, br. s.).

### Synthesis of 3-(5-tert-butyl-2-methyl-2H-pyrazol-3-yl)-5-[1-(4-chloro-benzenesulfonyl)-1-methyl-ethyl]-4H-[1,2,4]triazole (Example 12 in Table 7)

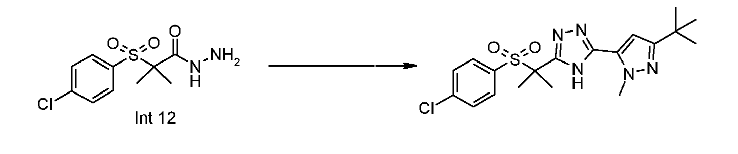

NaOMe, MeOH, reflux

The title compound is prepared from intermediate 12 by those skilled in the art by adaptation of a literature procedure (Francis et al, Tetrahedron Lett., 1987, 28,43,5133-6).

A mixture of sodium methoxide (18 mg, 0.33 mmol), 2-(4-chloro-benzenesulfonyl)-2-methyl-propionic acid hydrazide (Intermediate 12) (184 mg, 0.67 mmol) and 5-tert-butyl-2-methyl-2H-pyrazole-3-carboxamidine (Intermediate 11) (120 mg, 0.67 mmol) in methanol (3.5 mL) is heated at reflux for 42 h. After this time, the mixture is cooled to room temperature and ethyl acetate (20 mL) is introduced. The mixture is washed with a saturated aqueous solution of ammonium chloride (10 mL) and then the organic layer separated, dried (Na₂SO₄), filtered and concentrated under reduced pressure. The residue is purified by chromatography on silica eluting with a dichloromethane/ethyl acetate gradient (1/0 to 3/2) to afford the title compound as a yellow oil, m/z 422 [M+H⁺]. ¹H NMR (400 MHz, *CHLOROFORM-d*) δ ppm 1.32 (9 H, s), 1.88 (6 H, s), 4.03 (3 H, s), 6.53 (1 H, s), 7.40 - 7.51 (4H, m), 11.53 (1 H, br. s.).

### Method D-1:

### Synthesis of 3-(3-tert-butyl-isoxazol-5-yl)-5-[1-(4-chloro-benzenesulfonyl)-1-methyl-ethyl]-4H-[1,2,4]triazole (Example 13 in Table 7) and 2-(3-tert-butyl-isoxazol-5-yl)-5-[1-(4-chlorobenzenesulfonyl)-1-methyl-ethyl] [1,3,4]oxadiazole (Example 22 in Table 7)

### Synthesis of 3-tert-butyl-isoxazole-5-carboxamidine (Intermediate 13)

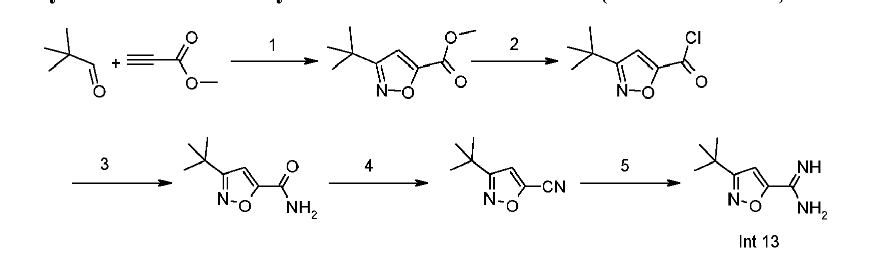

1 i NH₂OH HCl, NaOH, 1:1 t-butanol/H₂O, r.t.; ii chloramine-T, Cu/CuSO₄, r.t.; iii methyl propiolate, reflux
2 i TMSOK, THF, r.t.; ii. SOCl₂, cat. DMF, DCM, r.t
3 NH₄OH, DCM, 0 °C - r.t
4 POCl₃ 60 °C
5 i NaH, MeOH, 0 - 40 °C; ii NH₄Cl, 70 °C

### Step 1: Synthesis of 3-tert-butyl-isoxazole-5-carboxylic acid methyl ester

The title compound is prepared from commercially available materials by those skilled in the art by adaptation of a literature reference (Hansen et al, J. Org. Chem., 2005, 70, 19, 7761-4).

To a solution of pivaldehyde (10.09 g, 117 mmol) in 1:1 tert-butanol/water (400 mL) are added hydroxylamine hydrochloride (8.13 g, 117 mmol) and sodium hydroxide (4.70 g, 117 mmol). The solution is stirred at room temperature for 30 min before chloramine-T trihydrate (54.49 g, 234 mmol) is added in portions over 5 min followed by copper sulfate (3.27 g, 13 mmol), copper powder (0.75 g, 12 mmol) and methyl propiolate (10.4 mL, 9.84 g, 117 mmol). The reaction mixture is heated at reflux where it is maintained for 2 h. After this time, the mixture is cooled to room temperature and poured onto ice/water (500 g). Ammonium hydroxide (100 mL) is added and the solution is extracted with dichloromethane (3 x 200 mL). The organic layers are combined, dried (Na₂SO₄), filtered and concentrated under reduced pressure. The solid is suspended in heptane (3 x 500 mL), filtered and the combined filtrates are concentrated under reduced pressure. The residue is purified by chromatography on silica eluting with 9/1 heptane/ethyl acetate to afford the title compound as a yellow oil (5.97 g, 28%), m/z 184 [M+H⁺]. ¹H NMR (400 MHz, *CHLOROFORM-d*) δ ppm 1.36 (9 H, s), 3.96 (3 H, s), 6.88 (1 H, s).

### Step 2: Synthesis of 3-tert-butyl-isoxazole-5-carbonyl chloride

To a solution of 3-tert-butyl-isoxazole-5-carboxylic acid methyl ester (2.72 g, 14.9 mmol) in tetrahydrofuran (75 mL) is added potassium trimethylsilanoate (2.49 g, 19.4 mmol). The mixture is stirred at room temperature for 16 h. After this time, the reaction is quenched with water (25 mL) and tetrahydrofuran is removed under reduced pressure. The solution is washed with diethyl ether (50 mL) and then the pH of the aqueous phase is adjusted to ∼1 with a 6N aqueous solution of hydrochloric acid. The aqueous layer is extracted with ethyl acetate (3 x 50 mL) and the organic layers are combined, dried (Na₂SO₄), filtered and concentrated under reduced pressure to give 3-tert-butyl-isoxazole-5-carboxylic acid as a yellow solid (2.6 g, 100%), m/z 170 [M+H⁺]. ¹H NMR (400 MHz, *CHLOROFORM-d*) δ ppm 1.38 (9 H, s), 6.99 (1 H, s), 9.44 (1 H, br. s.).

To a solution of 3-tert-butyl-isoxazole-5-carboxylic acid (1.02 g, 6 mmol) in anhydrous dichloromethane (10 mL) is added under nitrogen thionyl chloride (2.6 mL, 4.24 g, 36 mmol) and *N,N*-dimethylformamide (1 drop). The reaction is stirred at room temperature for 16 h. After this time, the mixture is concentrated under reduced pressure and the crude acid chloride is used without further purification.

**Step 3:** Synthesis of 3-tert-butyl-isoxazole-5-carboxylic acid amide is done using a similar procedure to the synthesis of 5-tert-butyl-2-methyl-2H-pyrazole-3-carboxylic acid amide (Intermediate 11, step 1) but with 3-tert-butyl-isoxazole-5-carbonyl chloride as starting material. **Step 4:** Synthesis of 3-tert-butyl-isoxazole-5-carbonitrile is done using a similar procedure to the synthesis of 5-tert-butyl-2-methyl-2H-pyrazole-3-carbonitrile (Intermediate 11, step 2) but with 3-tert-butyl-isoxazole-5-carboxylic acid amide as starting material and it is achieved at 50 °C for 4 h.

**Step 5:** Synthesis of 3-tert-butyl-isoxazole-5-carboxamidine (Intermediate 13) is done using a similar procedure to the synthesis of 5-tert-butyl-2-methyl-2H-pyrazole-3-carboxamidine (Intermediate 11, step 3) but with 3-tert-butyl-isoxazole-5-carbonitrile as a starting material and in the presence of 12.5 eq ammonium chloride (500 mg, 82%), m/z 168 [M+H⁺]. ¹H NMR (250 MHz, *CHLOROFORM-d*) δ ppm 1.38 (9 H, s), 4.16 (3 H, br. s.), 6.76 (1 H, s).

### Synthesis of 2-methyl-2-(tetrahydro-pyran-4-ylmethanesulfonyl)-propionic acid hydrazide (Intermediate 14, Table 3)

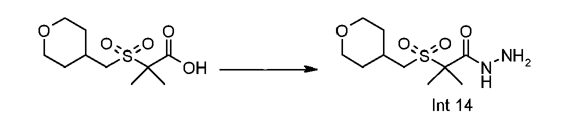

i Oxalyl chloride, cat. DMF, DCM, rt; ii BocHNNH₂, DIPEA, DCM, r.t.; iii 6N HCl, MeOH, r.t.; iv Ambersep 900-OH resin, MeOH, r.t.

The synthesis of intermediate 14 is done in a similar manner as the synthesis of 2-(4-chlorobenzenesulfonyl)-2-methyl-propionic acid hydrazide (Intermediate 12) with the following modifications. The acid chloride formation is achieved using an excess of oxalyl chloride and a few drops of *N,N*-dimethylformamide at room temperature in dichloromethane for 3 h. The mixture of crude acid chloride and tert-butyl carbazate is stirred at room temperature for 2 days. The hydrazide is deprotected using a 6N aqueous solution of hydrochloric acid and freebased on Ambersep 900-OH resin (948 mg, 90%), m/z 265 [M+H⁺]. ¹H NMR (500 MHz, *CHLOROFORM-d*) δ ppm 1.37 - 1.52 (2 H, m), 1.61 (6 H, s), 1.81 (2 H, d, *J*=13.07 Hz), 2.25 - 2.42 (1 H, m), 2.99 (2 H, d, *J*=6.60 Hz), 3.34 - 3.48 (2 H, m), 3.67 - 4.42 (4 H, m), 8.18 (1 H, br. s.).

Intermediates listed in Table 3 are prepared according to a similar procedure with the following modifications noted. Intermediate 17 is deprotected using trifluoroacetic acid in methanol.

**Table 3: Hydrazide intermediates**

| # | Structure | Name | Yield [%] | m/z [M+H⁺] |
|---|---|---|---|---|
| Int 12 | 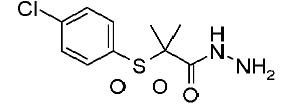 | 2-(4-chloro-benzenesulfonyl)-2-methyl-propionic acid hydrazide | 80 | 277 |
| Int 14 | 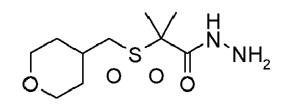 | 2-methyl-2-(tetrahydro-pyran-4-ylmethanesulfonyl)-propionic acid hydrazide | 90 | 265 |
| Int 15 | 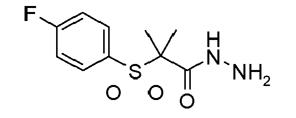 | 2-(4-fluoro-benzenesulfonyl)-2-methyl-propionic acid hydrazide | 75 | 261 |
| Int 16 | 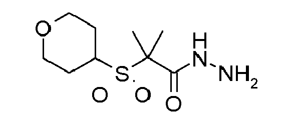 | 2-methyl-2-(tetrahydro-pyran-4-sulfonyl)-propionic acid hydrazide | 100 | 251 |
| Int 17 | 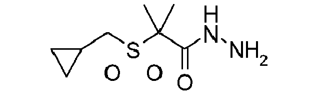 | 2-cyclopropylmethanesulfonyl-2-methyl-propionic acid hydrazide | 100 | 221 |
| Int 18 | 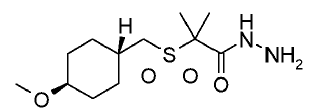 | 2-(4-methoxy-cyclohexylmethanesulfonyl)-2-methyl-propionic acid hydrazide | 89 | 293 |

### Synthesis of 2-(4-fluoro-phenylmethanesulfonyl)-2-methyl-propionic acid hydrazide (Intermediate 19)

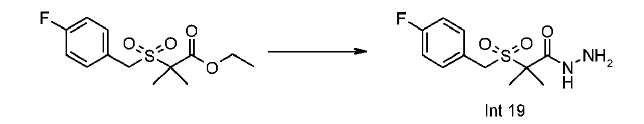

NH₂NH₂. H₂O EtOH, 100 °C

Intermediate 19 is prepared according to the following procedure developed in house.

A solution of 2-(4-fluoro-phenylmethanesulfonyl)-2-methyl-propionic acid ethyl ester (1.97 g, 6.8 mmol) and hydrazine hydrate (35% wt in water, 4 mL, 4.04 g, 44.1 mmol) in ethanol (20 mL) is heated at 100 °C for 4 h then at room temperature over 2 days. More hydrazine hydrate is added (2 mL, 1.98 g, 21.6 mmol) and the solution is heated at 100 °C for another 8 h. The reaction mixture is concentrated under reduced pressure and the residue taken up in dichloromethane (50 mL), washed with water (25 mL), dried (MgSO₄), filtered and concentrated under reduced pressure to give the title compound as a clear oil (1.23 g, 66%), m/z 275 [M+H⁺]. ¹H NMR (500 MHz, *CHLOROFORM-d*) δ ppm 1.60 (6 H, s), 3.51 (1 H, br. s.), 4.26 (2 H, s), 7.01 (2 H, t, *J*=8.62 Hz), 7.31 (2 H, dd, *J*=8.39, 5.34 Hz), 7.83 (4 H, br. s.).

### Synthesis of 3-(3-tert-butyl-isoxazol-5-yl)-5-[1-(4-chloro-benzenesulfonyl)-1-methyl-ethyl]-4H-[1,2,4]triazole (Example 13 in Table 7) and 2-(3-tert-butyl-isoxazol-5-yl)-5-[1-(4-chlorobenzenesulfonyl)-1-methyl-ethyl] [1,3,4]oxadiazole (Example 22 in Table 7)

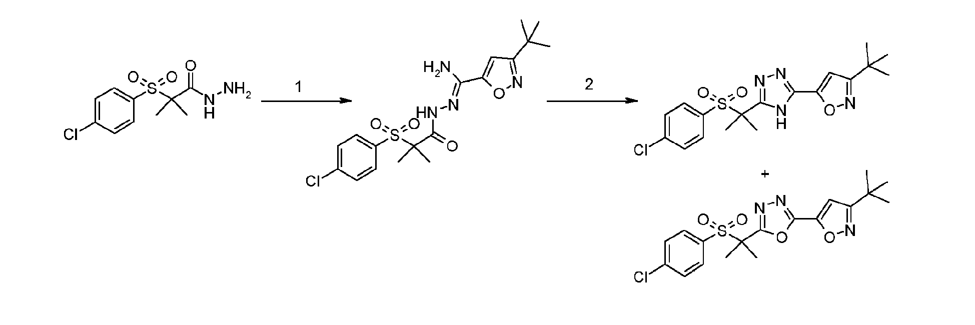

1 NaOMe, MeOH, reflux
2 o-xylene, 140 °C

### Synthesis of 2-(4-chloro-benzenesulfonyl)-2-methyl-propionic acid [1-amino-1-(3-tertbutyl-isoxazol-5-yl)-methylidene]-hydrazide

To a solution of sodium methoxide (42 mg, 0.77 mmol) and **3-tert-butyl-isoxazole-5-carboxamidine** (Intermediate 13) (127 mg, 0.76 mmol) in methanol (5 mL) is added a solution of 2-(4-chloro-benzenesulfonyl)-2-methyl-propionic acid hydrazide (Intermediate 12) (197 mg, 0.76 mmol) in methanol (5 mL). The mixture is heated at reflux for 3 h. After this time, the mixture is concentrated under reduced pressure and the residue purified by mass triggered preparative HPLC to afford the title compound as a yellow oil (39 mg) which is used without further purification.

### Synthesis of 3-(3-tert-butyl-isoxazol-5-yl)-5-[1-(4-chloro-benzenesulfonyl)-1-methyl-ethyl]-4H-[1,2,4]triazole (Example 13 in Table 7) and 2-(3-tert-butyl-isoxazol-5-yl)-5-[1-(4-chlorobenzenesulfonyl)-1-methyl-ethyl] [1,3,4]oxadiazole (Example 22 in Table 7)

A solution of crude 2-(4-chloro-benzenesulfonyl)-2-methyl-propionic acid [1-amino-1-(3-tert-butyl-isoxazol-5-yl)-methylidene]-hydrazide (39 mg, ∼0.09 mmol) in o-xylene (3 mL) is heated at 140 °C for 1 h. After this time, the reaction is cooled to room temperature and concentrated under reduced pressure. The residue is purified by chromatography on silica eluting with a heptane/ethyl acetate gradient (1/0 to 7/3) 3-(3-tert-butyl-isoxazol-5-yl)-5-[1-(4-chlorobenzenesulfonyl)-1-methyl-ethyl]-4H-[1,2,4]triazole and 2-(3-tert-butyl-isoxazol-5-yl)-5-[1-(4-chloro-benzenesulfonyl)-1-methyl-ethyl] [1,3,4]oxadiazole (5.6 mg, 2%), m/z 410 [M+H⁺]. ¹H NMR (360 MHz, *CHLOROFORM-d*) δ ppm 1.41 (9 H, s), 1.94 (6 H, s), 6.99 (1 H, s), 7.46 - 7.60 (4 H, m).

The reaction is repeated using 69 mg of crude 2-(4-chloro-benzenesulfonyl)-2-methyl-propionic acid [1-amino-1-(3-tert-butyl-isoxazol-5-yl)-methylidene]-hydrazide (∼0.16 mmol) and the 3-(3-tert-butyl-isoxazol-5-yl)-5-[1-(4-chloro-benzenesulfonyl)-1-methyl-ethyl]-4H-[1,2,4]triazole obtained after chromatography is combined with the material from the first reaction and repurified by chromatography using the same conditions to give the desired product as a white solid (7.1 mg, 1 %), m/z 409 [M+H⁺]. ¹H NMR (360 MHz, *CHLOROFORM-d*) δ ppm 1.38 (9 H, s), 1.88 (6 H, s), 6.73 (1 H, s), 7.40 - 7.52 (4 H, m).

Examples listed in Table 7 Method D-1 are prepared according to a similar procedure.procedure with the following modifications noted:
- Compound 14 is purified by chromatography on silica eluting with a heptane/ethyl acetate gradient (1/0 to 1/1).
- For examples 44 to 50, no purification by mass triggered preparative HPLC is attempted after the first step. The cyclisation stage is carried out at 140 °C for 1.5 to 3 h and additional purification steps are required. For examples 44, 47 and 50, after purification by chromatography on silica eluting with a heptane/ethyl acetate gradient (1/0 to 6/4), the compounds are purified by preparative HPLC. Compounds 45, 46 and 48 require a second purification by chromatography on silica eluting with a dichloromethane/ethyl acetate gradient (1/0 to 6/4) before preparative HPLC (examples 45 and 46) or recrystallisation in 1/1 ethyl acetate/heptane (example 48).
- Example 49 is purified by preparative HPLC only.

### Method D-2:

### Synthesis of 4-{2-[5-(5-tert-butyl-2-methyl-2H-pyrazol-3-yl)-4H-[1,2,4]triazol-3-yl]-propane-2-sulfonylmethyl}-cyclohexanol (Example 51 in Table 7)

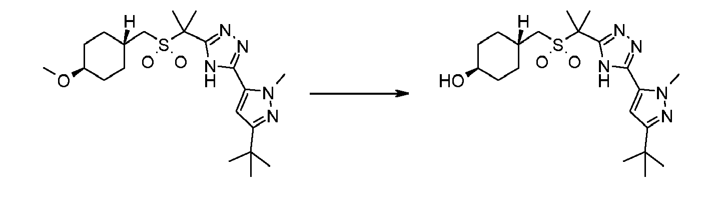

AlBr₃, EtSH, r.t.

The title compound is prepared from example 50 by those skilled in the art by adaptation of a literature procedure (van Muijlwijk-Koezen et al, J. Mend Chem. 2001, 44, 5, 749-62).

To a solution of 3-(5-tert-butyl-2-methyl-2H-pyrazol-3-yl)-5-[1-(4-methoxycyclohexylmethanesulfonyl)-1-methyl-ethyl]-4H-[1,2,4]triazole (100 mg, 0.23 mmol) in ethanethiol (5 mL) is added under nitrogen aluminium tribromide (721 mg, 2.70 mmol) and the mixture is stirred at room temperature for 3 h. The reaction is then cautiously quenched with a 12N aqueous solution of hydrochloric acid and water (5 mL) is added. The pH is adjusted to 8 with a 2N aqueous solution of sodium hydroxide and the mixture is extracted with ethyl acetate (3 x 15 mL). The organic layers are combined, dried (MgSO₄), filtered and concentrated under reduced pressure. The residue is purified by chromatography on silica eluting with a heptane/ethyl acetate gradient (1/0 to 0/1) to afford the title compound as a off-white solid (32.2 mg, 33%), m/z 424 [M+H⁺]. ¹H NMR (250 MHz, *CHLOROFORM-d*) δ ppm 1.35 (9 H, s), 1.45 - 1.80 (9 H, m), 1.91 (6 H, s), 2.11 (1 H, br. s.), 2.91 (2 H, d), 3.99 (1 H, br. s.), 4.19 (3 H, s), 6.64 (1 H, s)

### Method E

### Synthesis of 5-tert-butyl-3-[1-methyl-1-(4-trifluoromethyl-benzenesulfonyl)-ethyl]-[1,2,4]oxadiazole (Example 15 in Table 7)

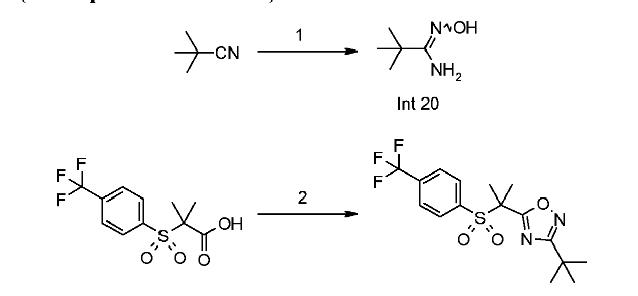

1 NH₂OH.HCl, K₂CO₃, 2/1 water/ethanol, reflux
2 i SOCl₂, cat. DMF, r.t. ; ii Pyridine, 4A MS, 110 °C

### Step 1: Synthesis of N-hydroxy-2,2-dimethyl-propionamidine (Intermediate 20, Table 4)

The title compound is prepared from commercially available materials by those skilled in the art by adaptation of a patent reference (Neighbors et al, US 3,547,621).

To a solution of hydroxylamine hydrochloride (6.80 g, 97.9 mmol) in water (15 mL) is added slowly a solution of potassium carbonate (6.15 g, 44.5 mmol) in water (10 mL) and 2,2-dimethyl propionitrile (6.25 g, 89.0 mmol) in ethanol (50 mL). The mixture is stirred at room temperature for 30 min then heated at reflux for 16 h. After this time, dichloromethane (75 mL) and water (50 mL) are added and the aqueous layer is extracted with dichloromethane (3 x 50 mL). The organic layers are combined, washed with brine (20 mL), dried (Na₂SO₄), filtered and concentrated under reduced pressure to afford the title compound as a white solid (6.15 g, 60%). ¹H NMR (400 MHz, *CHLOROFORM-d*) δ ppm 1.21 (9 H, s), 4.57 (2 H, br. s.), 9.15 (1 H, br. s.).

Intermediates listed in Table 4 are prepared according to a similar procedure with the following modifications noted. Intermediate 22 is synthesised from 3-tert-butyl-isoxazole-5-carbonitrile (intermediate 13, step 4). After 16 h at reflux in ethanol, the solvent is removed under reduced pressure. The residue is taken up in dichloromethane (100 mL) and washed with water (50 mL). The aqueous layer is extracted with dichloromethane (2 x 100 mL) and the organic layers are combined, washed with brine (230 mL), dried (Na₂SO₄), filtered and concentrated under reduced pressure to afford intermediate 22 as a yellow oil which is used without further purification in the next step.

**Table 4: Amidoxime intermediates**

| # | Structure | Name | m/z **[M+H⁺]** | Yield [%] |
|---|---|---|---|---|
| Int 20 | 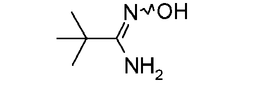 | *N*-hydroxy-2,2-dimethyl-propionamidine | N/A | 60 |
| Int 21 | 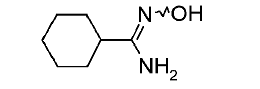 | *N*-hydroxy-cyclohexanecarboxamidine | N/A | 16 |
| Int 22 | 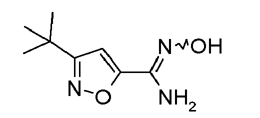 | 3-tert-butyl-*N*-hydroxy-isoxazole-5-carboxamidine | 184 | N/A |

### Step 2: Synthesis of 5-tert-butyl-3-[1-methyl-1-(4-trifluoromethyl-benzenesulfonyl)-ethyl]-[1,2,4]oxadiazole (Example 15 in Table 7)

To a solution of 2-methyl-2-(4-trifluoromethyl-benzenesulfonyl)-propionic acid (200 mg, 0.75 mmol) in anhydrous dichloromethane (2 mL) is added under nitrogen thionyl chloride (326 µL, 532 mg, 4.47 mmol) and *N,N*-dimethylformamide (2 drops). The reaction is stirred at room temperature for 16 h. After this time, the mixture is concentrated under reduced pressure and the crude acid chloride is used without further purification.

To a solution of the acid chloride (∼0.75 mmol) in anhydrous pyridine (1 mL) are added N-hydroxy-2,2-dimethyl-propionamidine (Intermediate 20) (87 mg, 0.75 mmol) and 4Ǻ molecular sieves. The mixture is heated at 110 °C for 3 h. After this time, the mixture is cooled to room temperature and concentrated under reduced pressure. The residue is suspended in dichloromethane (15 mL), washed with a saturated aqueous solution of sodium bicarbonate (5 mL), brine (5 mL), dried (Na₂SO₄), filtered and concentrated under reduced pressure. The residue is purified by chromatography on silica eluting with 1/1 dichloromethane/heptane to provide the title compound as a white solid (171.1 mg, 61%), m/z 377 [M+H⁺]. ¹H NMR (400 MHz, *CHLOROFORM-d*) δ ppm 1.26 (9 H, s), 1.90 (6 H, s), 7.75 (4 H, s).

Examples listed in Table 7 Method E are prepared according to a similar procedure, with the following modifications noted:
- For example 17, the compound is purified by chromatography on silica eluting with 9/1 dichloromethane/heptane.
- For examples 34 to 36, the acid chloride formation is achieved with oxalyl chloride and the cyclisation in pyridine at 100 °C without addition of 4Å molecular sieves. Compounds 34 and 35 are purified by chromatography on silica eluting with dichloromethane followed by trituration in cyclohexane (2 x 6 mL). Example 36 is purified by chromatography on silica eluting with 99.5/0.5 dichloromethane/methanol.

### Method F

### Synthesis of 5-(5-tert-butyl-2-methyl-2H-pyrazol-3-yl)-3-[1-(4-chloro-benzenesulfonyl)-1-methyl-ethyl]-[1,2,4]oxadiazole (Example 18 in Table 7)

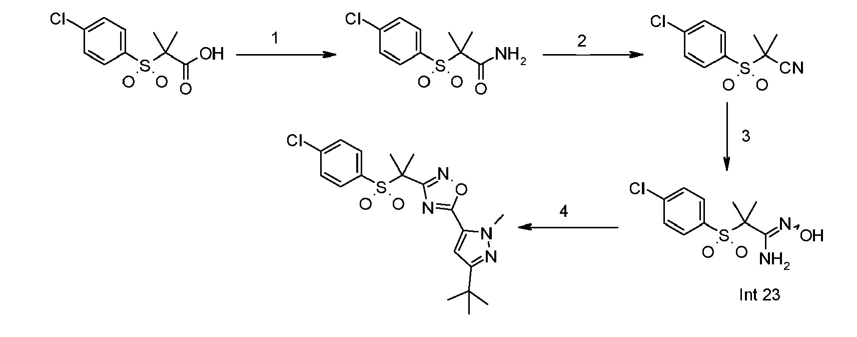

1 i (COCl)₂, cat. DMF, DCM, r.t.; ii NH₄OH, DCM, 0 °C - r.t
2 POCl₃, 60 °C
3 NH₂OH.HCl, K₂CO₃, 2/1 EtOH/water, reflux
4 Pyridine, 80 °C

### Step 1: Synthesis of 2-(4-chloro-benzenesulfonyl)-2-methyl-propionamide

To a solution of 2-(4-chloro-benzenesulfonyl)-2-methyl-propionic acid (5.08 g, 19.3 mmol) in dichloromethane (50 mL) is added oxalyl chloride (9.7 mL, 14.36 g, 113.6 mmol) and *N,N-*dimethylformamide (5 drops). The mixture is stirred at room temperature for 16 h. After this time, the mixture is concentrated under reduced pressure and the crude acid chloride product is used without further purification.

To a vigorously stirred aqueous solution of ammonium hydroxide (100 mL) at 0 °C is added a solution of the crude acid chloride (∼19.3 mmol) in dichloromethane (50 mL) dropwise. The mixture is stirred at 0 °C for 1 h and then the reaction mixture is extracted with dichloromethane (3 x 50 mL). The organic layers are combined, washed with brine (50 mL), dried (Na₂SO₄), filtered and concentrated under reduced pressure to afford the title compound as a white solid (4.88 g, 97%), m/z 262 [M+H⁺]. ¹H NMR (400 MHz, *CHLOROFORM-d*) δ ppm 1.59 (6 H, s), 5.72 (1 H, br. s.), 6.94 (1 H, br. s.), 7.50 - 7.60 (2 H, m), 7.77 - 7.88 (2 H, m).

### Step 2: Synthesis of 2-(4-chloro-benzenesulfonyl)-2-methyl-propionitrile

2-(4-Chloro-benzenesulfonyl)-2-methyl-propionamide (4.88 g, 18.6 mmol) is dissolved in phosphorus oxychloride (50 mL) and the solution is heated at 60 °C for 16 h. After this time, the mixture is concentrated under reduced pressure and the residue dissolved in ethyl acetate (150 mL). The solution is washed with a saturated aqueous solution of sodium bicarbonate (3 x 50 mL), brine (50 mL), dried (Na₂SO₄), filtered and concentrated under reduced pressure to give the title compound as a yellow solid (4.41 g, 97%), m/z 244 [M+H⁺]. ¹H NMR (400 MHz, *CHLOROFORM-d*) δ ppm 1.73 (6 H, s), 7.57 - 7.68 (2 H, m), 7.92 - 8.01 (2 H, m).

### Step 3: Synthesis of 2-(4-chloro-benzenesulfonyl)-N-hydroxy-2-methyl-propionamidine (Intermediate 23, Table 5)

The title compound is prepared from 2-(4-chloro-benzenesulfonyl)-2-methyl-propionitrile by those skilled in the art by adaptation of a patent reference (Neighbors et al, US 3,547,621).

To a solution of hydroxylamine hydrochloride (77.8 mg, 1.12 mmol) and 2-(4-chlorobenzenesulfonyl)-2-methyl-propionitrile (236 mg, 0.97 mmol) in 2/1 ethanol/water (9 mL) is added slowly potassium carbonate (78.4 g, 0.57 mmol). The mixture is stirred at room temperature for 30 min then heated to reflux where it is maintained for 16 h. After this time, the mixture is cooled to room temperature, and concentrated under reduced pressure to remove ethanol. The residue is diluted with water (10 mL) and extracted with dichloromethane (3 x 25 mL). The organic layers are combined, dried (Na₂SO₄), filtered and concentrated under reduced pressure to give the title compound which is used without further purification in the next step.

### Synthesis of N-hydroxy-2-methyl-2-(tetrahydro-pyran-4-ylmethanesulfonyl)-propionamidine (Intermediate 24, Table 5)

The synthesis is done in the same manner as the synthesis of 2-(4-chloro-benzenesulfonyl)-*N-*hydroxy-2-methyl-propionamidine (Intermediate 23) with the following modifications noted. Additional hydroxylamine hydrochloride (1.1 eq) and potassium carbonate (1.1 eq) are added to the solution and the mixture is heated at reflux for a further 8 h to obtain complete conversion. The mixture is concentrated under reduced pressure to remove ethanol, diluted with water (3 mL), saturated with sodium chloride and extracted with ethyl acetate (3 x 25 mL). The organic layers are combined, dried (Na₂SO₄), filtered and concentrated under reduced pressure to give the title compound which is used without further purification in the next step.

### Synthesis of 2-(4-fluoro-benzenesulfonyl)-N-hydroxy-2-methyl-propionamidine

### (Intermediate 25, Table 5)

The synthesis is done using a similar procedure as for intermediate 23 with the following modifications noted. Additional hydroxylamine hydrochloride (0.6 eq) and potassium carbonate (0.3 eq) are added to the solution and the mixture is heated at reflux for a further 16 h. The mixture is concentrated under reduced pressure to remove ethanol, extracted with dichloromethane (3 x 50 mL). The organic layers are combined, dried (Na₂SO₄), filtered and concentrated under reduced pressure to give the title compound which is used without further purification in the next step.

**Table 5: Propionamide intermediates**

| # | Structure | Name | m/z [M+H⁺] | Yield [%] |
|---|---|---|---|---|
| Int 23 | 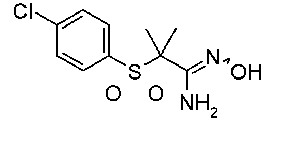 | 2-(4-chloro-benzenesulfonyl)-*N*-hydroxy-2-methyl-propionamidine | 277 | N/A |
| Int 24 | 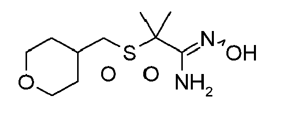 | *N*-hydroxy-2-methyl-2-(tetrahydro-pyran-4-ylmethanesulfonyl)-propionamidine | 265 | N/A |
| Int 25 | 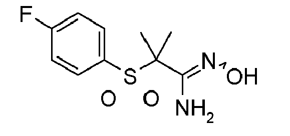 | 2-(4-fluoro-benzenesulfonyl)-*N*-hydroxy-2-methyl-propionamidine | 261 | N/A |

### Step 4: Synthesis of 5-(5-tert-butyl-2-methyl-2H-pyrazol-3-yl)-3-[1-(4-chlorobenzenesulfonyl)-1-methyl-ethyl]-[1,2,4]oxadiazole (Example 18 in Table 7)

To a solution of 2-(4-chloro-benzenesulfonyl)-*N*-hydroxy-2-methyl-propionamidine (Intermediate 23) (∼0.97 mmol) in anhydrous pyridine (10 mL) is added 3-(tert-butyl)-1-methyl-1H-pyrazole-5-carbonyl chloride (196 mg, 0.97 mmol). The mixture is heated at 80 °C for 16 h. After this time, the mixture is cooled to room temperature and concentrated under reduced pressure. The residue is suspended in ethyl acetate (50 mL) washed with a saturated aqueous solution of sodium bicarbonate (10 mL), dried (Na₂SO₄), filtered and concentrated under reduced pressure. The residue is purified first by chromatography on silica eluting with an ethyl acetate/heptane gradient (0/1 to 4/6) and then by mass-triggered preparative HPLC. The purified product is converted to the freebase using Ambersep 900-OH resin to provide the title compound as a white solid (33.6 mg, 9%), m/z 423 [M+H⁺].¹H NMR (400 MHz, *CHLOROFORM-d*) δ ppm 1.35 (9 H, s), 1.89 (6 H, s), 4.13 (3 H, s), 6.86 (1 H, s), 7.48 (2 H, d, *J*=8.56 Hz), 7.63 (2 H, d, *J*=8.80 Hz).

### Synthesis of 5-(5-tert-butyl-2-methyl-2H-pyrazol-3-yl)-3-[1-methyl-1-(tetrahydro-pyran-4-ylmethanesulfonyl)-ethyl]-[1,2,4]oxadiazole (Example 19 in Table 7)

The title compound is prepared using a similar procedure to the synthesis of 5-(5-tert-butyl-2-methyl-2H-pyrazol-3-yl)-3-[1-(4-chloro-benzenesulfonyl)-1-methyl-ethyl]-[1,2,4]oxadiazole (Example 18) with the following modifications noted. The cyclisation stage is carried out at 100 °C for 5 h. After column chromatography, the solid is dissolved in dichloromethane (10 mL) and washed with a saturated aqueous solution of sodium bicarbonate (2 x 5 mL) and then purified by mass-triggered preparative HPLC before being converted to the freebase using Ambersep 900-OH resin to provide the title compound as a cream solid (80.4 mg, 22%), m/z 411 [M+H⁺]. ¹H NMR (360 MHz, *CHLOROFORM-d*) δ ppm 1.35 (9 H, s), 1.41 - 1.58 (2 H, m), 1.81 - 1.89 (2 H, m), 1.92 (6 H, s), 2.27 - 2.49 (1 H, m), 3.16 (2 H, d, *J*=6.58 Hz), 3.43 (2 H, td, *J*=11.92, 2.04 Hz), 3.89 - 4.00 (2 H, m), 4.25 (3 H, s), 6.91 (1 H, s).

Examples listed in Table 7 Method F are prepared according to a similar procedure.

### Method G

### Synthesis of 3-tert-butyl-5-{2-[1-(4-chloro-benzenesulfonyl)-1-methyl-ethyl]-3H-imidazol-4-yl}-1-methyl-1H-pyrazole (Example 20 in Table 7)

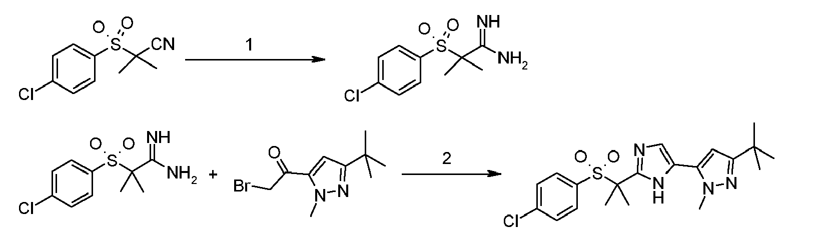

1 i NaH, MeOH, 0 - 40 °C; ii NH₄Cl, 70 °C
2 i-PrOH, 70 °C

### Step 1: Synthesis of 2-(4-chloro-benzenesulfonyl)-2-methyl-propionamidine

To methanol (10 mL) at 0 °C under nitrogen is added sodium hydride (60% dispersion in mineral oil, 492 mg, 12.3 mmol) in portions. The suspension is stirred for 10 min at 0 °C then a solution of 2-(4-chloro-benzenesulfonyl)-2-methyl-propionitrile (300 mg, 1.2 mmol) in methanol (10 mL) is added and the mixture is heated at 30 °C for 2 h. After this time, solid ammonium chloride (1.96 g, 36.9 mmol) is added and the mixture is heated at 70 °C for 16 h. The reaction is poured onto a 0.5N aqueous solution of hydrochloric acid (60 mL) and washed with tert-butyl dimethyl ether (2 x 30 mL). The pH of the aqueous layer is adjusted to ∼9 with a saturated aqueous solution of sodium carbonate. The mixture is extracted with ethyl acetate (6 x 30 mL) and the combined organic fractions are washed with brine (30 mL) dried (Na₂SO₄), filtered and concentrated under reduced pressure to give the title compound as a cream solid (120 mg, 37%), m/z 261 [M+H⁺]. ¹H NMR (400 MHz, *CHLOROFORM-d*) δ ppm 1.63 (6 H, s), 7.46 - 7.59 (2 H, m), 7.76 - 7.90 (2 H, m).

### Step 2: Synthesis of 3-tert-butyl-5-{2-[1-(4-chloro-benzenesulfonyl)-1-methyl-ethyl]-3H-imidazol-4-yl}-1-methyl-1H-pyrazole (Example 20 in Table 7)

The title compound is prepared from 2-(4-chloro-benzenesulfonyl)-2-methyl-propionamidine by those skilled in the art by adaptation of a literature reference (Gueilffier et al, J. Heterocyclic Chem., 1990, 27, 2, 421-5).

To a solution of 2-bromo-1-(5-tert-butyl-2-methyl-2H-pyrazol-3-yl)-ethanone (60 mg, 0.23 mmol) in isopropanol (1.2 mL) is added 2-(4-chloro-benzenesulfonyl)-2-methyl-propionamidine (60 mg, 0.23 mmol). The mixture is heated at 70 °C for 3 h. After this time, the mixture is dissolved in dichloromethane (5 mL) and the solution is washed with brine (2 mL), dried (Na₂SO₄), filtered and concentrated under reduced pressure. The residue is purified by chromatography on silica eluting with a dichloromethane/ethyl acetate gradient (1/0 to 1/1) to provide the title compound as a yellow oil 19 mg, 20%)" m/z 421 [M+H⁺]. ¹H NMR (400 MHz, *CHLOROFORM-d*) δ ppm 1.31 (9 H, s), 1.84 (6 H, s), 3.79 (3 H, s), 6.14 (1 H, s), 7.29 (1 H, d, *J*=2.08 Hz), 7.31 - 7.37 (2 H, m), 7.37 - 7.45 (2 H, m), 9.91 (1 H, br. s.).

Examples listed in Table 7 Method G are prepared according to a similar procedure.

### Method H

### Synthesis of 3-tert-butyl-5-{5-[1-(4-chloro-benzenesulfonyl)-1-methyl-ethyl]-1H-imidazol-2-yl}-1-methyl-1H-pyrazole (Example 21 in Table 7)

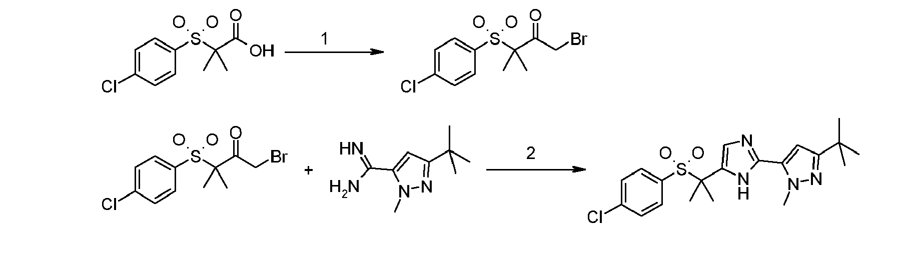

1 i (COCl)₂, cat. DMF, DCM, r.t.; ii MeOH, DCM, r.t.; iii CH₂Br₂, MeLi, THF, -78 °C
2 i-PrOH, 70 °C

### Step 1: Synthesis of 1-bromo-3-(4-chloro-benzenesulfonyl)-3-methyl-butan-2-one

To a solution of 2-(4-chloro-benzenesulfonyl)-2-methyl-propionic acid (2.0 g, 7.61 mmol) in dichloromethane (40 mL) is added oxalyl chloride (3.92 mL, 5.8 g, 45.68 mmol) and *N,N-*dimethylformamide (5 drops). The mixture is stirred at room temperature for 16 h. After this time, the mixture is concentrated under reduced pressure and the crude acid chloride product is used without further purification.

Methanol (15 mL) is added to a solution of the crude acid chloride (∼7.61 mmol) in dichloromethane (40 mL) and the mixture is stirred at room temperature for 1 h. The mixture is concentrated under reduced pressure and the residue dissolved in dichloromethane (30 mL). The solution is washed with an aqueous solution of sodium bicarbonate (5 mL), dried (Na₂SO₄) filtered and concentrated under reduced pressure to afford 2-(4-chloro-benzenesulfonyl)-2-methyl-propionic acid methyl ester (2.1 g, 100%) which is used without further purification.

To a solution of crude methyl ester (2.1 g, 7.61 mmol) in tetrahydrofuran (40 mL) under nitrogen is added dibromomethane (1.07 mL, 2.65 g, 15.23 mmol). The mixture is cooled to -78 °C and methyl lithium (9.52 mL of a 1.6M solution in diethyl ether, 15.23 mmol) is added dropwise. The solution is stirred at -78 °C for 1 h and then quenched by the addition of acetic acid (1.74 mL, 1.83 g, 30.45 mmol). The mixture is warmed to 0 °C and poured onto ice/water (200 mL) and extracted with tert-butyl methyl ether (3 x 400 mL). The organic layers are combined, dried (Na₂SO₄), filtered and concentrated under reduced pressure. The residue is purified by chromatography on silica eluting with 85/10/5 cyclohexane/ethyl acetate/toluene to provide the title compound as a white solid (731.7 mg, 23%). ¹H NMR (250 MHz, *CHLOROFORM-d*) δ ppm 1.59 (6 H, s), 4.54 (2 H, s), 7.44 - 7.58 (2 H, m), 7.58 - 7.70 (2 H, m).

### Step 2: Synthesis of 3-tert-butyl-5-{5-[1-(4-chloro-benzenesulfonyl)-1-methyl-ethyl]-1H-imidazol-2-yl}-1-methyl-1H-pyrazole (Example 21 in Table 7)

The title compound is prepared from 2-(4-chloro-benzenesulfonyl)-2-methyl-propionic acid by those skilled in the art by adaptation of a literature procedure (Kaluza et al, Tetrahedron, 2003, 59, 31, 5893-5903).

To a solution of 1-bromo-3-(4-chloro-benzenesulfonyl)-3-methyl-butan-2-one (439 mg, 1.29 mmol) in isopropanol (4 mL) is added 5-tert-butyl-2-methyl-2H-pyrazole-3-carboxamidine (225 mg, 1.25 mmol). The mixture is heated at 70 °C for 3 h. After this time, the mixture is concentrated under reduced pressure and the residue is dissolved in dichloromethane (25 mL). The solution is washed with a saturated aqueous solution of sodium bicarbonate (10 mL), brine (10 mL), dried (Na₂SO₄), filtered and concentrated under reduced pressure. The residue is purified by chromatography on silica eluting with a dichloromethane/ethyl acetate gradient (1/0 to 1/1). The reaction is repeated using 322 mg of 1-bromo-3-(4-chloro-benzenesulfonyl)-3-methyl-butan-2-one (0.95 mmol) and the materials obtained after column chromatography are combined. Purification by mass-triggered preparative HPLC followed by preparative HPLC provides the title compound as an off-white solid (17.2 mg, 2%), m/z 421 [M+H⁺]. ¹H NMR (250 MHz, *MeOD*) δ ppm 1.28 (9 H, m), 1.75 (6 H, s), 3.82 (3 H, s), 6.43 (1 H, s), 7.12 (1 H, s), 7.38-7.52 (4 H, m).

Examples listed in Table 7 Method H are prepared according to a similar procedure.

### Method I

### Synthesis of 2-(3-tert-butyl-isoxazol-5-yl)-5-[1-(4-fluoro-benzenesulfonyl)-1-methyl-ethyl]-[1,3,4]oxadiazole (Example 23 in Table 7)

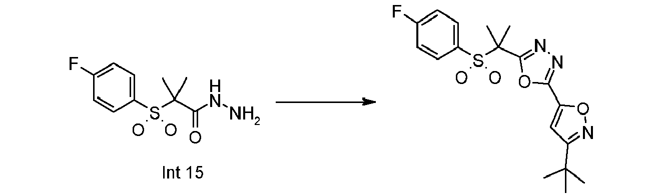

POCl₃, 100 °C

The title compound is prepared from 2-(4-fluoro-benzenesulfonyl)-2-methyl-propionic acid hydrazide by those skilled in the art by adaptation of a literature procedure (Kadi et al, Eur. J. Med. Chem. Chim Ther., 2007, 42, 2, 235-42).

A solution of 2-(4-fluoro-benzenesulfonyl)-2-methyl-propionic acid hydrazide (197 mg, 0.75 mmol) and 3-tert-butyl-isoxazole-5-carbonyl chloride (121 mg, 0.72 mmol) in phosphorus oxychloride (2 mL) is heated in a sealed tube at 100 °C for 3 h. After this time, the reaction mixture is concentrated under reduced pressure and the residue suspended in ethyl acetate (50 mL), washed with a saturated aqueous solution of sodium bicarbonate (2 x 10 mL), dried (Na₂SO₄), filtered and concentrated under reduced pressure. The residue is purified by chromatography on silica eluting with an ethyl acetate/heptane gradient (0/1 to 4/6) to provide the title compound as a white solid (101.9 mg, 34%), m/z 394 [M+H⁺]. ¹H NMR (250 MHz, *CHLOROFORM-d*) δ ppm 1.42 (9 H, s), 1.93 (6 H, s), 6.99 (1 H, s), 7.11 - 7.30 (2 H, m), 7.52 - 7.75 (2 H, m).

Examples listed in Table 7 Method I are prepared according to a similar procedure with the following modifications noted:
- For example 26, 5-tert-butyl-2-methyl-2H-pyrazole-3-carboxylic acid is prepared quantitatively by saponification of the ethyl ester derivative according to the procedure described for intermediate 13 (step 2i) (white solid, 4.5 g, 100%), m/z 170 [M+H⁺]. ¹H NMR (400 MHz, *CHLOROFORM-d*) δ ppm 1.40 (9 H, s), 6.44 (1 H, s), 6.07 (1 H, br. s.).
- For examples 24 to 31, the cyclisation stage is carried out at 100 °C for 6 to 20 h and additional purification steps are required. For example 24, a second chromatography on silica eluting with 4/6 ethyl acetate/heptane is required. For examples 25 and 26, trituration in diethyl ether (example 25) and 1/1 diethyl ether/hexane (example 26) is required. For example 30, purification is achieved by chromatography on silica eluting with an ethyl acetate/heptane gradient (0/1 to 1/1) then with a second chromatograhy on silica with a dichloromethane/ethyl acetate gradient (1/0 to 6/4). Example 31 is purified by chromatography on silica eluting with 7/3 cyclohexane/ethyl acetate followed by preparative HPLC

### Method I-1:

### Synthesis of 2-(5-{5-[1-(4-fluoro-benzenesulfonyl)-1-methyl-ethyl]-[1,3,4]oxadiazol-2-yl}-isoxazol-3-yl)-2-methyl-propan-1-ol (Example 33 in Table 7)

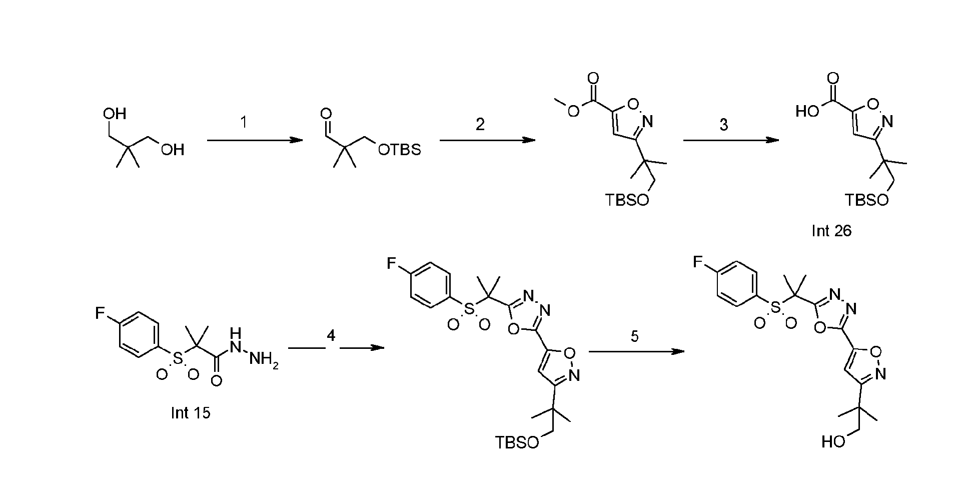

1 i TBSC1, TEA, DMAP, THF, r.t.; ii (COCl)₂, DMSO, TEA, DCM, -78 °C -> r.t.
2 i NH₂OH HCl, NaOH, 1:1 t-butanol/H₂O, r.t.; ii chloramine-T, Cu/CuSO₄, r.t.; iii methyl propiolate, reflux
3 TMSOK, THF, r.t.
4 POCl₃ 100 °C
5 TBAF, THF, r.t.

### Step 1: Synthesis of 3-(tert-butyl-dimethyl-silanyloxy)-2,2-dimethyl-propionaldehyde

A solution of triethylamine (25 mL, 18.15 g, 179 mmol), 2,2-dimethyl-propane-1,3-diol (16.64 g, 160 mmol) and 4-dimethylaminopyridine (0.41 g, cat.) in tetrahydrofuran (100 mL) is stirred at room temperature under nitrogen for 30 min. A solution of tert-butyldimethylsilyl chloride (7.68 g, 51 mmol) in tetrahydrofuran (20 mL) is added and the mixture is stirred at room temperature for 16 h. The solvent is removed under vacuum and the residue taken up in diethyl ether (300 mL), washed with a 5% aqueous solution of acetic acid (100 mL), a saturated aqueous solution of sodium bicarbonate (100 mL), brine (100 mL), dried (MgSO₄), filtered and concentrated under reduced pressure. The residue is purified by chromatography on silica eluting with 3/1 heptane/diethyl ether to give 3-(tert-butyl-dimethyl-silanyloxy)-2,2-dimethyl-propan-1-ol as a yellow oil (8.84 tg, 79%). ¹H NMR (500 MHz, *CHLOROFORM-d*) δ ppm 0.07 (6 H, s), 0.86 - 0.93 (15 H, m), 2.88 (1 H, br. s.), 3.47 (4 H, s).

To a solution of oxalyl chloride (1.9 mL, 2.79 g, 21.98 mmol) in dichloromethane (75 mL) cooled to -78 °C is added dropwise under nitrogen dimethylsulfoxide (1.6 mL, 1.72 g, 21.98 mmol). The solution is stirred at this temperature for 30 min then a solution of 3-(tert-butyldimethyl-silanyloxy)-2,2-dimethyl-propan-1-ol (2 g, 9.16 mmol) in dichloromethane (30 mL) is added dropwise and the mixture is stirred at -78 °C for 1 h. Triethylamine (6.4 mL, 4.63 g, 45.79 mmol) is then added and the mixture is stirred at -78 °C for 1 h before being warmed up to room temperature.

The solution is quenched with water (50 mL) and extracted with diethyl ether (3 x 100 mL). The organic layers are combined, dried (MgSO₄), filtered and concentrated under reduced pressure to provide the title compound as a yellow oil (2.13 g, 86%). ¹H NMR (500 MHz, *CHLOROFORM-d*) δ ppm 0.04 (6 H, s), 0.88 (9 H, s), 1.05 (6 H, s), 3.60 (2 H, s), 9.58 (1 H, s).

**Step 2:** Synthesis of 3-[2-(tert-butyl-dimethyl-silanyloxy)-1,1-dimethyl-ethyl]-isoxazole-5-carboxylic acid methyl ester is done using a similar procedure as described previously for intermediate 13 (step 1) with 3-(tert-butyl-dimethyl-silanyloxy)-2,2-dimethyl-propionaldehyde as starting material (1.34 g, 55%), m/z 314 [M+H⁺]. ¹H NMR (500 MHz, *CHLOROFORM-d*) δ ppm 0.01 (6 H, s), 0.87 (9 H, s), 1.33 (6 H, s), 3.59 (2 H, s), 3.96 (3 H, s), 6.93 (1 H, s).

**Step 3:** Synthesis of 3-[2-(tert-butyl-dimethyl-silanyloxy)-1,1-dimethyl-ethyl]-isoxazole-5-carboxylic acid (Intermediate 26) is done using a similar procedure as described previously for intermediate 13 (step 2i) with 3-[2-(tert-butyl-dimethyl-silanyloxy)-1,1-dimethyl-ethyl]-isoxazole-5-carboxylic acid methyl ester as starting material (635.1 mg, 61%), m/z 300 [M+H⁺]. ¹H NMR (500 MHz, *CHLOROFORM-d*) δ ppm 0.01 (6 H, s), 0.87 (9 H, s), 1.35 (6 H, s), 3.60 (2 H, s), 7.02 (1 H, s).

**Step 4:** Synthesis of 2-{3-[2-(tert-butyl-dimethyl-silanyloxy)-1,1-dimethyl-ethyl]-isoxazol-5-yl}-5-[1-(4-fluoro-benzenesulfonyl)-1-methyl-ethyl]-[1,3,4]oxadiazole is done using a similar procedure as described previously for example 23 with 3-[2-(tert-butyl-dimethyl-silanyloxy)-1,1-dimethyl-ethyl]-isoxazole-5-carboxylic acid (Intermediate 26) as starting material (110.8 mg, 12%), m/z 524 [M+H⁺]. ¹H NMR (500 MHz, *CHLOROFORM-d*) δ ppm 0.03 (6 H, s), 0.89 (9 H, s), 1.39 (6 H, s), 1.93 (6 H, s), 3.63 (2 H, s), 7.05 (1 H, s), 7.17 - 7.24 (2 H, m), 7.61 - 7.67 (2 H, m).

### Step 5: Synthesis of 2-(5-{5-[1-(4-fluoro-benzenesulfonyl)-1-methyl-ethyl]-[1,3,4]oxadiazol-2-yl}-isoxazol-3-yl)-2-methyl-propan-1-ol (Example 33 in Table 7)

The title compound is prepared by those skilled in the art by adaptation of a literature procedure (Corey et al, J. Am. Chem. Soc., 1972, 94, 17, 6190-1).

To a solution of 2-{3-[2-(tert-butyl-dimethyl-silanyloxy)-1,1-dimethyl-ethyl]-isoxazol-5-yl}-5-[1-(4-fluoro-benzenesulfonyl)-1-methyl-ethyl]-[1,3,4]oxadiazole (111 mg, 0.21 mmol) in tetrahydrofuran (2 mL) is added tetrabutylammonium fluoride 1M in tetrahydrofuran (1 mL, 1 mmol) and the solution is stirred at room temperature for 4 h. The solvent is then removed under reduced pressure and the residue purified by chromatography on silica eluting with a heptane/ethyl acetate gradient (2/1 to 1/2) followed by trituration with diethyl ether/heptane to afford the title compound as an off-white solid (39.1 mg, 45%), m/z 410 [M+H⁺]. ¹H NMR (250 MHz, *CHLOROFORM-d)* δ ppm 1.40 (6 H, s), 1.94 (6 H, s), 2.14 (1 H, t, *J*=6.55 Hz), 3.77 (2 H, d, *J*=6.55 Hz), 7.06 (1 H, s), 7.15 - 7.26 (2 H, m), 7.57 - 7.75 (2 H, m).

### Method I-2:

### Synthesis of 2-(5-tert-butyl-2-methyl-2H-pyrazol-3-yl)-5-[1-(4-chloro-benzenesulfonyl)-cyclopropyl]-[1,3,4]oxadiazole (Example 32 in Table 7)

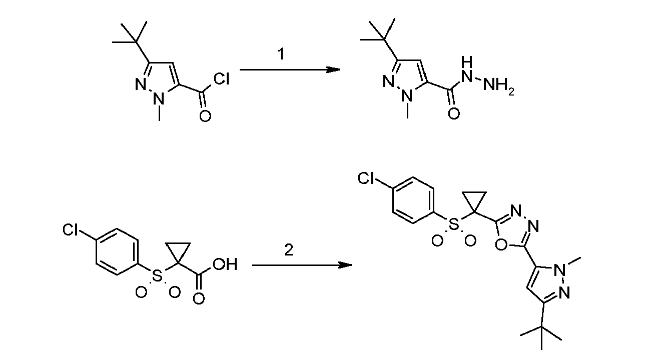

1 i BocHNNH₂, DCM, r.t.; ii 6N HCl, MeOH, r.t.; iii Ambersep 900-OH resin, MeOH, r.t.
2 POCl₃, pyridine, r.t.

### Step 1: Synthesis 5-tert-butyl-2-methyl-2H-pyrazole-3-carboxylic acid hydrazide

The title compound is prepared using a similar procedure as described previously for intermediate 12 (steps ii to iv) with 5-tert-butyl-2-methyl-2H-pyrazole-3-carbonyl chloride as starting material (1.5 g, 88%), m/z 197 [M+H⁺]. ¹H NMR (500 MHz, *CHLOROFORM-d*) δ ppm 1.30 (9 H, s), 4.10 (3 H, s), 6.38 (1 H, s)

### Step 2: Synthesis of 2-(5-tert-butyl-2-methyl-2H-pyrazol-3-yl)-5-[1-(4-chlorobenzenesulfonyl)-cyclopropyl]-[1,3,4]oxadiazole (Example 32 in Table 7)

To a solution of 5-tert-butyl-2-methyl-2H-pyrazole-3-carboxylic acid hydrazide (197 mg, 1.00 mmol) and 1-(4-chloro-benzenesulfonyl)-cyclopropanecarboxylic acid (258 mg, 0.99 mmol) in pyridine (5 mL) is added phosphorus oxychloride (0.18 mL, 0.29 g, 1.92 mmol) and the mixture is stirred at room temperature for 16 h. After this time, the reaction mixture is quenched with a saturated aqueous solution of ammonium chloride (10 mL) and extracted with dichloromethane (3 x 10 mL). The organic layers are combined, dried (MgSO₄), filtered and concentrated under reduced pressure. The residue is purified twice by chromatography on silica eluting with an ethyl acetate/heptane gradient (0/1 to 4/6) followed by preparative HPLC to provide the title compound as a white solid (24.1 mg, 6%), m/z 421 [M+H⁺]. ¹H NMR (500 MHz, *CHLOROFORM-d*) δ ppm 1.35 (9 H, s), 1.66 - 1. 84 (2 H, m), 2.06 - 2.21 (2 H, m), 4.22 (3 H, s), 6.62 (1 H, s), 7.48 - 7.63 (2 H, m), 7.75 - 7.86 (2 H, m).

### Method J:

### Synthesis of 2-(3-tert-butyl-isoxazol-5-yl)-5-[1-(4-fluoro-benzenesulfonyl)-1-methyl-ethyl]-[1,3,4]thiadiazole (Example 39 in Table 7)

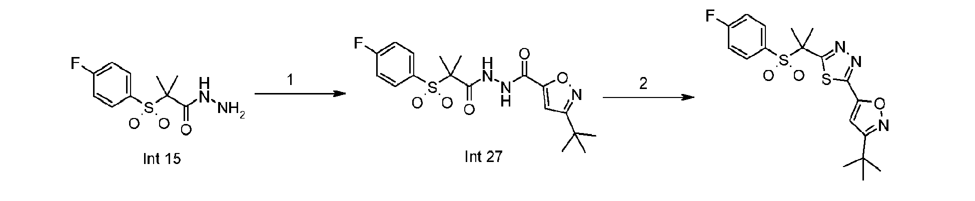

1 POCl₃, pyridine, 0 °C
2 Lawesson's reagent, toluene, reflux

### Step 1: Synthesis of 3-tert-butyl-isoxazole-5-carboxylic acid N'-[2-(4-fluorobenzenesulfonyl)-2-methyl-propionyl]-hydrazide (Intermediate 27, Table 6)

The title compound is prepared from 2-(4-fluoro-benzenesulfonyl)-2-methyl-propionic acid hydrazide (Intermediate 15) by those skilled in the art by adaptation of a literature procedure (Kadi et al, Eur. J Med Chem. Chim Ther., 2007, 42, 2, 235-42).

To a solution of 2-(4-fluoro-benzenesulfonyl)-2-methyl-propionic acid hydrazide (300 mg, 1.15 mmol) and 3-tert-butyl-isoxazole-5-carboxylic acid prepared as described previously (intermediate 13, step 2i) (195 mg, 1.15 mmol) in pyridine (3 mL) is added dropwise under nitrogen and at 0 °C phosphorus oxychloride (106 µL, 177 mg, 1.15 mmol). The mixture is stirred at 0 °C for 3 h and then quenched with a 1N aqueous solution of ammonium chloride (2 mL). The solution is extracted with dichloromethane (3 x 5 mL). The organic layers are combined and washed with a 1N aqueous solution of hydrochloric acid, dried (Na₂SO₄), filtered and concentrated under reduced pressure. The residue is purified by chromatography on silica eluting with 8/2 cyclohexane/ethyl acetate to provide the title compound as a off-white solid (182.4 mg, 39%), m/z 412 [M+H⁺]. ¹H NMR (500 MHz, *CHLOROFORM-d*) δ ppm 1.34 (9 H, s), 1.63 (6 H, s), 6.93 (1 H, s), 7.23 (2 H, t, *J*=8.43 Hz), 8.06 (2 H, dd, *J*=8.74, 5.00 Hz), 9.24 (2 H, br. s.).

Intermediates 27 to 29 listed in Table 6 are prepared according to a similar procedure with the following modifications noted. Intermediate 29 is purified by chromatography on silica eluting with 1/1 cyclohexane/ethyl acetate.

Intermediate 30 is obtained as a by-product of the preparation of example 33 (step 4) and purified by chromatography on silica eluting with a heptane/ethyl acetate gradient (1/0 to 1/1).

**Table 6: Bis-hydrazide intermediates**

| # | Structure | Name | Yield [%] |
|---|---|---|---|
| Int 27 | 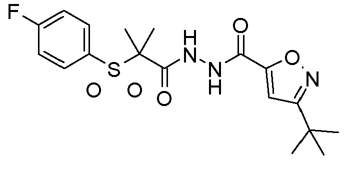 | 3-tert-butyl-isoxazole-5-carboxylic acid *N*'-[2-(4-fluoro-benzenesulfonyl)-2-methyl-propionyl]-hydrazide | 39 |
| Int 28 | 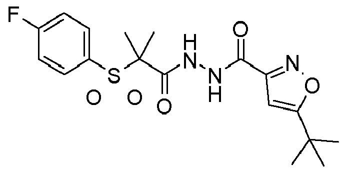 | 5-tert-butyl-isoxazole-3-carboxylic acid *N*'-[2-(4-fluoro-benzenesulfonyl)-2-methyl-propionyl]-hydrazide | 47 |
| Int 29 | 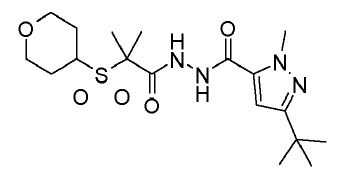 | 5-tert-butyl-2-methyl-2H-pyrazole-3-carboxylic acid *N*'-[2-methyl-2-(tetrahydro-pyran-4-sulfonyl)-propionyl]-hydrazide | 45 |
| Int 30 | 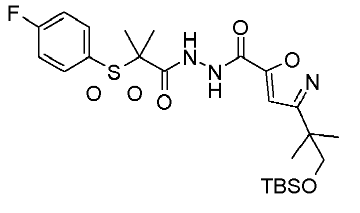 | 3-[2-(tert-butyl-dimethyl-silanyloxy)-1,1-dimethyl-ethyl]-isoxazole-5-carboxylic acid N'-[2-(4-fluoro-benzenesulfonyl)-2-methyl-propionyl]-hydrazide | 13 |

### Step 2: Synthesis of 2-(3-tert-butyl-isoxazol-5-yl)-5-[1-(4-fluoro-benzenesulfonyl)-1-methyl-ethyl]-[1,3,4]thiadiazole (Example 39 in Table 7)

The title compound is prepared from 3-tert-butyl-isoxazole-5-carboxylic acid *N*'-[2-(4-fluorobenzenesulfonyl)-2-methyl-propionyl]-hydrazide by adaptation of a literature precedent (Clitherow et al, Bioorg. Med. Chem. Lett., 1996, 6; 7; 833-8).

To a solution of 3-tert-butyl-isoxazole-5-carboxylic acid *N*'-[2-(4-fluoro-benzenesulfonyl)-2-methyl-propionyl]-hydrazide (182 mg, 0.44 mmol) in toluene (4 mL) is added Lawesson's reagent (359 mg, 0.89 mmol) and the mixture is heated at 110 °C for 2 h. The reaction mixture is concentrated under reduced pressure and the residue is purified by chromatography on silica eluting with dichloromethane followed by trituration in cyclohexane (2 x 3 mL) to provide the title compound as a white solid (90.9 mg, 50%), m/z 410 [M+H⁺]. ¹H NMR (500 MHz, *CHLOROFORM-d*) δ ppm 1.42 (9 H, s), 1.99 (6 H, s), 6.98 (1 H, s), 7.16 (2 H, t, *J*=8.47 Hz), 7.56 (2 H, dd, *J*=8.77, 4.96 Hz).

Examples listed in Table 7 Method J are prepared according to a similar procedure with the following modifications noted. Example 41 is purified by chromatography on silica eluting with 85/15 dichloromethane/ethyl acetate and example 42 is purified twice by chromatography on silica eluting with 7/3 dichloromethane/ethyl acetate then with a dichloromethane/ethyl acetate gradient (1/0 to 8/2).

### Method J-1:

### Synthesis of 2-(5-{5-[1-(4-fluoro-benzenesulfonyl)-1-methyl-ethyl]-[1,3,4]thiadiazol-2-yl}-isoxazol-3-yl)-2-methyl-propan-1-ol (Example 43 in Table 7)

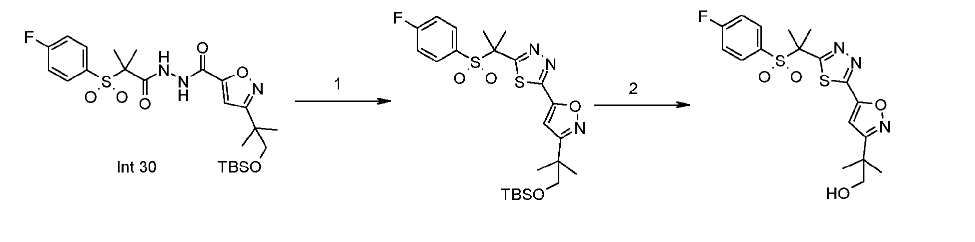

1 Lawesson's reagent, toluene, reflux
2 TBAF, THF, r.t.

### Step 1: Synthesis of 2-[3-[2-(tert-butyl-dimethyl-silanyloxy)-1,1-dimethyl-ethyl]-isoxazol-5-yl}-5-[1-(4-fluoro-benzenesulfonyl)-1-methyl-ethyl]-[1,3,4]thiadiazole

The title compound is prepared using a similar procedure to that described previously for example 39 (step 2) with 3-[2-(tert-butyl-dimethyl-silanyloxy)-1,1-dimethyl-ethyl]-isoxazole-5-carboxylic acid *N*-[2-(4-fluoro-benzenesulfonyl)-2-methyl-propionyl]-hydrazide (Intermediate 30) as starting material. The residue is purified by chromatography on silica eluting with a heptane/ethyl acetate gradient (1/0 to 8/2) to provide the title compound as a white solid (107.9 mg, 83%), m/z 540 [M+H⁺]. ¹H NMR (500 MHz, *CHLOROFORM-d*) δ ppm 0.03 (6 H, s), 0.88 (9 H, s), 1.39 (6 H, s), 1.99 (6 H, s), 3.64 (3 H, s), 7.03 (1 H, s), 7.11 - 7.18 (2 H, m), 7.52 - 7.60 (2 H, m).

### Step 2: Synthesis of 2-(5-{5-[1-(4-fluoro-benzenesulfonyl)-1-methyl-ethyl]-[1,3,4]thiadiazol-2-yl}-isoxazol-3-yl)-2-methyl-propan-1-ol (Example 43 in Table 7)

The title compound is prepared using a similar procedure to that described previously for example 33 (step 5) with 2-{3-[2-(tert-butyl-dimethyl-silanyloxy)-1,1-dimethyl-ethyl]-isoxazol-5-yl}-5-[1-(4-fluoro-benzenesulfonyl)-1-methyl-ethyl]-[1,3,4]thiadiazole as starting material (40.1 mg, 47%), m/z 426 [M+H⁺]. ¹H NMR (250 MHz, *CHLOROFORM-d*) δ ppm 1.36 - 1.45 (6 H, m), 1.58 (6 H, s), 2.19 (1 H, t, *J*=6.62 Hz), 3.77 (2 H, d, *J*=6.70 Hz), 7.03 (1 H, s), 7.10 - 7.22 (2 H, m), 7.50 - 7. 62 (2 H, m).

**Table 7: Examples**

| # | Structure | Name | m/z [M+H +] | Meth od |
|---|---|---|---|---|
| 1 | 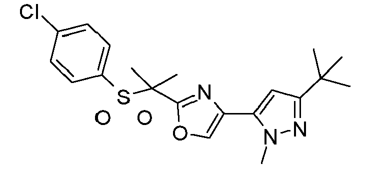 | 4-(5-tert-butyl-2-methyl-2H-pyrazol-3-yl)-2-[1-(4-chloro-benzenesulfonyl)-1-methyl-ethyl]-oxazole | 422 | A |
| 2 | 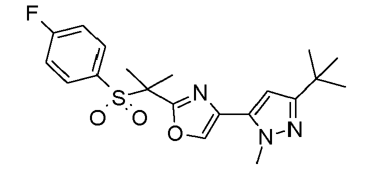 | 4-(5-tert-butyl-2-methyl-2H-pyrazol-3-yl)-2-[1-(4-fluoro-benzenesulfonyl)-1-methyl-ethyl]-oxazole | 406 | A |
| 3 | 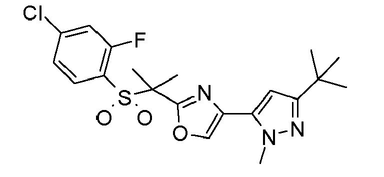 | 4-(5-tert-butyl-2-methyl-2H-pyrazol-3-yl)-2-[1-(4-chloro-2-fluoro-benzenesulfonyl)-1-methyl-ethyl]-oxazole | 440 | A |
| 4 | 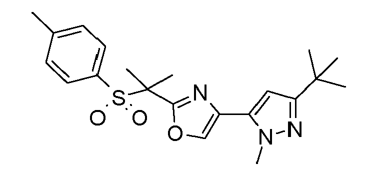 | 4-(5-tert-butyl-2-methyl-2H-pyrazol-3-yl)-2-[1-methyl-1-(toluene-4-sulfonyl)-ethyl]-oxazole | 402 | A |
| 5 | 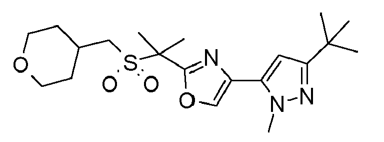 | 4-(5-tert-butyl-2-methyl-2H-pyrazol-3-yl)-2-[1-methyl-1-(tetrahydro-pyran-4-ylmethanesulfonyl)-ethyl]-oxazole | 410 | A |
| 6 | 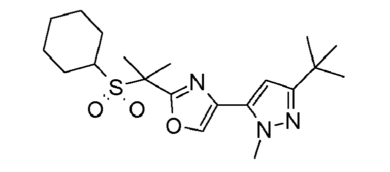 | 4-(5-tert-butyl-2-methyl-2H-pyrazol-3-yl)-2-(1-cyclohexanesulfonyl-1-methyl-ethyl)-oxazole | 394 | A |
| 7 | 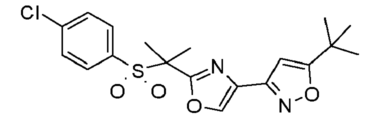 | 5-tert-butyl-3-{2-[1-(4-chloro-benzenesulfonyl)-1-methyl-ethyl]-oxazol-4-yl}-isoxazole | 409 | B |
| 8 | 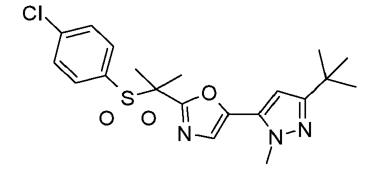 | 5-(5-tert-butyl-2-methyl-2H-pyrazol-3-yl)-2-[1-(4-chloro-benzenesulfonyl)-1-methyl-ethyl]-oxazole | 422 | C |
| 9 | 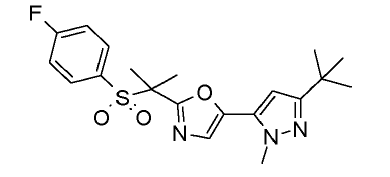 | 5-(5-tert-butyl-2-methyl-2H-pyrazol-3-yl)-2-[1-(4-fluoro-benzenesulfonyl)-1-methyl-ethyl]-oxazole | 406 | C |
| 10 | 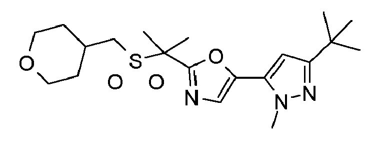 | 5-(5-tert-butyl-2-methyl-2H-pyrazol-3-yl)-2-[1-methyl-1-(tetrahydro-pyran-4-ylmethanesulfonyl)-ethyl]-oxazole | 410 | C |
| 11 | 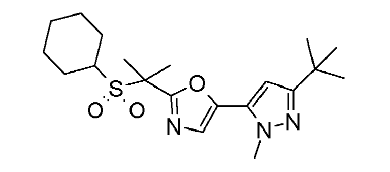 | 4-(5-tert-butyl-2-methyl-2H-pyrazol-3-yl)-2-(1-cyclohexanesulfonyl-1-methyl-ethyl)-oxazole | 394 | C |
| 12 | 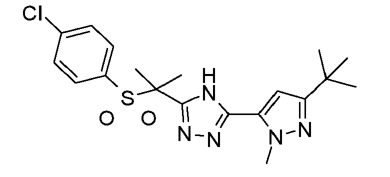 | 3-(5-tert-butyl-2-methyl-2H-pyrazol-3-yl)-5-[1-(4-ch loro-benzenesulfonyl)-1-methyl-ethyl]-4H-[1,2,4]triazole | 422 | D |
| 13 | 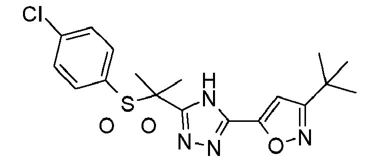 | 3-(3-tert-butyl-isoxazol-5-yl)-5-[1-(4-chloro-benzenesulfonyl)-1-methyl-ethyl]-4H-[1,2,4]triazole | 409 | D-1 |
| 14 | 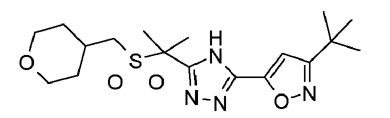 | 3-(3-tert-butyl-isoxazol-5-yl)-5-[1-methyl-1-(tetrahydro-pyran-4-ylmethanesulfonyl)-ethyl]-4H-[1,2,4]triazole | 397 | D-1 |
| 15 | 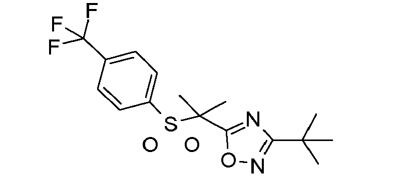 | 3-tert-butyl-5-[1-methyl-1-(4-trifluoromethyl-benzenesulfonyl)-ethyl]-[1,2,4]oxadiazole | 377 | E |
| 16 | 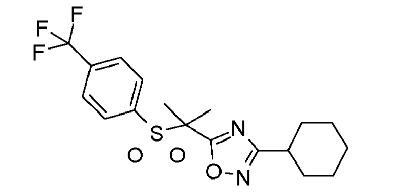 | 3-cyclohexyl-5-[1-methyl-1-(4-trifluoromethyl-benzenesulfonyl)-ethyl]-[1,2,4]oxadiazole | 403 | E |
| 17 | 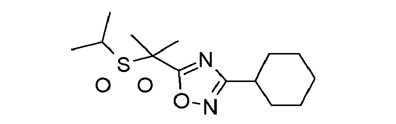 | 3-cyclohexyl-5-[1-methyl-1-(propane-2-sulfonyl)-ethyl]-[1,2,4]oxadiazole | 301 | E |
| 18 | 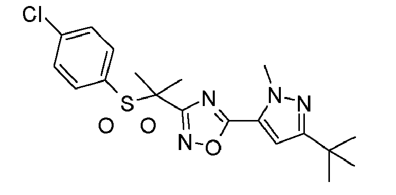 | 5-(5-tert-butyl-2-methyl-2H-pyrazol-3-yl)-3-[1-(4-chloro-benzenesulfonyl)-1-methyl-ethyl]-[1,2,4]oxadiazole | 423 | F |
| 19 | 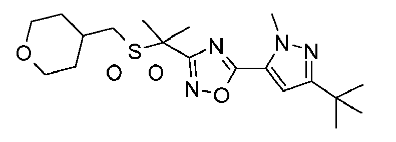 | 5-(5-tert-butyl-2-methyl-2H-pyrazol-3-yl)-3-[1-methyl-1-(tetrahydro-pyran-4-ylmethanesulfonyl)-ethyl]-[1,2,4]oxadiazole | 411 | F |
| 20 | 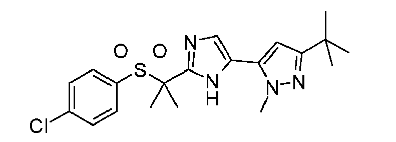 | 3-tert-butyl-5-{2-[1-(4-chloro-benzenesulfonyl)-1-methyl-ethyl]-3H-imidazol-4-yl}-1-methyl-1 H-pyrazole | 421 | G |
| 21 | 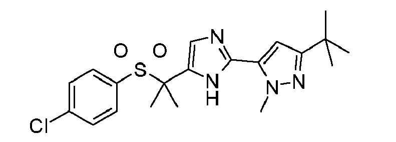 | 3-tert-butyl-5-{5-[1-(4-chloro-benzenesulfonyl)-1-methyl-ethyl]-1H-imidazol-2-yl}-1-methyl-1 H-pyrazole | 421 | H |
| 22 | 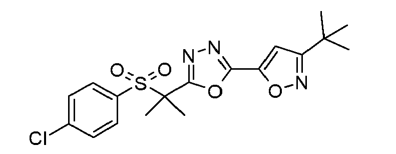 | 2-(3-tert-butyl-isoxazol-5-yl)-5-[1-(4-chloro-benzenesulfonyl)-1-methyl-ethyl]-[1,3,4]oxadiazole | 410 | D-1 |
| 23 | 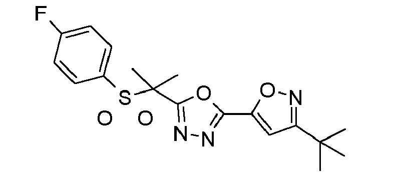 | 2-(3-tert-butyl-isoxazol-5-yl)-5-[1-(4-fluoro-benzenesulfonyl)-1-methyl-ethyl]-[1,3,4]oxadiazole | 394 | I |
| 24 | 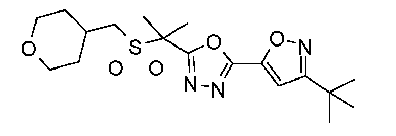 | 2-(3-tert-butyl-isoxazol-5-yl)-5-[1-methyl-1-(tetrahydro-pyran-4-ylmethanesulfonyl)-ethyl]-[1,3,4]oxadiazole | 398 | I |
| 25 | 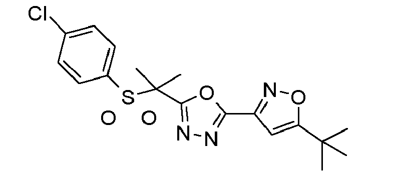 | 2-(5-tert-butyl-isoxazol-3-yl)-5-[1-(4-chloro-benzenesulfonyl)-1-methyl-ethyl]-[1,3,4]oxadiazole | 410 | I |
| 26 | 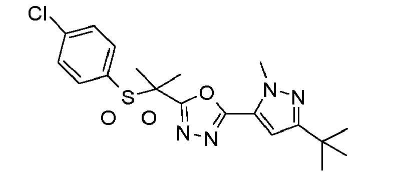 | 2-(5-tert-butyl-2-methyl-2H-pyrazol-3-yl)-5-[1-(4-chloro-benzenesulfonyl)-1-methyl-ethyl]-[1,3,4]oxadiazole | 423 | I |
| 27 | 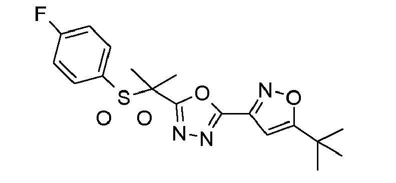 | 2-(5-tert-butyl-isoxazol-3-yl)-5-[1-(4-fluoro-benzenesulfonyl)-1-methyl-ethyl]-[1,3,4]oxadiazole | 394 | I |
| 28 | 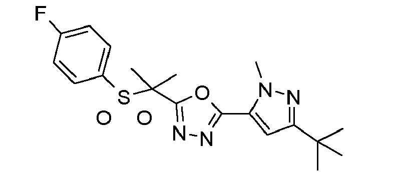 | 2-(5-tert-butyl-2-methyl-2H-pyrazol-3-yl)-5-[1-(4-fluoro-benzenesulfonyl)-1-methyl-ethyl]-[1,3,4]oxadiazole | 407 | I |
| 29 | 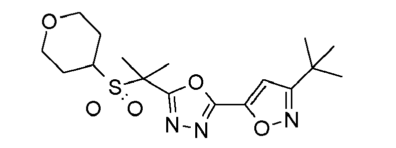 | 2-(3-tert-butyl-isoxazol-5-yl)-5-[1-methyl-1-(tetrahydro-pyran-4-sulfonyl)-ethyl]-[1,3,4]oxadiazole | 384 | I |
| 30 | 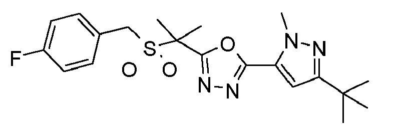 | 2-(5-tert-butyl-2-methyl-2H-pyrazol-3-yl)-5-[1-(4-fluoro-phenylmethanesulfonyl)-1-methyl-ethyl]-[1,3,4]oxadiazole | 421 | I |
| 31 | 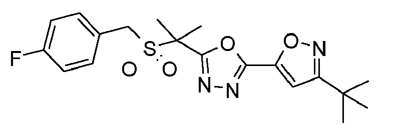 | 2-(3-tert-butyl-isoxazol-5-yl)-5-[1-(4-fluoro-phenylmethanesulfonyl)-1-methyl-ethyl]-[1,3,4]oxadiazole | 408 | I |
| 32 | 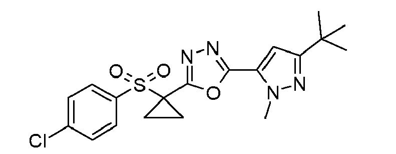 | 2-(5-tert-butyl-2-methyl-2H-pyrazol-3-yl)-5-[1-(4-chloro-benzenesulfonyl)-cyclopropyl]-[1,3,4]oxadiazole | 421 | I-2 |
| 33 | 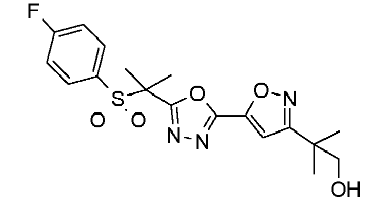 | 2-(5-{5-[1-(4-fluoro-benzenesulfonyl)-1-methyl-ethyl]-[1,3,4]oxadiazol-2-yl}-isoxazol-3-yl)-2-methyl-propan-1-ol | 410 | I-1 |
| 34 | 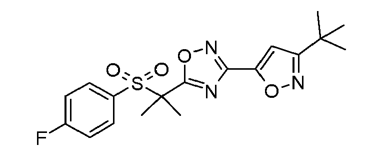 | 3-(3-tert-butyl-isoxazol-5-yl)-5-[1-(4-fluoro-benzenesulfonyl)-1-methyl-ethyl]-[1,2,4]oxadiazole | 394 | E |
| 35 | 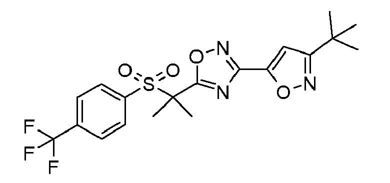 | 3-(3-tert-butyl-isoxazol-5-yl)-5-[1-methyl-1-(4-trifluoromethyl-benzenesulfonyl)-ethyl]-[1,2,4]oxadiazole | 444 | E |
| 36 | 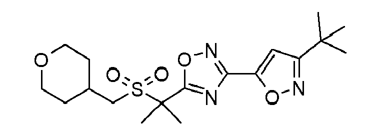 | 3-(3-tert-butyl-isoxazol-5-yl)-5-[1-methyl-1-(tetrahydro-pyran-4-ylmethanesulfonyl)-ethyl]-[1,2,4]oxadiazole | 398 | E |
| 37 | 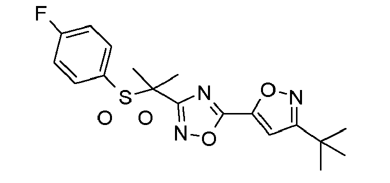 | 5-(3-tert-butyl-isoxazol-5-yl)-3-[1-(4-fluoro-benzenesulfonyl)-1-methyl-ethyl]-[1,2,4]oxadiazole | 394 | F |
| 38 | 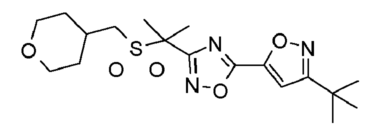 | 5-(3-tert-butyl-isoxazol-5-yl)-3-[1-methyl-1-(tetrahydro-pyran-4-ylmethanesulfonyl)-ethyl]-[1,2,4]oxadiazole | 398 | F |
| 39 | 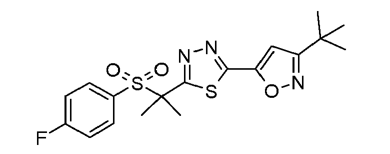 | 2-(3-tert-butyl-isoxazol-5-yl)-5-[1-(4-fluoro-benzenesulfonyl)-1-methyl-ethyl]-[1,3,4]thiadiazole | 410 | J |
| 40 | 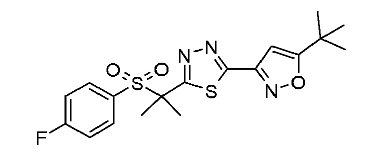 | 2-(5-tert-butyl-isoxazol-3-yl)-5-[1-(4-fluoro-benzenesulfonyl)-1-methyl-ethyl]-[1,3,4]thiadiazole | 410 | J |
| 41 | 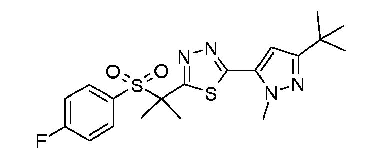 | 2-(5-tert-butyl-2-methyl-2H-pyrazol-3-yl)-5-[1-(4-fluoro-benzenesulfonyl)-1-methyl-ethyl]-[1,3,4]thiadiazole | 423 | J |
| 42 | 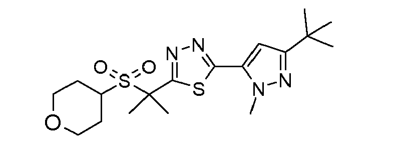 | 2-(5-tert-butyl-2-methyl-2H-pyrazol-3-yl)-5-[1-methyl-1-(tetrahydro-pyran-4-sulfonyl)-ethyl]-[1,3,4]thiadiazole | 413 | J |
| 43 | 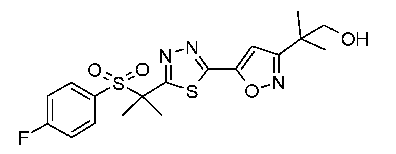 | 2-(5-{5-[1-(4-fluoro-benzenesulfonyl)-1-methyl-ethyl]-[1,3,4]thiadiazol-2-yl}-isoxazol-3-yl)-2-methyl-propan-1-ol | 426 | J-1 |
| 44 | 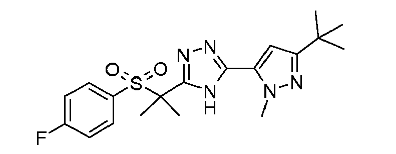 | 3-(5-tert-butyl-2-methyl-2H-pyrazol-3-yl)-5-[1-(4-fluoro-benzenesulfonyl)-1-methyl-ethyl]-4H-[1,2,4]triazole | 406 | D-1 |
| 45 | 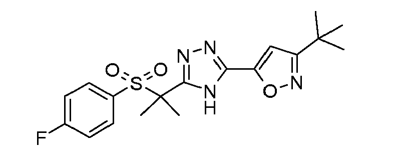 | 3-(3-tert-butyl-isoxazol-5-yl)-5-[1-(4-fluoro-benzenesulfonyl)-1-methyl-ethyl]-4H-[1,2,4]triazole | 393 | D-1 |
| 46 | 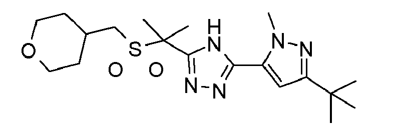 | 3-(5-tert-butyl-2-methyl-2H-pyrazol-3-yl)-5-[1-methyl-1-(tetrahydro-pyran-4-ylmethanesulfonyl)-ethyl]-4H-[1,2,4]triazole | 410 | D-1 |
| 47 | 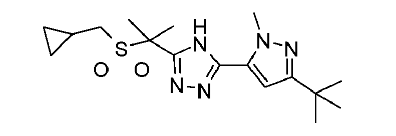 | 3-(5-tert-butyl-2-methyl-2H-pyrazol-3-yl)-5-(1-cyclopropylmethanesulfonyl-1-methyl-ethyl)-4H-[1,2,4]triazole | 366 | D-1 |
| 48 | 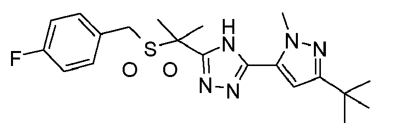 | 3-(5-tert-butyl-2-methyl-2H-pyrazol-3-yl)-5-[1-(4-fluoro-phenylmethanesulfonyl)-1-methyl-ethyl]-4H-[1,2,4]triazole | 420 | D-1 |
| 49 | 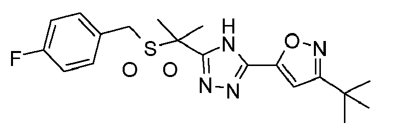 | 3-(3-tert-Butyl-isoxazol-5-yl)-5-[1-(4-fluoro-phenylmethanesulfonyl)-1-methyl-ethyl]-4H-[1,2,4]triazole | 407 | D-1 |
| 50 | 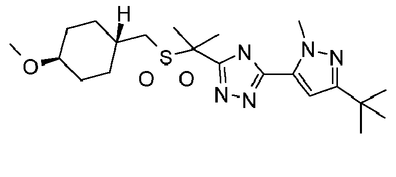 | 3-(5-tert-butyl-2-methyl-2H-pyrazol-3-yl)-5-[1-(4-methoxy-cyclohexylmethanesulfonyl)-1-methyl-ethyl]-4H-[1,2,4]triazole | 438 | D-1 |
| 51 | 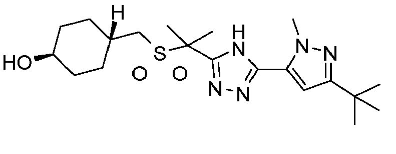 | 4-{2-[5-(5-tert-butyl-2-methyl-2H-pyrazol-3-yl)-4H-[1,2,4]triazol-3-yl]-propane-2-sulfonylmethyl}-cyclohexanol | 424 | D-2 |

### Assessment of Biological Properties

The biological properties of the compounds of the formula I and (IA) were assessed using the assays described below.

### A. Human CB1 and CB2 Receptor Binding-:

Experimental Method:
CB2 membranes were purchased and made from HEK293 EBNA cells stably transfected with human CB2 receptor cDNA (Perkin Elmer Life and Analytical Sciences). CB1 membranes were isolated from HEK cells stably co-transfected with human CB1 receptor and Gα16 cDNA's.
The membrane preparation was bound to scintillation beads (Ysi-Poly-L-lysine SPA beads, GE Healthcare) for 4 hours at room temperature in assay buffer containing 50mM Tris, pH 7.5, 2.5mM EDTA, 5mM MgCl₂, 0.8% fatty acid free Bovine Serum Albumin. Unbound membrane was removed by washing in assay buffer. Membrane-bead mixture was added to 96-well assay plates in the amounts of 15ug membrane per well (CB2) or 2.5ug per well (CB 1) and 1mg SPA bead per well. Compounds were added to the membrane-bead mixture in dose-response concentrations ranging from 1x10⁻⁵ M to 1x10⁻¹⁰ M with 0.25% DMSO, final. The competition reaction was initiated with the addition of ³H-CP55940 (Perkin Elmer Life and Analytical Sciences) at a final concentration of 1.5nM (CB2) or 2.5nM (CB1). The reaction was incubated at room temperature for 18hours and read on TopCount NXT plate reader. Total and non-specific binding was determined in the absence and presence of 1.25uM Win 55212 (Sigma). IC50 values for each compound were calculated as the concentration of compound that inhibits the specific binding of the radioactively labeled ligand to the receptor by 50% using the XLFit 4.1 four parameter logistic model. IC50 values were converted to inhibition constant (Ki) values using Cheng-Prusoff equation.

### B. CB2R mediated modulation of cAMP synthesis:

Compounds of the invention were evaluated for their CB2 agonist or inverse agonistic activity in accordance with the following experimental method. Compounds which were shown to bind to CB2 by the binding assay described above but which were not shown to exhibit CB2R-mediated modulation of cAMP synthesis by this assay were presumed to be CB2 antagonists.

Experimental Method:
CHO cells expressing human CB2R (Euroscreen) were plated at a density of 5000 cells per well in 384 well plates and incubated overnight at 37°C. After removing the media, the cells were treated with test compounds diluted in stimulation buffer containing 1mM IBMX, 0.25% BSA and 10uM Forskolin. The assay was incubated for 30 minutes at 37°C. Cells were lysed and the cAMP concentration was measured using DiscoverX -XS cAMP kit, following the manufacturer's protocol. In this setting, agonists will decrease forskolin induced production of cAMP while inverse agonists will further increase forskolin induced production of cAMP. EC50 of agonists were calculated as follows. The maximal amount of cAMP produced by forskolin compared to the level of cAMP inhibited by 1uM CP55940 is defined as 100%. The EC50 value of each test compound was determined as the concentration at which 50% of the forskolin-stimulated cAMP synthesis was inhibited. Data was analyzed using a four-parameter logistic model. (Model 205 of XLfit 4.0).

### C. CB1R mediated modulation of cAMP synthesis:

Compounds of the invention were evaluated for their CB1 agonist or inverse agonistic activity in accordance with the following experimental method. Compounds which were shown to bind to CB1 by the binding assay described above but which were not shown to exhibit CB1R-mediated modulation of cAMP synthesis by this assay were presumed to be CB1 antagonists.

Experimental Method:
CHO cells expressing human CB1R (Euroscreen) were plated at a density of 5000 cells per well in 384 well plates and incubated overnight at 37°C. After removing the media, the cells were treated with test compounds diluted in stimulation buffer containing 1mM IBMX, 0.25% BSA and 10uM Forskolin. The assay was incubated for 30 minutes at 37°C. Cells were lysed and the cAMP concentration was measured using DiscoverX -XS cAMP kit, following the manufacturer's protocol. In this setting, agonists will decrease forskolin induced production of cAMP while inverse agonists will further increase forskolin induced production of cAMP. EC50 of agonists were calculated as follows. The maximal amount of cAMP produced by forskolin compared to the level of cAMP inhibited by 1uM CP55940 is defined as 100%. The EC50 value of each test compound was determined as the concentration at which 50% of the forskolin-stimulated cAMP synthesis was inhibited. Data was analyzed using a four-parameter logistic model. (Model 205 of XLfit 4.0).

### Compounds Having Agonist Activity

Through the use of the above described assays compounds were found to exhibit agonistic activity and thus to be particularly well suited for the treatment of pain as well as for the treatment of inflammation.

### Therapeutic Use

As can be demonstrated by the assays described above, the compounds of the invention are useful in modulating the CB2 receptor function. By virtue of this fact, these compounds have therapeutic use in treating disease-states and conditions mediated by the CB2 receptor function or that would benefit from modulation of the CB2 receptor function.

As the compounds of the invention modulate the CB2 receptor function, they have very useful anti-inflammatory and immune-suppressive activity and they can be used in patients as drugs, particularly in the form of pharmaceutical compositions as set forth below, for the treatment of disease-states and conditions.

As noted before, those compounds which are CB2 agonists can also be employed for the treatment of pain.

The agonist compounds according to the invention can be used in patients as drugs for the treatment of the following disease-states or indications that are accompanied by inflammatory processes:
(i) Lung diseases: e.g. asthma, bronchitis, allergic rhinitis, emphysema, adult respiratory distress syndrome (ARDS), pigeon fancier's disease, farmer's lung, chronic obstructive pulmonary disease (COPD), asthma including allergic asthma (atopic or non-atopic) as well as exercise-induced bronchoconstriction, occupational asthma, viral- or bacterial exacerbation of asthma, other non-allergic asthmas and "wheezy-infant syndrome", pneumoconiosis, including aluminosis, anthracosis, asbestosis, chalicosis, ptilosis, siderosis, silicosis, tabacosis and byssinosis;
(ii) Rheumatic diseases or autoimmune diseases or musculoskeletal diseases: all forms of rheumatic diseases, especially rheumatoid arthritis, acute rheumatic fever, and polymyalgia rheumatica; reactive arthritis; rheumatic soft tissue diseases; inflammatory soft tissue diseases of other genesis; arthritic symptoms in degenerative joint diseases (arthroses); tendinitis, bursitis, osteoarthritis, traumatic arthritis; collagenoses of any genesis, e.g., systemic lupus erythematosus, scleroderma, polymyositis, dermatomyositis, Sjögren syndrome, Still disease, Felty syndrome; and osteoporosis and other bone resorption diseases;
(iii) Allergic diseases: all forms of allergic reactions, e.g., angioneurotic edema, hay fever, insect bites, allergic reactions to drugs, blood derivatives, contrast agents, etc., anaphylactic shock (anaphylaxis), urticaria, angioneurotic edema, and contact dermatitis;
(iv) Vascular diseases: panarteritis nodosa, polyarteritis nodosa, periarteritis nodosa, arteritis temporalis, Wegner granulomatosis, giant cell arthritis, atherosclerosis, reperfusion injury and erythema nodosum;
(v) Dermatological diseases: e.g. dermatitis, psoriasis; sunburn, burns, eczema;
(vi) Renal diseases: e.g. nephrotic syndrome; and all types of nephritis, e.g., glomerulonephritis; pancreatits;
(vii) Hepatic diseases: e.g. acute liver cell disintegration; acute hepatitis of various genesis, e.g., viral, toxic, drug-induced; and chronically aggressive and/or chronically intermittent hepatitis;
(viii) Gastrointestinal diseases: e.g. inflammatory bowel diseases, irritable bowel syndrome, regional enteritis (Crohns disease), colitis ulcerosa; gastritis; aphthous ulcer, celiac disease, regional ileitis, gastroesophageal reflux disease;
(ix) Neuroprotection: e.g. in the treatment of neurodegeneration following stroke; cardiac arrest; pulmonary bypass; traumatic brain injury; spinal cord injury or the like;
(x) Eye diseases: allergic keratitis, uveitis, or iritis; conjunctivitis; blepharitis; neuritis nervi optici; choroiditis; glaucoma and sympathetic ophthalmia;
(xi) Diseases of the ear, nose, and throat (ENT) area: e.g. tinnitus; allergic rhinitis or hay fever; otitis externa; caused by contact eczema, infection, etc.; and otitis media;
(xii) Neurological diseases: e.g. brain edema, particularly tumor-related brain edema; multiple sclerosis; acute encephalomyelitis; meningitis; acute spinal cord injury; trauma; dementia, particularly degenerative dementia (including senile dementia, Alzheimer's disease; Parkinson's disease and Creutzfeldt-Jacob disease; Huntington's chorea, Pick's disease; motor neuron disease), vascular dementia (including multi-infarct dementia) as well as dementia associated with intracranial space occupying lesions; infections and related conditions (including HIV infection); Guillain-Barre syndrome; myasthenia gravis, stroke; and various forms of seizures, e.g., nodding spasms;
(xiii) Blood diseases: acquired hemolytic anemia; aplastic anemia, and idiopathic thrombocytopenia;
(xiv) Tumor diseases: acute lymphatic leukemia; Hodgkin's disease, malignant lymphoma; lymphogranulomatoses; lymphosarcoma; solid malignant tumors; extensive metastases,;
(xv) Endocrine diseases: endocrine ophthalmopathy; endocrine orbitopathia; thyrotoxic crisis; Thyroiditis de Quervain; Hashimoto thyroiditis; Morbus Basedow; granulomatous thyroiditis; struma lymphomatosa; and Graves disease; type I diabetes (insulin-dependent diabetes);
(xvi) Organ and tissue transplantations and graft-versus-host diseases;
(xvii) Severe states of shock, e.g., septic shock, anaphylactic shock, and systemic inflammatory response syndrome (SIRS);
(xviii) Acute pain such as dental pain, perioperative, post-operative pain, traumatic pain, muscle pain, pain in burned skin, sun burn, trigeminal neuralgia, sun burn; spasm of the gastrointestinal tract or uterus, colics;
(xix) Visceral pain such as pain associated with chronic pelvic pain, pancreatitis, peptic ulcer, interstitial cystitis, renal colic, angina, dysmenorrhoea, menstruation, gynaecological pain, irritable bowel syndrome (IBS), non-ulcer dyspepsia, non-cardiac chest pain, myocardial ischemia;
(xx) Neuropathic pain such as low back pain, non-herpetic neuralgia, post herpetic neuralgia, diabetic neuropathy, nerve injury, acquired immune deficiency syndrome (AIDS) related neuropathic pain, head trauma, painful traumatic mononeuropathy, toxin and chemotherapy induced pain, phantom limb pain, painful polyneuropathy, thalamic pain syndrome, post-stroke pain, central nervous system injury, post surgical pain, stump pain, repetitive motion pain, pain induced by post mastectomy syndrome, multiple sclerosis, root avulsions, postthoracotomy syndrome, neuropathic pain associated hyperalgesia and allodynia.
(xxi) Inflammatory/nociceptive pain induced by or associated with disorders such as osteoarthritis, rheumatoid arthritis, rheumatic disease, teno-synovitis, gout, vulvodynia, myofascial pain (muscular injury, fibromyalgia), tendonitis, osteoarthritis, juvenile arthritis, spondylitis, gouty arthritis, psoriatic arthritis, muscoskeletal pain, fibromyalgia, sprains and strains, sympathetically maintained pain, myositis, pain associated with migraine, toothache, influenza and other viral infections such as the common cold, rheumatic fever, systemic lupus erythematosus;
(xxii) Cancer pain induced by or associated with tumors such as lymphatic leukemia; Hodgkin's disease, malignant lymphoma; lymphogranulomatoses; lymphosarcoma; solid malignant tumors; extensive metastases;
(xxiii) Headache such as cluster headache, migraine with and without aura, tension type headache, headache with different origins, headache disorders including prophylactic and acute use;
(xxiv) various other disease-states or conditions including, restenosis following percutaneous transluminal coronary angioplasty, acute and chronic pain, atherosclerosis, reperfusion injury, congestive heart failure, myocardial infarction, thermal injury, multiple organ injury secondary to trauma, necrotizing enterocolitis and syndromes associated with hemodialysis, leukopheresis, and granulocyte transfusion, sarcoidosis, gingivitis, pyrexia edema resulting from trauma associated with bums, sprains or fracture, cerebral oedema and angioedema, Diabetes such as diabetic vasculopathy, diabetic neuropathy, diabetic retinopathy, post capillary resistance or diabetic symptoms associated with insulitis (e.g. hypergiycemia, diuresis, proteinuria and increased nitrite and kallikrein urinary excretion).

Other indications include: epilepsy, septic shock e.g. as antihypovolemic and/or antihypotensive agents, cancer, sepsis, osteoporosis, benign prostatic hyperplasia and hyperactive bladder, pruritis, vitiligo, general gastrointestinal disorders, disturbances of visceral motility at respiratory, genitourinary, gastrointestinal or vascular regions, wounds, burns, tissue damage and postoperative fever, syndromes associated with itching.

Besides being useful for human treatment, these compounds are also useful for veterinary treatment of companion animals, exotic animals and farm animals, including mammals, rodents, and the like.

For treatment of the above-described diseases and conditions, a therapeutically effective dose will generally be in the range from about 0.01 mg to about 100 mg/kg of body weight per dosage of a compound of the invention; preferably, from about 0.1 mg to about 20 mg/kg of body weight per dosage. For example, for administration to a 70 kg person, the dosage range would be from about 0.7 mg to about 7000 mg per dosage of a compound of the invention, preferably from about 7.0 mg to about 1400 mg per dosage. Some degree of routine dose optimization may be required to determine an optimal dosing level and pattern. The active ingredient may be administered from 1 to 6 times a day.

### General Administration and Pharmaceutical Compositions

When used as pharmaceuticals, the compounds of the invention are typically administered in the form of a pharmaceutical composition. Such compositions can be prepared using procedures well known in the pharmaceutical art and comprise at least one compound of the invention. The compounds of the invention may also be administered alone or in combination with adjuvants that enhance stability of the compounds of the invention, facilitate administration of pharmaceutical compositions containing them in certain embodiments, provide increased dissolution or dispersion, increased inhibitory activity, provide adjunct therapy, and the like. The compounds according to the invention may be used on their own or in conjunction with other active substances according to the invention, optionally also in conjunction with other pharmacologically active substances. In general, the compounds of this invention are administered in a therapeutically or pharmaceutically effective amount, but may be administered in lower amounts for diagnostic or other purposes.

Administration of the compounds of the invention, in pure form or in an appropriate pharmaceutical composition, can be carried out using any of the accepted modes of administration of pharmaceutical compositions. Thus, administration can be, for example, orally, buccally (e.g., sublingually), nasally, parenterally, topically, transdermally, vaginally, or rectally, in the form of solid, semi-solid, lyophilized powder, or liquid dosage forms, such as, for example, tablets, suppositories, pills, soft elastic and hard gelatin capsules, powders, solutions, suspensions, or aerosols, or the like, preferably in unit dosage forms suitable for simple administration of precise dosages. The pharmaceutical compositions will generally include a conventional pharmaceutical carrier or excipient and a compound of the invention as the/an active agent, and, in addition, may include other medicinal agents, pharmaceutical agents, carriers, adjuvants, diluents, vehicles, or combinations thereof. Such pharmaceutically acceptable excipients, carriers, or additives as well as methods of making pharmaceutical compositions for various modes or administration are well-known to those of skill in the art. The state of the art is evidenced, e.g., by Remington: The Science and Practice of Pharmacy, 20th Edition, A. Gennaro (ed.), Lippincott Williams & Wilkins, 2000; Handbook of Pharmaceutical Additives, Michael & Irene Ash (eds.), Gower, 1995; Handbook of Pharmaceutical Excipients, A.H. Kibbe (ed.), American Pharmaceutical Ass'n, 2000; H.C. Ansel and N.G. Popovish, Pharmaceutical Dosage Forms and Drug Delivery Systems, 5th ed., Lea and Febiger, 1990; each of which is incorporated herein by reference in their entireties to better describe the state of the art.

As one of skill in the art would expect, the forms of the compounds of the invention utilized in a particular pharmaceutical formulation will be selected (e.g., salts) that possess suitable physical characteristics (e.g., water solubility) that is required for the formulation to be efficacious.

## Claims

1. A compound of the formula I, wherein 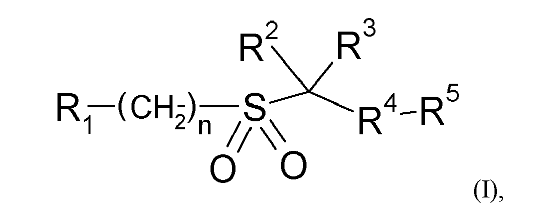 **R¹** is phenyl optionally independently substituted with 1-3 substituents chosen from C₁₋₃ alkyl, C₃₋₆ cycloalkyl, cyano, phenyl, and halogen,
or
**R¹** is C₁₋₆ alkyl, C₃₋₆ cycloalkyl or tetrahydropyranyl optionally substituted with 1-3 substituents chosen from C₁₋₆ alkyl, C₃₋₇ cycloalkyl, C₁₋₆ acyl, cyano, phenyl, oxo, hydroxyl and halogen; each **R¹** and **R¹** substituent where possible is optionally substituted with 1 to 3 halogen atoms; **R² and R³** are methyl or R² and R³ together with the carbon which they are attached to form cyclopropyl;
**R⁴** is oxazolyl, oxadiazolyl, triazolyl, imidazolyl or thiadiazolyl optionally substituted with 1 to 3 substituents chosen from C₁₋₆ alkyl (which is optionally substituted with 1 to 3 halogen atoms), hydroxyl, halogen and cyano;
**R⁵** is cyclohexyl, isoxazolyl or pyrazolyl, each independently substituted with 1 to 3 substituents chosen from C₁₋₆ alkyl (which is optionally substituted with 1 to 3 halogen atoms), C₁₋₆ alkoxy (which is optionally substituted with 1 to 3 halogen atoms), C₁₋₆ cycloalkyl, phenoxy, halogen, cyano, dimethylaminoalkyl, phenyl (which is optionally substituted with 1 to 2 halogen atoms or C₁₋₄ alkyl optionally substituted with halogen), thienyl (which is optionally substituted with 1 to 2 halogen atoms or C₁₋₄ alkyl optionally substituted with halogen), and pyridinyl (which is optionally substituted with 1 to 2 C₁₋₄ alkyl optionally substituted with halogen)
n is 0, 1, 2 or 3;
or a pharmaceutically acceptable salt thereof.

2. The compound according to claim 1 wherein,
**R¹** is C₁₋₄ alkyl, C₃₋₆ cycloalkyl and phenyl; each optionally independently substituted with 1-3 substituents chosen from C₁₋₃ alkyl, C₃₋₆ cycloalkyl, cyano, phenyl, and halogen, and n is 0 or
**R¹** is tetrahydropyranyl
and n is 0 or 1;
**R²** and **R³** are methyl or R² and R³ together with the carbon which they are attached to form cyclopropyl;
**R⁴** is imidazolyl, oxazolyl, oxadiazolyl, triazoyl or thiadiazolyl, each optionally independently substituted with one substituent chosen from C₁₋₆ alkyl, hydroxyl and halogen;
**R⁵** is cyclohexyl, isoxazolyl or pyrazolyl, each independently substituted with 1 to 3 substituents chosen from C₁₋₆ alkyl (which is optionally substituted with 1 to 3 halogen atoms).

3. The compound according to claim 2 wherein,
**R¹** is phenyl optionally independently substituted with 1 to 3 substituents chosen from C₁₋₃ alkyl (which is optionally substituted with 1 to 3 halogen atoms), halogen and cyano,
or **R¹** is C₁₋₅ alkyl or cyclohexyl, each optionally independently substituted with 1 to 3 substituents chosen from C₁₋₂ alkyl (which is optionally substituted with 1 to 3 atoms), hydroxyl, fluoro and chloro;
**R² and R³** are methyl or R² and R³ together with the carbon which they are attached to form cyclopropyl.

4. A compound of the formula IA 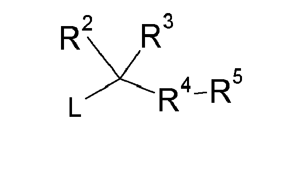 wherein for the formula (IA) 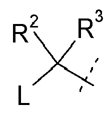 is chosen independently from members of column A in Table I, and 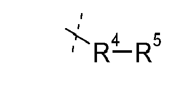 is chosen independently from members of column B in Table I: 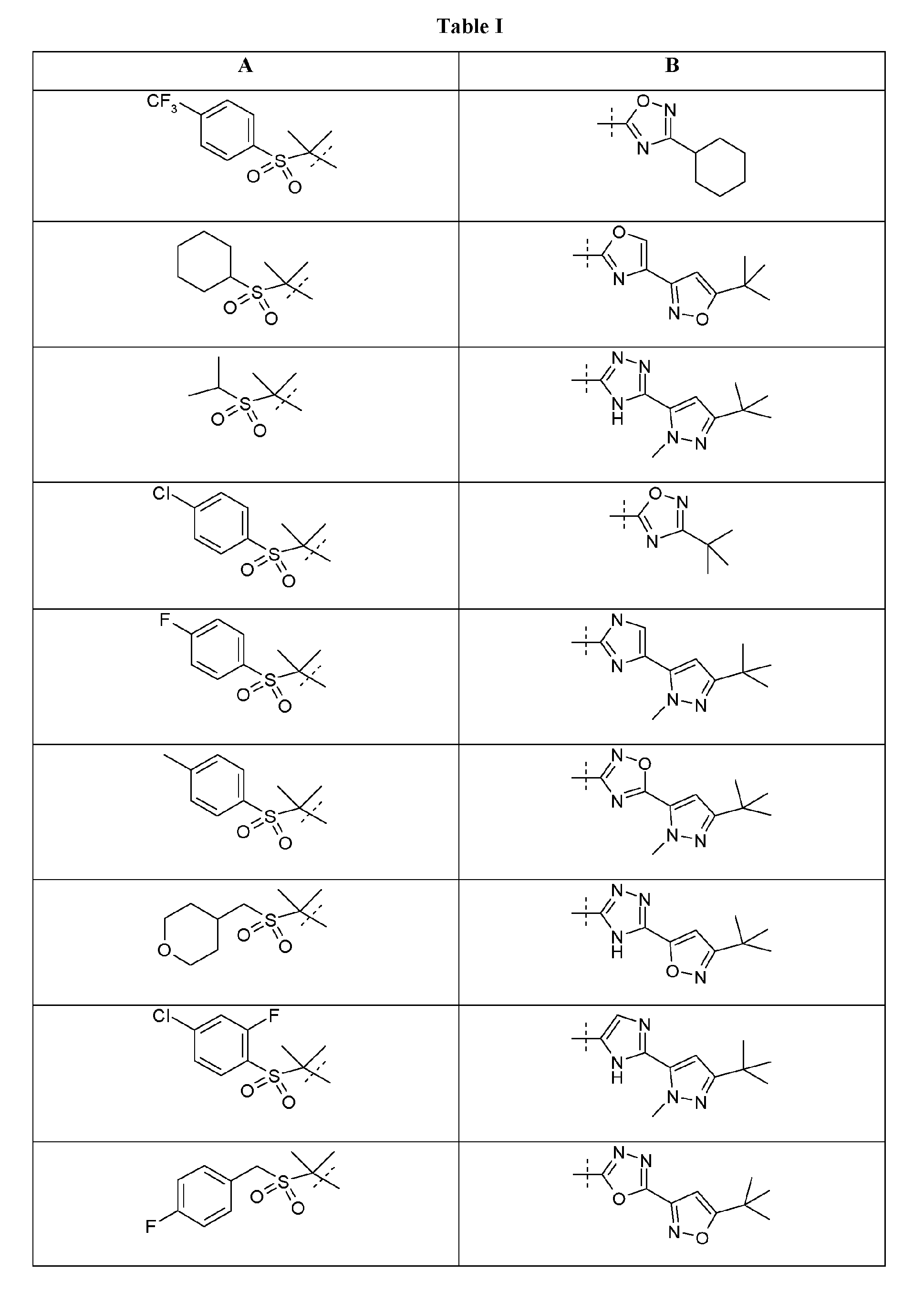 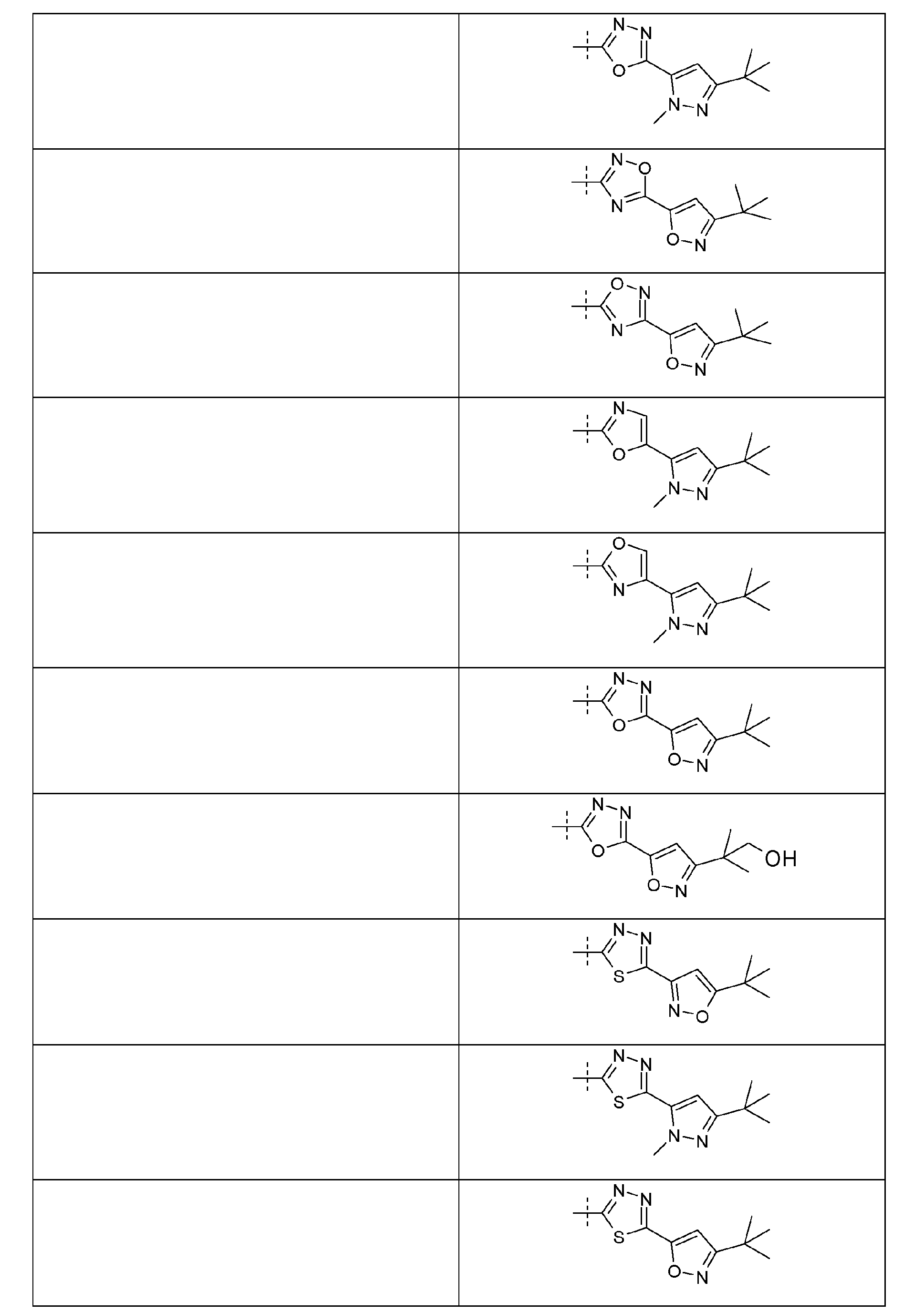 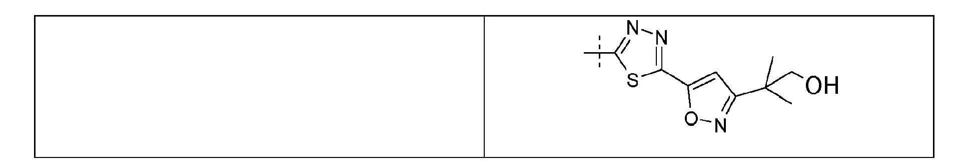 or a pharmaceutically acceptable salt thereof.

5. A compound chosen from
4-(5-tert-butyl-2-methyl-2H-pyrazol-3-yl)-2-[1-(4-chloro-benzenesulfonyl)-1-methyl-ethyl]-oxazole
4-(5-tert-butyl-2-methyl-2H-pyrazol-3-yl)-2-[1-(4-fluoro-benzenesulfonyl)-1-methyl-ethyl]-oxazole
4-(5-tert-butyl-2-methyl-2H-pyrazol-3-yl)-2-[1-(4-chloro-2-fluoro-benzenesulfonyl)-1-methyl-ethyl]-oxazole
4-(5-tert-butyl-2-methyl-2H-pyrazol-3-yl)-2-[1-methyl-1-(toluene-4-sulfonyl)-ethyl]-oxazole
4-(5-tert-butyl-2-methyl-2H-pyrazol-3-yl)-2-[1-methyl-1-(tetrahydro-pyran-4-ylmethane-sulfonyl)-ethyl]-oxazole
4-(5-tert-butyl-2-methyl-2H-pyrazol-3-yl)-2-(1-cyclohexanesulfonyl-1-methyl-ethyl)-oxazole
5-tert-butyl-3-{2-[1-(4-chloro-benzenesulfonyl)-1-methyl-ethyl]-oxazol-4-yl}-isoxazole
5-(5-tert-butyl-2-methyl-2H-pyrazol-3-yl)-2-[1-(4-chloro-benzenesulfonyl)-1-methyl-ethyl]-oxazole
5-(5-tert-butyl-2-methyl-2H-pyrazol-3-yl)-2-[1-(4-fluoro-benzenesulfonyl)-1-methyl-ethyl]-oxazole
5-(5-tert-butyl-2-methyl-2H-pyrazol-3-yl)-2- 1-methyl-1-(tetrahydro-pyran-4-ylmethane-sulfonyl)-ethyl]-oxazole
4-(5-tert-butyl-2-methyl-2H-pyrazol-3-yl)-2-(1-cyclohexanesulfonyl-1-methyl-ethyl)-oxazole
3-(5-tert-butyl-2-methyl-2H-pyrazol-3-yl)-5-[1-(4-chloro-benzenesulfonyl)-1-methyl-ethyl]-4H-[1,2,4]triazole
3-(3-tert-butyl-isoxazol-5-yl)-5-[1-(4-chloro-benzenesulfonyl)-1-methyl-ethyl]-4H-[1,2,4]-triazole
3-(3-tert-butyl-isoxazol-5-yl)-5-[1-methyl-1-(tetrahydro-pyran-4-ylmethanesulfonyl)-ethyl]-4H-[1,2,4]triazole
3-tert-butyl-5-[1-methyl-1-(4-trifluoromethyl-benzenesulfonyl)-ethyl]-[1,2,4]oxadiazole
3-cyclohexyl-5-[1-methyl-1-(4-trifluoromethyl-benzenesulfonyl)-ethyl]-[1,2,4]oxadiazole
3-cyclohexyl-5-[1-methyl-1-(propane-2-sulfonyl)-ethyl]-[1,2,4]oxadiazole
5-(5-tert-butyl-2-methyl-2H-pyrazol-3-yl)-3-[1-(4-chloro-benzenesulfonyl)-1-methyl-ethyl]-[1,2,4]oxadiazole
5-(5-tert-butyl-2-methyl-2H-pyrazol-3-yl)-3-[1-methyl-1-(tetrahydro-pyran-4-ylmethane-sulfonyl)-ethyl]-[1,2,4]oxadiazole
3-tert-butyl-5-{2-[1-(4-chloro-benzenesulfonyl)-1-methyl-ethyl]-3H-imidazol-4-yl}-1-methyl-1H-pyrazole
3-tert-butyl-5-{5-[1-(4-chloro-benzenesulfonyl)-1-methyl-ethyl]-1H-imidazol-2-yl}-1-methyl-1H-pyrazole
2-(3-tert-butyl-isoxazol-5-yl)-5-[1-(4-chloro-benzenesulfonyl)-1-methyl-ethyl]-[1,3,4]oxadiazole
2-(3-tert-butyl-isoxazol-5-yl)-5-[1-(4-fluoro-benzenesulfonyl)-1-methyl-ethyl]-[1,3,4]oxadiazole
2-(3-tert-butyl-isoxazol-5-yl)-5-[1-methyl-1-(tetrahydro-pyran-4-ylmethanesulfonyl)-ethyl]-[1,3,4]oxadiazole
2-(5-tert-butyl-isoxazol-3-yl)-5-[1-(4-chloro-benzenesulfonyl)-1-methyl-ethyl]-[1,3,4]oxadiazole
2-(5-tert-butyl-2-methyl-2H-pyrazol-3-yl)-5-[1-(4-chloro-benzenesulfonyl)-1-methyl-ethyl]-[1,3,4]oxadiazole
2-(5-tert-butyl-isoxazol-3-yl)-5-[1-(4-fluoro-benzenesulfonyl)-1-methyl-ethyl]-[1,3,4]oxadiazole
2-(5-tert-butyl-2-methyl-2H-pyrazol-3-yl)-5-[1-(4-fluoro-benzenesulfonyl)-1-methyl-ethyl]-[1,3,4]oxadiazole
2-(3-tert-butyl-isoxazol-5-yl)-5-[1-methyl-1-(tetrahydro-pyran-4-sulfonyl)-ethyl]-[1,3,4]oxadiazole
2-(5-tert-butyl-2-methyl-2H-pyrazol-3-yl)-5-[1-(4-fluoro-phenylmethanesulfonyl)-1-methyl-ethyl]-[1,3,4]oxadiazole
2-(3-tert-butyl-isoxazol-5-yl)-5-[1-(4-fluoro-phenylmethanesulfonyl)-1-methyl-ethyl]-[1,3,4]-oxadiazole
2-(5-tert-butyl-2-methyl-2H-pyrazol-3-yl)-5-[1-(4-chloro-benzenesulfonyl)-cyclopropyl]-[1,3,4]oxadiazole
2-(5-{5-[1-(4-fluoro-benzenesulfonyl)-1-methyl-ethyl]-[1,3,4]oxadiazol-2-yl}-isoxazol-3-yl)-2-methyl-propan-1-ol
3-(3-tert-butyl-isoxazol-5-yl)-5-[1-(4-fluoro-benzenesulfonyl)-1-methyl-ethyl]-[1,2,4]oxadiazole
3-(3-tert-butyl-isoxazol-5-yl)-5-[1-methyl-1-(4-trifluoromethyl-benzenesulfonyl)-ethyl]-[1,2,4]oxadiazole
3-(3-tert-butyl-isoxazol-5-yl)-5-[1-methyl-1-(tetrahydro-pyran-4-ylmethanesulfonyl)-ethyl]-[1,2,4]oxadiazole
5-(3-tert-butyl-isoxazol-5-yl)-3-[1-(4-fluoro-benzenesulfonyl)-1-methyl-ethyl]-[1,2,4]oxadiazole
5-(3-tert-butyl-isoxazol-5-yl)-3-[1-methyl-1-(tetrahydro-pyran-4-ylmethanesulfonyl)-ethyl]-[1,2,4]oxadiazole
2-(3-tert-butyl-isoxazol-5-yl)-5-[1-(4-fluoro-benzenesulfonyl)-1-methyl-ethyl]-[1,3,4]thiadiazole
2-(5-tert-butyl-isoxazol-3-yl)-5-[1-(4-fluoro-benzenesulfonyl)-1-methyl-ethyl]-[1,3,4]thiadiazole
2-(5-tert-butyl-2-methyl-2H-pyrazol-3-yl)-5-[1-(4-fluoro-benzenesulfonyl)-1-methyl-ethyl]-[1,3,4]thiadiazole
2-(5-tert-butyl-2-methyl-2H-pyrazol-3-yl)-5-[1methyl-1-(tetrahydro-pyran-4-sulfonyl)-ethyl]-[1,3,4]thiadiazole
2-(5-{5-[1-(4-fluoro-benzenesulfonyl)-1-methyl-ethyl]-[1,3,4]thiadiazol-2-yl}-isoxazol-3-yl)-2-methyl-propan-1-ol
3-(5-tert-butyl-2-methyl-2H-pyrazol-3-yl)-5-[1-(4-fluoro-benzenesulfonyl)-1-methyl-ethyl]-4H-[1,2,4]triazole
3-(3-tert-butyl-isoxazol-5-yl)-5-[1-(4-fluoro-benzenesulfonyl)-1-methyl-ethyl]-4H-[1,2,4]-triazole
3-(5-tert-butyl-2-methyl-2H-pyrazol-3-yl)-5-[1-methyl-1-(tetrahydro-pyran-4-ylmethane-sulfonyl)-ethyl]-4H-[1,2,4]triazole
3 -(5-tert-butyl-2-methyl-2H-pyrazol-3-yl)-5-(1-cyclopropylmethanesulfonyl-1-methyl-ethyl)-4H-[1,2,4]triazole
3-(5-tert-butyl-2-methyl-2H-pyrazol-3-yl)-5-[1-(4-fluoro-phenylmethanesulfonyl)-1-methyl-ethyl]-4H-[1,2,4]triazole
3-(3-tert-Butyl-isoxazol-5-yl)-5-[1-(4-fluoro-phenylmethanesulfonyl)-1-methyl-ethyl]-4H-[1,2,4]triazole
3-(5-tert-butyl-2-methyl-2H-pyrazol-3-yl)-5-[1-(4-methoxy-cyclohexylmethanesulfonyl)-1-methyl-ethyl]-4H-[1,2,4]triazole
4-{2-[5-(5-tert-butyl-2-methyl-2H-pyrazol-3-yl)-4H-[1,2,4]triazol-3-yl]-propane-2-sulfonylmethyl}-cyclohexanol
5-(5-tert-Butyl-2-methyl-2H-pyrazol-3-yl)-2-(1-cyclohexanesulfonyl-1-methyl-ethyl)-oxazole
2-(5-tert-tutyl-isoxazol-3-yl)-5-[1-(4-fluoro-benzenesulfonyl)-1-methyl-ethyl]-[1,3,4]oxadiazole
or a pharmaceutically acceptable salt thereof.

6. A pharmaceutical composition comprising a therapeutically effective amount of a compound according to any one of claims 1 - 5.

7. A compound according to any one of claims 1 - 5 for use in the treatment of a disease or condition chosen from pain, a lung disease, a rheumatic disease, an autoimmune disease, a musculoskeletal disease, an allergic disease, an allergic reaction, a vascular disease, a dermatological disease, a renal disease, a hepatic disease, a gastrointestinal disease, neurodegeneration eye disease, diseases of the ear, nose, and throat, neurological disease blood disease, tumors, endocrine diseases, organ and tissue transplantations and graft-versus-host diseases, severe states of shock, acute pain, visceral pain, spasm of the gastrointestinal tract or uterus, colics, neuropathic pain, inflammatory and nociceptive pain, cancer pain, headache, restenosis, atherosclerosis, reperfusion injury, congestive heart failure, myocardial infarction, thermal injury, multiple organ injury secondary to trauma, necrotizing enterocolitis and syndromes associated with hemodialysis, leukopheresis, and granulocyte transfusion, sarcoidosis, gingivitis and pyrexia.
